# EUROPEAN PATENT APPLICATION

(11) **EP 2 072 519 A1**
(43) Date of publication of application: **24.06.2009**
(21) Application number: 07828506.1
(22) Date of filing: 27.09.2007
(51) Int. Cl.: C07D 491/20, A61K 31/438, A61K 31/444, A61K 31/497, A61K 31/506, A61P 1/00, A61P 1/16, A61P 3/04, A61P 3/06, A61P 3/10, A61P 5/00, A61P 9/04, A61P 9/10, A61P 9/12, A61P 11/00, A61P 13/12, A61P 15/06, A61P 15/10, A61P 17/00, A61P 19/06

(54) **DIARYL KETIMINE DERIVATIVE**

(30) Priority: 28.09.2006 JP 2006264323; 05.07.2007 JP 2007177354
(71) Applicant: BANYU PHARMACEUTICAL CO., LTD., Tokyo 102-8667 (JP)
(72) Inventor: SUZUKI, Takao, Tsukuba-shi, Ibaraki 300-2611 (JP); ANDO, Makoto, Tsukuba-shi, Ibaraki 300-2611 (JP); MIYAZOE, Hiroshi, Tsukuba-shi, Ibaraki 300-2611 (JP); KAMEDA, Minoru, Tsukuba-shi, Ibaraki 300-2611 (JP); SEKINO, Etsuko, Tsukuba-shi, Ibaraki 300-2611 (JP); MORIYA, Minoru, Tsukuba-shi, Ibaraki 300-2611 (JP)
(74) Representative: Buchan, Gavin MacNicol
(86) International application number: PCT/JP2007/068760
(87) International publication number: WO 2008/038692

(57) **Abstract**

Provided is a compound of a formula (I): 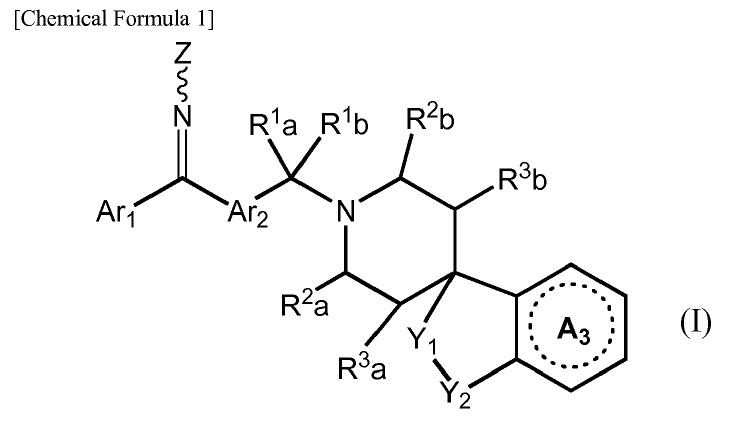 [wherein R^{1a} and R^{1b} are the same or different, representing a hydrogen atom, etc.; R^{2a} and R^{2b} are the same or different, representing a hydrogen atom, etc., or R^{2a} and R^{2b}, taken together, form -CH₂CH₂-, R^{3a} and R^{3b} are the same or different, representing a hydrogen atom, etc.; or R^{3a} and R^{3b}, taken together, form - CH₂CH₂-etc.; Y₁ and Y₂ represent -C(R)₂-, etc.; Z represents OR, NR₂, etc.; R represents a hydrogen atom, a C₁₋₆ alkyl group, etc.; Ar₁ represents a 6-membered aromatic carbocyclic group, etc.; Ar₂ represents a 6-membered aromatic carbocyclic group, etc; A₃ represents a 6-membered aromatic carbocyclic group etc.]. The compound is useful as a medicine for central disorders, cardiovascular disorders, metabolic disorders.

## Description

### TECHNICAL FIELD

The present invention relates to a novel diarylketimine derivative. The compound acts as a melanin concentrating hormone receptor antagonist, and is useful as a preventive or a remedy for various circular system diseases, nervous system diseases, metabolic diseases, reproductive diseases, respiratory diseases, digestive diseases, etc.

### BACKGROUND ART

Melanin concentrating hormone (hereafter abbreviated as "MCH") is a cyclic peptide hormone/neuro-peptide, which was for the first time isolated by Kawauchi, et al., in 1983 from sermon hypophysis [see Nature, Vol. 305, 321 (1983)]. The hormone is known to functionally antagonize for melanin cell stimulating hormone in fishes, to cause concentration of melanin granules in melanophore and participate in body color change [see International Review of Cytology, Vol. 126, 1 (1991); Trends in Endocrinology and Metabolism, Vol. 5, 120 (1994)]. Also in mammals, MCH-containing neuron cells are localized in the hypothalamus lateral field and uncertain zone, but their nerve fibers are projecting over a very wide scope in the brain [see The Journal of Comparative Neurology, Vol. 319, 218 (1992)], and MCH is considered to preside over various central functions in living bodies.

Hypothalamus lateral field is known of old as feeding center, and furthermore, recently molecular biological and pharmacological knowledge suggesting participation of MCH in controlling energetic homeostasis are being much accumulated. That is, it has been reported that expression of mRNA, which is an MCH precursor, is accelerated in the brains of ob/ob mice, db/db mice, KKAy mice and Zucker fatty rats which are model animals of hereditary obesity, and in the brains of fasted mice [see Nature, Vol. 380, 243 (1996); Diabetes, Vol. 47, 294 (1998); Biochemical and Biophysical Research Communications, Vol. 268, 88 (2000); Molecular Brain Research, Vol. 92, 43 (2001)].

Acute ventricular administration of MCH to rats was observed to induce accelerated feeding activity [Nature, Vol. 380, 243 (1996)] and chronic administration invites obesity accompanied by polyphagy [see Proceedings of the National Academy of Sciences of the United States of America, Vol. 99, 3240 (2002)]. Moreover, MCH precursor gene-deficient mice show reduced food ingestion or rise in oxygen consumption per body weight compared to wild type mice. Their low body weight due to decrease in body fat was observed [see Nature, Vol. 396, 670 (1998)].

On the contrary, transgenic mice which express excessive MCH precursor develop obesity accompanied by polyphagy and insulin resistance [see The Journal of Clinical Investigation, Vol. 107, 379 (2001)]. Consequently, it is suggested that MCH is an important factor for developing obesity and participates in diseases induced by metabolic disorders or respiratory diseases, of which one of risk factors is obesity. Besides, MCH is known to participate also in anxiety-causing action, epilepsy, memory, learning, diuretic action, excretory action of sodium and potassium, oxytocin secreting action, reproduction and reproductive function [see Peptides, Vol. 17, 171 (1996); Peptides, Vol. 18, 1095 (1997); Peptides, Vol. 15, 757 (1994); Journal of Neuroendocrinology, Vol. 8, 57 (1996); Critical Reviews in Neurobiology, Vol. 8, 221 (1994)].

MCH causes versatile pharmacological actions through MCH receptors which are present mainly in the central nervous system. As receptors of MCH, at least two types of receptors, type 1 receptors (MCH-1R or SLC-1) and type 2 receptors (MCH-2R or SLT) are known [see Nature, Vol. 400, 261 (1999); Nature, Vol. 400, 265 (1999); Biochemical and Biophysical Research Communications, Vol. 261, 622 (1999); Nature Cell Biology, Vol. 1, 267 (1999); FEBS Letters, Vol. 457, 522 (1999); Biochemical and Biophysical Research Communications, Vol. 283, 1013 (2001); The Journal of Biological Chemistry, Vol. 276, 20125 (2001); Proceedings of the National Academy of Sciences of the United States of America, Vol. 98, 7564 (2001); Proceedings of the National Academy of Sciences of the United States of America, Vol. 98, 7576 (2001); The Journal of Biological Chemistry, Vol. 276, 34664 (2001); Molecular Pharmacology, Vol. 60, 632 (2001)].

Of those, the pharmacological action observed on rodents is induced mainly via MCH-1R [see Genomics, Vol. 79, 785 (2002)]. Because MCH-1R gene-deficient mice chronically administered with MCH do not develop polyphagy or obesity, it is known that controlling of energy metabolism by MCH is induced via MCH-1R. Furthermore, the deficiency of MCH-1R is known to promote the activity amount of mice [see Proceedings of the National Academy of Sciences of the United States of America, Vol. 99, 3240 (2002)], and its participation in central diseases accompanied by behavioral disorders, for example, attention-deficit hyperactivity disorder, schizophrenia, depression and the like is also strongly suggested [see Molecular Medicine Today, Vol. 6, 43 (2000); Trends in Neuroscience, Vol. 24, 527 (2001)].

It is also reported that an autoantibody to MCH-1R is present in serum of vitiligo vulgaris patients [see The Journal of Clinical Investigation, Vol. 109, 923 (2002)]. Furthermore, expression of MCH-1R in certain species of cancer cells was reported, and in vivo expression sites of MCH and MCH-1R also suggest MCH's participation in cancer, sleep, vigil, drug dependence and digestive disorders [see Biochemical and Biophysical Research Communications, Vol. 289, 44 (2001); Neuroendocrinology, Vol. 61, 348 (1995); Endocrinology, Vol. 137, 561 (1996); The Journal of Comparative Neurology, Vol. 435, 26 (2001)].

Functions of MCH are expressed upon it binding to MCH receptors. Therefore, when its binding to MCH receptor is inhibited, then expression of MCH action can be inhibited. In consequence, substances which are antagonists for binding of MCH with its receptor are useful as preventing or treating agents for those various diseases in which MCH participates, for example, metabolic disorders such as obesity, diabetes, hormone disorder, hyperlipidemia, gout, fatty liver; cardiovascular disorders such as stenocardia, acute or congestive heart failure, myocardial infarction, coronary atherosclerosis, hypertension, renal diseases, electrolyte abnormality; central nervous system or peripheral nervous system disorders such as bulimia, emotional disturbance, depression, anxiety, epilepsy, delirium, dementia, schizophrenia, attention-deficit hyperactivity disorder, memory impairment, sleep disorders, cognitive failure, dyskinesia, paresthesias, smell disorders, morphine tolerance, drug dependence, alcoholism; reproductive disorders such as infertility, preterm labor and sexual dysfunction; and other digestive disorders, respiratory disorders, cancer or pigmentation.

As to compounds having an MCH receptor antagonistic effect, for example, a lot of 4-phenylpiperidine derivatives are disclosed in WO03/004027 (Patent Reference 1). However, the reference specification does not at all disclose compounds having imine skeleton.

WO96/26196 (Patent Reference 2) discloses benzylpiperidine derivatives as a muscarine antagonist. The reference specification discloses compounds having imine skeleton. However, it does not disclose compounds having a piperidine skeleton and an imine skeleton, which is a key point of the invention. Further, the reference specification describes nothing about an MCH receptor antagonistic effect.
Patent Reference 1: WO03/004027
Patent Reference 2: WO96/26196

### DISCLOSURE OF THE INVENTION

### PROBLEMS TO BE SOLVED BY THE INVENTION

The present inventors have assiduously studied compounds having an MCH receptor antagonistic effect, and as a result, have found that compounds having two aryl groups bonding to the carbon atom of imine, in which piperidine bonds to one aryl group via methylene, are novel compounds unknown in literature and have an MCH receptor antagonistic effect and are effective for prevention or remedy of various MCH receptor-associated diseases, and have completed the present invention.

Specifically, the invention provides:
(1) a diarylketimine derivative of a formula (I) or a pharmaceutically-acceptable salt thereof:

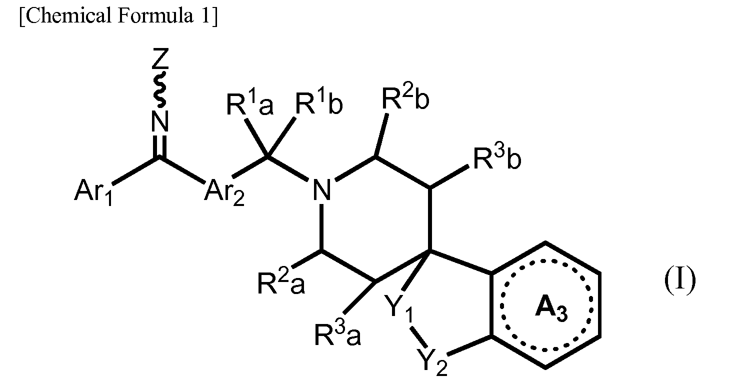

[wherein R^{1a} and R^{1b} are the same or different, each representing a hydrogen atom, or a C₁₋₆ alkyl group optionally having a substituent;
R^{2a} and R^{2b} are the same or different, each representing a hydrogen atom, or a C₁₋₆ alkyl group optionally having a substituent, or R^{2a} and R^{2b}, taken together, form -C(R⁴)₂-C(R⁵)₂-;
R^{3a} and R^{3b} are the same or different, each representing a hydrogen atom, or a C₁₋₆ alkyl group optionally having a substituent, or R^{3a} and R^{3b}, taken together, form -C(R⁶)₂-C(R⁷)₂-;
Y₁ represents -O- or -C(R⁸)₂-;
Y₂ represents -C(O)- or -C(R⁹)₂-, or Y₁ and Y₂, taken together, form -C(R¹⁰)=C(R¹¹)-;
Z represents -OR¹², -N(R^{13a})(R^{13b}), -NR¹⁴-COOR¹⁵, -NR¹⁶-COR¹⁷, -C(R^{18a})(R^{18b})(R^{18c}), - O-SO₂R¹⁹ or -SO₂R²⁰;
R⁴, R⁵, R⁶, R⁷, R⁸, R⁹, R¹⁰, R¹¹, R^{13a}, R^{13b}, R¹⁴, R¹⁵, R¹⁶, R¹⁷, R^{18a}, R^{18b} and R^{18c} are the same or different, each representing a hydrogen atom, or a C₁₋₆ alkyl group optionally having a substituent;
R¹² represents a hydrogen atom, a C₁₋₆ alkyl group optionally having a substituent, or a C₃₋₆ cycloalkyl group optionally having a substituent; the C₁₋₆ alkyl group or the C₃₋₆ cycloalkyl group may be substituted with a substituent selected from a group consisting of halogen, hydroxy, C₁₋₆ alkoxy, C₁₋₆ alkoxycarbonyl, C₁₋₆ alkylsulfonyl, (C₁₋₆ alkyl)amino, di(C₁₋₆ alkyl)amino, carbamoyl, (C₁₋₆ alkyl)carbamoyl, di(C₁₋₆ alkyl)carbamoyl and cyano;
R¹⁹ and R²⁰ each represent a C₁₋₆ alkyl group or a phenyl group optionally substituted with a C₁₋₆ alkyl group;
Ar₁ represents a 6-membered aromatic carbocyclic group optionally substituted with a substituent selected from a group α, or represents a 6-membered aromatic nitrogen-containing heterocyclic group optionally substituted with a substituent selected from the group α;
Ar₂ represents a group derived from a 6-membered aromatic carbocyclic ring, a 6-membered aromatic nitrogen-containing heterocyclic ring, a 5-membered aromatic heterocyclic ring or a pyridone ring by removing two hydrogen atoms from the ring, in which the 6-membered aromatic carbocyclic ring, the 6-membered aromatic nitrogen-containing heterocyclic ring, the 5-membered aromatic heterocyclic ring or the pyridone ring may be optionally substituted with a substituent selected from the group α;
the formula:

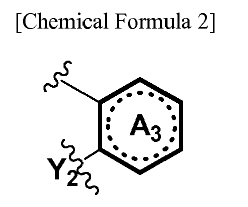

[this is hereinafter referred to as A₃; and in the formula, Y₂ is shown for indicating the bonding position] represents a group selected from the following group:

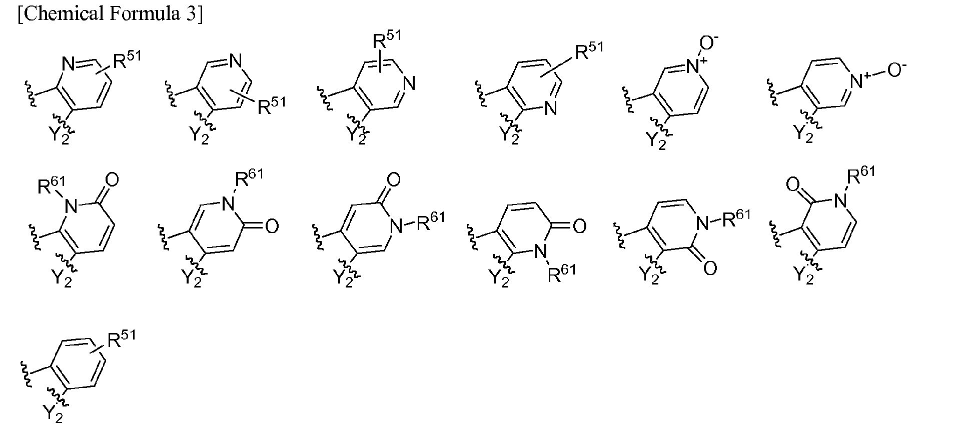

[wherein R⁵¹ represents a hydrogen atom, a hydroxyl group, a halogen, a C₁₋₆ alkyl group, a halo-C₁₋₆ alkyl group, a C₁₋₆ alkyloxy group, a halo-C₁₋₆ alkyloxy group or a C₁₋₆ alkylcarbonylamino group; R⁶¹ represents a hydrogen atom, or a C₁₋₆ alkyl group optionally having a substituent]].

Substituent group α:
halogen, cyano, hydroxy, amino, mono-C₁₋₆ alkylamino, di-C₁₋₆ alkylamino, C₁₋₆ alkyl, halo-C₁₋₆ alkyl, C₁₋₆ alkyloxy, halo-C₁₋₆ alkyloxy, C₁₋₆ alkyloxy-C₁₋₆ alkyl, C₁₋₆ alkyloxycarbonyl, C₁₋₆ alkyloxycarbonylamino, C₁₋₆ alkyloxycarbonyl(C₁₋₆ alkyl)amino, C₁₋₆ alkylcarbonyl, C₁₋₆ alkylcarbonyloxy, C₁₋₆ alkylcarbonylamino, C₁₋₆ alkylcarbonyl(C₁₋₆ alkyl)amino, carbamoyl, mono-C₁₋₆ alkylcarbamoyl, di-C₁₋₆ alkylcarbamoyl, carbamoylamino, mono-C₁₋₆ alkylcarbamoylamino, di-C₁₋₆ alkylcarbamoylamino, mono-C₁₋₆ alkylcarbamoyl(C₁₋₆ alkyl)amino, di-C₁₋₆ alkylcarbamoyl(C₁₋₆ alkyl)amino, carbamoyloxy, mono-C₁₋₆ alkylcarbamoyloxy, di C₁₋₆-alkylcarbamoyloxy, C₁₋₆ alkylsulfonyl, C₁₋₆ alkylsulfonylamino, C₁₋₆ alkylsulfonyl-(C₁₋₆ alkyl)amino, sulfamoyl, mono-C₁₋₆ alkylsulfamoyl, di-C₁₋₆ alkylsulfamoyl, sulfamoylamino, mono-C₁₋₆ alkylsulfamoylamino, di-C₁₋₆ alkylsulfamoylamino, mono-C₁₋₆ alkylsulfamoyl(C₁₋₆ alkyl)amino and di-C₁₋₆ alkylsulfamoyl(C₁₋₆ alkyl)amino.

The invention further provides:
(2) a melanin concentrating hormone receptor antagonist comprising a compound of (1) or a pharmaceutically-acceptable salt thereof as the active ingredient,
(3) a pharmaceutical composition comprising a pharmaceutically-acceptable additive and a therapeutically-effective amount of a compound of (1) or a pharmaceutically-acceptable salt thereof,
(4) a preventive or a remedy for metabolic disorders such as obesity, diabetes, hormone disorder, hyperlipidemia, gout, fatty liver, hepatitis, cirrhosis; cardiovascular disorders such as stenocardia, acute or congestive heart failure, myocardial infarction, coronary atherosclerosis, hypertension, renal diseases, electrolyte abnormality; central nervous system or peripheral nervous system disorders such as bulimia, emotional disturbance, depression, anxiety, epilepsy, delirium, dementia, schizophrenia, attention-deficit hyperactivity disorder, memory impairment, sleep disorders, cognitive failure, dyskinesia, paresthesias, smell disorders, morphine tolerance, drug dependence, alcoholism; reproductive disorders such as infertility, preterm labor and sexual dysfunction; digestive disorders; respiratory disorders; cancer or pigmentation comprising a compound of (1) or a pharmaceutically-acceptable salt thereof as the active ingredient.

The invention is described in more detail hereinunder.

In this description, the term "lower" means that the number of the carbon atoms constituting the group or the compound with the term is at most 6, preferably at most 4.

"C₁₋₆ alkyl group" includes a linear alkyl group having from 1 to 6 carbon atoms or a branched alkyl group having from 3 to 6 carbon atoms, concretely, for example, a methyl group, an ethyl group, an n-propyl group, an isopropyl group, an n-butyl group, an isobutyl group, a sec-butyl group, a tert-butyl group, an n-pentyl group, an isopentyl group, a neopentyl group, a tert-amyl group, a 2-propyl group, a 2-methylbutyl group, a 1,2-dimethylpropyl group, a 1-ethylpropyl group, an n-hexyl group, an isohexyl group, a 1-methylpentyl group, a 2-methylpentyl group, a 3-methylpentyl group, a 1,1-dimethylbutyl group, a 1,2-dimethylbutyl group, a 2,2-dimethylbutyl group, a 1-ethylbutyl group, a 1,1,2-trimethylpropyl group, a 1,2,2-trimethylpropyl group, a 1-ethyl-2-methylpropyl group, 1-ethyl-1-methylpropyl group et al.

Examples of the substituent of the "C₁₋₆ alkyl group optionally having a substituent" for R^{1a}, R^{1b},R^{2a}, R^{2b}, R^{3a}, R^{3b}, R⁴, R⁵, R⁶, R⁷, R⁸, R⁹, R¹⁰, R¹¹, R^{13a}, R^{13b}, R¹⁴, R¹⁵, R¹⁶, R¹⁷, R^{18a}, R^{18b}, R^{18c} and R⁶¹ includes those of a group β, preferably halogen, hydroxy, and C₁₋₆ alkyloxy optionally substituted with a fluorine atom; and the group may be substituted with from 1 to 3, preferably one or two such substituents.

Substituents of group β:
halogen atom, cyano, hydroxy, amino, mono-C₁₋₆ alkylamino, di-C₁₋₆ alkylamino, C₁₋₆ alkyloxy optionally substituted with fluorine atom, C₁₋₆ alkyloxy-C₁₋₆ alkyl, C₁₋₆ alkyloxycarbonyl, C₁₋₆ alkyloxycarbonylamino, C₁₋₆ alkyloxycarbonyl(C₁₋₆ alkyl)amino, C₁₋₆ alkylcarbonyl, C₁₋₆ alkylcarbonyloxy, C₁₋₆ alkylcarbonylamino, C₁₋₆ alkylcarbonyl(C₁₋₆ alkyl)amino, carbamoyl, mono-C₁₋₆ alkylcarbamoyl, di-C₁₋₆ alkylcarbamoyl, carbamoylamino, mono-C₁₋₆ alkylcarbamoylamino, di-C₁₋₆ alkylcarbamoylamino, mono-C₁₋₆ alkylcarbamoyl(C₁₋₆ alkyl)amino, di-C₁₋₆ alkylcarbamoyl(C₁₋₆ alkyl)amino, carbamoyloxy, mono-C₁₋₆ alkylcarbamoyloxy, di-C₁₋₆-alkylcarbamoyloxy, C₁₋₆ alkylsulfonyl, C₁₋₆ alkylsulfonylamino, C₁₋₆ alkylsulfonyl-(C₁₋₆ alkyl)amino, sulfamoyl, mono-C₁₋₆ alkylsulfamoyl, di-C₁₋₆ alkylsulfamoyl, sulfamoylamino, mono-C₁₋₆ alkylsulfamoylamino, di-C₁₋₆ alkylsulfamoylamino, mono-C₁₋₆ alkylsulfamoyl(C₁₋₆ alkyl)amino and di-C₁₋₆ alkylsulfamoyl(C₁₋₆ alkyl)amino.

In the definition of the above substituents, "halogen atom" includes a fluorine atom, a chlorine atom, a bromine atom and an iodine atom.

"C₃₋₆ cycloalkyl group" means a cycloalkyl group having from 3 to 6 carbon atoms, concretely including a cyclopropyl group, a cyclobutyl group, a cyclopentyl group, a cyclohexyl group et al.

"Halo-C₁₋₆ alkyl group" includes a C₁₋₆ alkyl group in which a part or all of the hydrogen atoms are substituted with halogen, for example, a fluoromethyl group, a difluoromethyl group, a trifluoromethyl group, a 2-fluoroethyl group, a 1,2-difluoroethyl group et al.

"C₁₋₆ alkyloxy group" includes a group of a C₁₋₆ alkyl group bonding to an oxygen atom, concretely, for example, a methoxy group, an ethoxy group, an n-propyloxy group, an isopropyloxy group, an n-butoxy group, an n-pentyloxy group et al.

"Halo-C₁₋₆ alkyloxy group" includes a group of a halo-C₁₋₆ alkyl group bonding to an oxygen atom, concretely, for example, a fluoromethoxy group, a difluoromethoxy group, a trifluoromethoxy group, a 1,2-difluoroethoxy group et al.

"C₁₋₆ alkyl group optionally substituted with a fluorine atom" includes a C₁₋₆ alkyl group, or a C₁₋₆ alkyl group in which a part or all of the hydrogen atoms are substituted with fluorine atom; and the latter lower alkyl group substituted with a fluorine atom includes, for example, a fluoromethyl group, a difluoromethyl group, a trifluoromethyl group, a 2-fluoroethyl group, a 1,2-difluoroethyl group et al.

"C₁₋₆ alkyloxy group optionally substituted with a fluorine atom" includes a group of a C₁₋₆ alkyl group or a fluorine atom-substituted lower alkyl group bonding to an oxygen atom; and concretely, for example, the C₁₋₆ alkyloxy group includes a methoxy group, an ethoxy group, an n-propyloxy group, an isopropyloxy group an n-butoxy group, an n-pentyloxy group et al, and the fluorine atom-substituted C₁₋₆ alkyloxy group includes, for example, a fluoromethoxy group, a difluoromethoxy group, a trifluoromethoxy group, a 1,2-difluoroethoxy group et al.

"Mono-C₁₋₆ alkylamino group" is a group of an amino group (-NH₂) in which one hydrogen atom is substituted with a C₁₋₆ alkyl group, and concretely includes, for example, a methylamino group, an ethylamino group, an n-propylamino group, an isopropylamino group, an n-butylamino group et al.

"Di-C₁₋₆ alkylamino group" is a group of an amino group (-NH₂) in which two hydrogen atoms are substituted with a C₁₋₆ alkyl group, and concretely includes, for example, a dimethylamino group, a diethylamino group, an ethylmethylamino group, a di(n-propyl)amino group, a methyl(n-propyl)amino group, a diisopropylamino group et al.

"C₁₋₆ alkyloxy-lower alkyl group" is a C₁₋₆ alkyl group substituted with a C₁₋₆ alkyloxy group, and concretely includes, for example, a methoxymethyl group, an ethoxymethyl group, an n-propyloxymethyl group, an isopropyloxymethyl group, a 1-methoxyethyl group, a 2-methoxyethyl group et al.

"C₁₋₆ alkyloxycarbonyl group" is a C₁₋₆ alkyloxy group bonding to a carbonyl group (-CO-) and includes an alkyloxycarbonyl group having from 1 to 6 carbon atoms, concretely, for example, a methoxycarbonyl group, an ethoxycarbonyl group, an n-propyloxycarbonyl group, an isopropyloxycarbonyl group, an n-butoxycarbonyl group et al.

"C₁₋₆ alkyloxycarbonylamino group" is a group of an amino group (-NH₂) in which one hydrogen atom is substituted with a C₁₋₆ alkyloxycarbonyl group, and includes an alkyloxycarbonylamino group having from 1 to 6 carbon atoms, concretely, for example, a methoxycarbonylamino group, an ethoxycarbonylamino group, an n-propyloxycarbonylamino group, an isopropyloxycarbonylamino group, an n-butoxycarbonylamino group, an n-pentyloxycarbonylamino group et al.

"C₁₋₆ alkyloxycarbonyl(C₁₋₆ alkyl)amino group" is a group of a mono-C₁₋₆ alkylamino group in which the hydrogen atom on the nitrogen atom is substituted with a C₁₋₆ alkyloxycarbonyl group and concretely includes, for example, a methoxycarbonyl(methyl)amino group, an ethoxycarbonyl(methyl)amino group, an n-propyloxycarbonyl(methyl)amino group et al.

"C₁₋₆ alkylcarbonyl group" is a group of a carbonyl group (-CO-) bonding to a C₁₋₆ alkyl group, and includes an alkylcarbonyl group having from 1 to 6 carbon atoms, concretely, for example, an acetyl group, a propionyl group, a butyryl group, an isobutyryl group, a valeryl group, an isovaleryl group, a pivaloyl group et al.

"C₁₋₆ alkylcarbonyloxy group" is a C₁₋₆ alkylcarbonyl group bonding to an oxygen atom, and concretely includes, for example, an acetoxy group, a propionyloxy group, a valeryloxy group, an isovaleryloxy group, a pivaloyloxy group et al.

"C₁₋₆ alkylcarbonylamino group" is a group of an amino group (-NH₂) in which one hydrogen atom is substituted with a C₁₋₆ alkylcarbonyl group, and concretely includes, for example, an acetylamino group, a propionylamino group, an isobutyrylamino group, a valerylamino group, an isovalerylamino group, a pivaloylamino group et al.

"C₁₋₆ alkylcarbonyl(C₁₋₆ alkyl)amino group" is a mono-C₁₋₆ alkylamino group in which the hydrogen atom on the nitrogen atom is substituted with a C₁₋₆ alkylcarbonyl group, and includes, for example, a methylcarbonyl(methyl)amino group, an ethylcarbonyl(methyl)amino group, an n-propylcarbonyl(methyl)amino group et al.

"Mono-C₁₋₆ alkylcarbamoyl group" is a carbamoyl group (-CONH₂) in which one hydrogen atom is substituted with a C₁₋₆ alkyl group, and concretely includes, for example, a methylcarbamoyl group, an ethylcarbamoyl group, an n-propylcarbamoyl group, an isopropylcarbamoyl group, an n-butylcarbamoyl group et al.

"Di-C₁₋₆ alkylcarbamoyl group" is a carbamoyl group (-CONH₂) in which two hydrogen atoms are substituted with a C₁₋₆ alkyl group, and concretely includes, for example, a dimethylcarbamoyl group, a diethylcarbamoyl group, an ethylmethylcarbamoyl group, a di(n-propyl)carbamoyl group, a methyl(n-propyl)carbamoyl group, a diisopropylcarbamoyl group et al.

"Mono-C₁₋₆ alkylcarbamoylamino group" is an amino group (-NH₂) in which one hydrogen atom is substituted with a mono-C₁₋₆ alkylcarbamoyl group, and concretely includes, for example, a methylcarbamoylamino group, an ethylcarbamoylamino group, an n-propylcarbamoylamino group, an isopropylcarbamoylamino group, an n-butylcarbamoylamino group et al.

"Di-C₁₋₆ alkylcarbamoylamino group" is an amino group (-NH₂) in which one hydrogen atom is substituted with a di-C₁₋₆ alkylcarbamoyl group, and concretely includes, for example, a dimethylcarbamoylamino group, a diethylcarbamoylamino group, a di(n-propyl)carbamoylamino group, a diisopropylcarbamoylamino group et al.

"Mono-C₁₋₆ alkylcarbamoyl(C₁₋₆ alkyl)amino group" is a mono-C₁₋₆ alkylamino group in which the hydrogen atom on the nitrogen atom is substituted with a mono-C₁₋₆ alkylcarbamoyl group, and concretely includes, for example, a monomethylcarbamoyl(methyl)amino group, a monoethylcarbamoyl(methyl)amino group, a [mono(n-propyl)carbamoyl](methyl)amino group et al.

"Di-C₁₋₆ alkylcarbamoyl(C₁₋₆ alkyl)amino group" is a mono-C₁₋₆ alkylamino group in which the hydrogen atom on the nitrogen atom is substituted with a di-C₁₋₆ alkylcarbamoyl group, and concretely includes, for example, a dimethylcarbamoyl(methyl)amino group, a diethylcarbamoyl(methyl)amino group, a [di(n-propyl)carbamoyl](methyl)amino group et al.

"Mono-C₁₋₆ alkylcarbamoyloxy group" is a mono-C₁₋₆ alkylcarbamoyl group bonding to an oxygen atom, and concretely includes, for example, a methylcarbamoyloxy group, an ethylcarbamoyloxy group, an n-propylcarbamoyloxy group, an isopropylcarbamoyloxy group, an n-butylcarbamoyloxy group et al.

"Di-C₁₋₆ alkylcarbamoyloxy group" is a di-C₁₋₆ alkylcarbamoyl group bonding to an oxygen atom, and concretely includes, for example, a dimethylcarbamoyloxy group, a diethylcarbamoyloxy group, an ethylmethylcarbamoyloxy group, a di(n-propyl)carbamoyloxy group, a methyl(n-propyl)carbamoyloxy group, a diisopropylcarbamoyloxy group et al.

"C₁₋₆ alkylsulfonyl group" is a C₁₋₆ alkyl group bonding to a sulfonyl group (-SO₂- and concretely includes, for example, a methanesulfonyl group, an ethanesulfonyl group, an n-propanesulfonyl group, an isopropanesulfonyl group, an n-butanesulfonyl group et al.

"C₁₋₆ alkylsulfonylamino group" is an amino group (-NH₂) in which one hydrogen atom is substituted with a C₁₋₆ alkylsulfonyl group, and concretely includes, for example, a methanesulfonylamino group, an ethanesulfonylamino group, an n-propanesulfonylamino group, an isopropanesulfonylamino group, an n-butanesulfonylamino group et al.

"C₁₋₆ alkylsulfonyl(lower alkyl)amino group" is a group of a mono-C₁₋₆ alkylamino group in which the hydrogen atom on the nitrogen atom is substituted with a C₁₋₆ alkylsulfonyl group, and concretely includes, for example, a methanesulfonyl(methyl)amino group, an ethanesulfonyl(methyl)amino group, an n-propanesulfonyl(methyl)amino group, an isopropanesulfonyl(methyl)amino group et al.

"Mono-C₁₋₆ alkylsulfamoyl group" is a group of a sulfamoyl group (-SO₂NH₂) in which one hydrogen atom is substituted with a C₁₋₆ alkyl group, and concretely includes, for example, a monomethylsulfamoyl group, a monoethylsulfamoyl group, a mono(n-propyl)sulfamoyl group, a monoisopropylsulfamoyl group, a mono(n-butyl)sulfamoyl group et al.

"Di-C₁₋₆ alkylsulfamoyl group" is a group of a sulfamoyl group (-SO₂NH₂) in which two hydrogen atoms are substituted with a C₁₋₆ alkyl group, and concretely includes, for example, a dimethylsulfamoyl group, a diethylsulfamoyl group, a di(n-propyl)sulfamoyl group, a diisopropylsulfamoyl group, a di(n-butyl)sulfamoyl group et al.

"Mono-C₁₋₆ alkylsulfamoylamino group" is a group of an amino group (-NH₂) in which one hydrogen atom is substituted with a mono-C₁₋₆ alkylsulfamoyl group, and concretely includes, for example, a (monomethylsulfamoyl)amino group, a (monoethylsulfamoyl)amino group, a [mono(n-propyl)sulfamoyl]amino group, a (monoisopropylsulfamoyl)amino group, a [mono(n-butyl)sulfamoyl] amino group et al.

"(Di-C₁₋₆ alkylsulfamoyl)amino group" is a group of an amino group (-NH₂) in which one hydrogen atom is substituted with a di-C₁₋₆ alkylsulfamoyl group, and concretely includes, for example, a (dimethylsulfamoyl)amino group, a (diethylsulfamoyl)amino group, an (ethylmethylsulfamoyl)amino group, a [di(n-propyl)sulfamoyl]amino group, a [methyl(n-propyl)sulfamoyl]amino group, a (diisopropylsulfamoyl)amino group et al.

"Mono-C₁₋₆ alkylsulfamoyl(C₁₋₆ alkyl)amino group" is a group of a mono-C₁₋₆ alkylamino group in which the hydrogen atom on the nitrogen atom is substituted with a mono-C₁₋₆ alkylsulfamoyl group, and concretely includes, for example, a monomethylsulfamoyl(methyl)amino group, a monoethylsulfamoyl(methyl)amino group, a [mono(n-propyl)sulfamoyl](methyl)amino group et al.

"Di-C₁₋₆ alkylsulfamoyl(C₁₋₆ alkyl)amino group" is a group of a mono-C₁₋₆ alkylamino group in which the hydrogen atom on the nitrogen atom is substituted with a di-C₁₋₆ alkylsulfamoyl group, and concretely includes, for example, a dimethylsulfamoyl(methyl)amino group, a diethylsulfamoyl(methyl)amino group, a [di(n-propyl)sulfamoyl](methyl)amino group et al.

"Pharmaceutically-acceptable salts" of a compound of formula (I) include ordinary salts that are acceptable as medicines. Their examples are acid-addition salts to the amine moiety of the compound of formula (I) or acid-addition salts to the nitrogen-containing heterocyclic ring thereof, or base-addition salts to the acidic substituent, if any, of the compound of formula (I).

The acid-addition salts include inorganic acid salts such as hydrochlorides, sulfates, nitrates, phosphates, perchlorates et al; organic acid salts such as maleates, fumarates, tartarates, citrates, ascorbates, trifluoroacetates et al; and sulfonates such as methanesulfonates, isothiocyanates, benzenesulfonates, p-toluenesulfonates et al.

The base-addition salts include alkali metal salts such as sodium salts, potassium salts et al; alkaline earth metal salts such as calcium salts, magnesium salts et al; and organic amine salts such as ammonium salts, trimethylamine salts, triethylamine salts, dicyclohexylamine salts, ethanolamine salts, diethanolamine salts, triethanolamine salts, procaine salts, N,N'-dibenzylethylenediamine salts et al.

For the purpose of more concretely disclosing the compounds of the invention hereinunder, various symbols used in formula (I) are described in detail with reference to their examples.

R^{1a} and R^{1b} are the same or different, each representing a hydrogen atom, or a C₁₋₆ alkyl group optionally having a substituent.

Concretely, R^{1a} and R^{1b} are the same or different, each representing a hydrogen atom, a methyl group, an ethyl group, an n-propyl group et al, preferably a hydrogen atom or a methyl group.

R^{2a} and R^{2b} are the same or different, each representing a hydrogen atom, or a C₁₋₆ alkyl group optionally having a substituent, or R^{2a} and R^{2b}, taken together, form -C(R⁴)₂-C(R⁵)₂-.

R⁴ and R⁵ are the same or different, each representing a hydrogen atom, or a C₁₋₆ alkyl group optionally having a substituent.

Concretely, R⁴ and R⁵ are the same or different, each representing, for example, a hydrogen atom, a methyl group et al.

Concretely, R^{2a} and R^{2b} are the same or different, each representing, for example, a hydrogen atom, a fluorine atom, a chlorine atom, a bromine atom, a methyl group, an ethyl group, an n-propyl group, an isopropyl group, an n-butyl group, a tert-butyl group; or R^{2a} and R^{2b}, taken together, form, for example, -CH₂CH₂-, -CH₂-CH(CH₃)-, -CH(CH₃)-CH₂- et al. Preferably, a hydrogen atom or a methyl group, or R^{2a} and R^{2b}, taken together, form -CH₂-CH₂-, are recommended.

R^{3a} and R^{3b} are the same or different, each representing a hydrogen atom, or a C₁₋₆ alkyl group optionally having a substituent, or R^{3a} and R^{3b}, taken together, form -C(R⁶)₂-C(R⁷)₂-.

R⁶ and R⁷ are the same or different, each representing, for example, a hydrogen atom or a methyl group et al.

Concretely, R^{3a} and R^{3b} are the same or different, each representing, for example, a hydrogen atom, a fluorine atom, a chlorine atom, a bromine atom, a methyl group, an ethyl group, an n-propyl group, an isopropyl group, an n-butyl group, a tert-butyl group; or R^{3a} and R^{3b}, taken together, form, for example, -CH₂CH₂-, -CH₂-CH(CH₃)-, -CH(CH₃)-CH₂- et al. Preferably, R^{3a} and R^{3b} are both hydrogen atoms.

Y₁ represents -O- or -C(R⁸)₂-,
Y₂ represents -C(O)- or -C(R⁹)₂-, or Y₁ and Y₂, taken together, form -C(R¹⁰)=C(R¹¹)-.

R⁸, R⁹, R¹⁰ and R¹¹ are the same or different, each representing, for example, a hydrogen atom or a methyl group et al.

Preferably, for example, Y₁ and Y₂ are the following:

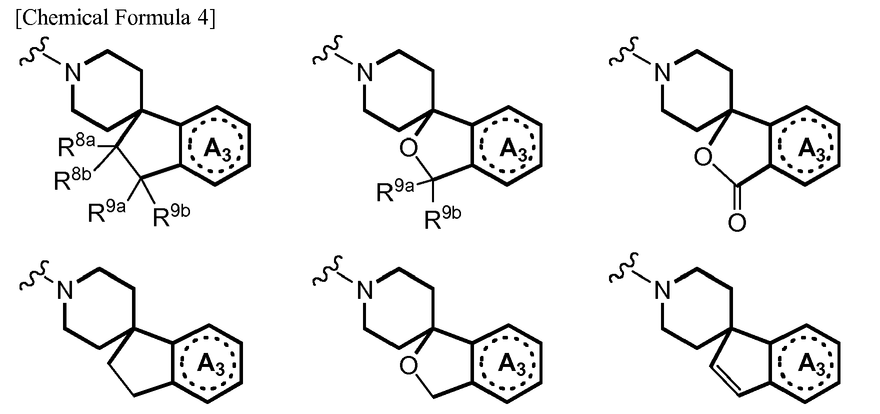

[wherein R^{8a} and R^{8b} are the same or different and have the same meaning as R⁸; and R^{9a} and R^{9b} are the same or different and have the same meaning as R⁹], more preferably the following is recommended.

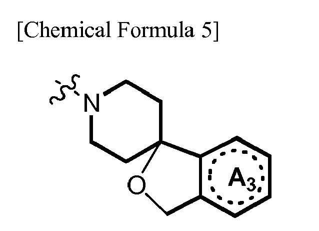

Z represents -OR¹², -N(R^{13a})(R^{13b}), -NR¹⁴-COOR¹⁵, -NR¹⁶-COR¹⁷, -C(R^{18a})(R^{18b})(R^{18c}), - O-SO₂R¹⁹ or -SO₂R²⁰_{.}

R¹² represents a hydrogen atom, a C₁₋₆ alkyl group optionally having a substituent, or a C₃₋₆ cycloalkyl group optionally having a substituent; the C₁₋₆ alkyl group or the C₃₋₆ cycloalkyl group may be substituted with a substituent selected from a group consisting of halogen, hydroxy, C₁₋₆ alkoxy, C₁₋₆ alkoxycarbonyl, C₁₋₆ alkylsulfonyl, (C₁₋₆ alkyl)amino, di(C₁₋₆ alkyl)amino, carbamoyl, (C₁₋₆ alkyl)carbamoyl, di(C₁₋₆ alkyl)carbamoyl and cyano. The C₁₋₆ alkyl group or the C₃₋₆ cycloalkyl group may be substituted with one or more, preferably from 1 to 3, the same or different such substituents.

R^{13a}, R^{13b}, R¹⁴, R¹⁵, R¹⁶, R¹⁷, R^{18a}, R^{18b} and R^{18c} are the same or different, each representing a hydrogen atom, or a C₁₋₆ alkyl group optionally having a substituent;

R¹⁹ and R²⁰ each represent a C₁₋₆ alkyl group, or a phenyl group optionally substituted with a C₁₋₆ alkyl group.

Concretely, Z includes, for example, the following:
1) as -OR¹², a hydroxyl group, a methoxy group, an ethoxy group, an n-propyloxy group, an isopropyloxy group, a fluoromethoxy group, a difluoromethoxy group, a trifluoromethoxy group, a 2-fluoroethoxy group, a 2-chloroethoxy group, a 2,2-difluoroethoxy group, a 2-methoxyethoxy group, a 2-hydroxyethoxy group, a 2-hydroxy-2-methylpropyloxy group, a methoxycarbonylmethoxy group, a carbamoylmethoxy group, a methylcarbamoylmethoxy group, a dimethylcarbamoylmethoxy group, a 2-dimethylaminoethoxy group, a cyanomethyloxy group, a cyanoethyloxy group, a cyclopropyloxy group et al;
2) as -N(R^{13a})(R^{13b}), an amino group, a dimethylamino group, a diethylamino group et al;
3) as -NR¹⁴-COOR¹⁵, a methoxycarbonylamino group, an ethoxycarbonylamino group et al,
4) as -NR¹⁶-COR¹⁷, a methylcarboxamino group, an ethylcarboxamino group et al,
5) as -C(R^{18a})(R^{18b})(R^{18e}), a methyl group, an ethyl group, an n-butyl group, an isobutyl group, a t-butyl group, a difluoromethyl group et al,
6) as -O-SO₂R¹⁹, a methylsulfonyloxy group, an ethylsulfonyloxy group, a p-toluenesulfonyloxy group, a benzenesulfonyloxy group et al,
7) as -SO₂R²⁰, a methylsulfonyl group, an ethylsulfonyl group et al.

Z is preferably -OR¹², for example, a hydroxyl group, a methoxy group, an ethoxy group, an n-propyloxy group, an isopropyloxy group, a 2-fluoroethoxy group, a 2,2-difluoroethoxy group, a 2-hydroxyethoxy group, a dimethylamino group, a dimethylcarbamoylmethoxy group, a difluoromethyl group, a 2-hydroxy-2-methylpropyloxy group, a cyanomethyloxy group et al.

Ar₁ represents a 6-membered aromatic carbocyclic group optionally substituted with a substituent selected from a group α, or represents a 6-membered aromatic nitrogen-containing heterocyclic group optionally substituted with a substituent selected from the group α. Ar₁ may be substituted with from 1 to 4, preferably 1 or 2, the same or different substituents selected from the group α.

The 6-membered aromatic carbocyclic ring for Ar₁ is, for example, a benzene ring; and the 6-membered aromatic nitrogen-containing heterocyclic ring includes, for example, a pyridine ring, a pyrazine ring, a pyrimidine ring, a pyridazine ring et al.

The substituent selected from the group α for Ar₁ is preferably halogen, more preferably a fluorine atom or a chlorine atom.

Concretely, the 6-membered aromatic carbocyclic group for Ar₁ includes a phenyl group, a 4-fluorophenyl group, a 3,4-difluorophenyl group et al; and the 6-membered aromatic nitrogen-containing heterocyclic group includes a pyridyl group, a 5-fluoropyridin-2-yl group, a 5-chloropyridin-2-yl group et al. Preferably, Ar₁ is a 6-membered aromatic carbocyclic group substituted with one or two fluorine atoms or chlorine atoms, or a 6-membered aromatic nitrogen-containing heterocyclic group substituted with one or two fluorine atoms or chlorine atoms; more preferably a 3,4-difluorophenyl group, or a 5-chloropyridin-2-yl group.

Ar₂ represents a group derived from a 6-membered aromatic carbocyclic ring, a 6-membered aromatic nitrogen-containing heterocyclic ring, a 5-membered aromatic heterocyclic ring or a pyridone ring by removing two hydrogen atoms from the ring, in which the 6-membered aromatic carbocyclic ring, the 6-membered aromatic nitrogen-containing heterocyclic ring, the 5-membered aromatic heterocyclic ring or the pyridone ring may be optionally substituted with a substituent selected from the group α. Ar₂ may be substituted with from 1 to 4, preferably one or two, the same or different substituents selected from the group α.

An example of the 6-membered aromatic carbocyclic ring for Ar₂ is a benzene ring; the 6-membered aromatic nitrogen-containing heterocyclic ring includes, for example, a pyridine ring, a pyrazine ring, a pyrimidine ring, a pyridazine ring; and the 5-membered aromatic heterocyclic ring includes, for example, a thiophene ring, a thiazole ring, an oxazole ring, a thiadiazole ring, an oxadiazole ring et al.

Preferred examples of the substituent selected from the group α for Ar₂ are halogen such as fluorine, chlorine et al; C₁₋₆ alkyl such as methyl, ethyl et al; C₁₋₆ alkyloxy such as methoxy, ethoxy et al; C₁₋₆ alkylsulfonyl such as methanesulfonyl, ethanesulfonyl et al.

More concretely, Ar₂ is a 6-membered aromatic carbocyclic ring group such as a 1,4-phenylenediyl group, a 3-methoxyphenylene-1,4-diyl group, a 3-methanesulfonylphenylene-1,4-diyl group, a 2-fluorophenylene-1,4-diyl group, a 3-fluorophenylene-1,4-diyl group, a 2-methylphenylene-1,4-diyl group et al; a 6-membered aromatic nitrogen-containing heterocyclic ring group such as a pyridine-2,5-diyl group, a pyrimidine-2,5-diyl group, a pyrazine-2,5-diyl group, a pyridazine-3,6-diyl group et al; a 5-membered aromatic heterocyclic ring group such as a thiophene-2,5-diyl group; or a pyridonediyl group.

Ar₂ is preferably a 1,4-phenylenediyl group, a 3-methoxyphenylene-1,4-diyl group, a 3-methanesulfonylphenylene-1,4-diyl group, a 2-fluorophenylene-1,4-diyl group, a 3-fluorophenylene-1,4-diyl group, a 2-methylphenylene-1,4-diyl group, a pyridine-2,5-diyl group, a pyrimidine-2,5-diyl group, a pyrazine-2,5-diyl group, a pyridazine-3,6-diyl group, a thiophene-2,5-diyl group, a pyridone diyl group et al.

Preferred examples of A₃ are selected from the following group:

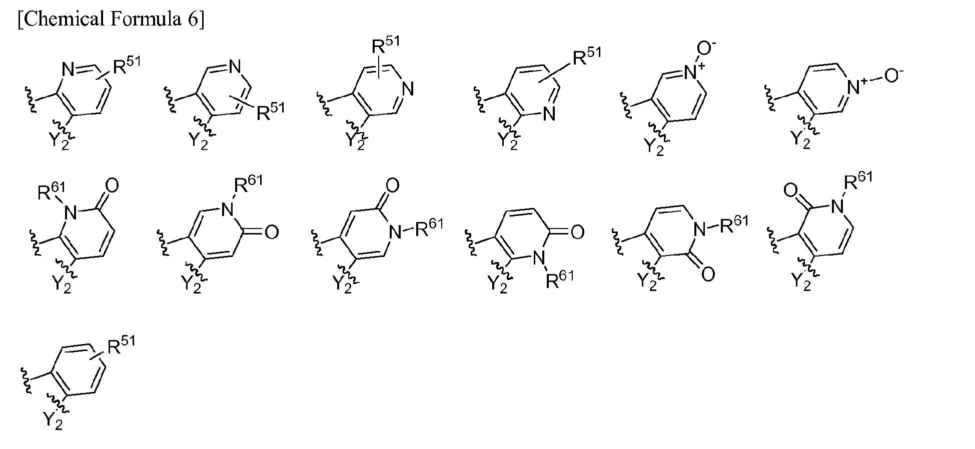

[wherein R⁵¹ represents a hydrogen atom, a hydroxyl group, a halogen, a C₁₋₆ alkyl group, a halo-C₁₋₆ alkyl group, a C₁₋₆ alkyloxy group, a halo-C₁₋₆ alkyloxy group or a C₁₋₆ alkylcarbonylamino group; R⁶¹ represents a hydrogen atom, or a C₁₋₆ alkyl group optionally having a substituent].

Concretely, R⁵¹ is a hydrogen atom, a hydroxyl group; a halogen such as a fluorine atom, a chlorine atom, a bromine atom; a C₁₋₆ alkyl group such as a methyl group, an ethyl group, an n-propyl group, an isopropyl group, an n-butyl group et al; a halo-C₁₋₆ alkyl group such as a chloromethyl group, a fluoromethyl group, a difluoromethyl group, a chloroethyl group, a fluoroethyl group et al; a C₁₋₆ alkyloxy group such as a methoxy group, an ethoxy group, an n-propyloxy group, an isopropyloxy group, an n-butyloxy group et al; a halo-C₁₋₆ alkyloxy group such as a chloromethoxy group, a fluoromethoxy group, a chloroethoxy group, a fluoroethoxy group et al; a C₁₋₆ alkylcarbonylamino group such as a methylcarbonylamino group, an ethylcarbonylamino group, an n-propylcarbonylamino group et al; and preferably a hydrogen atom, a fluorine atom, a bromine atom, a methoxy group, an ethoxy group, a methylcarbonylamino group, an ethylcarbonylamino group et al are recommended.

R⁶¹ is concretely a hydrogen atom, a methyl group, an ethyl group, an n-propyl group, an isopropyl group, an n-butyl group et al, preferably a hydrogen atom, a methyl group are recommended.

A₃ is preferably the following:

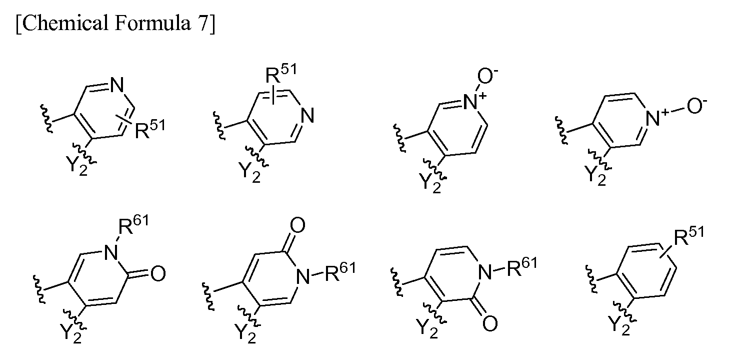

in which R⁵¹ is preferably a hydrogen atom, a fluorine atom, a bromine atom, a methoxy group, an ethoxy group, a methylcarbonylamino group, an ethylcarbonylamino group et al. R⁶¹ is preferably a hydrogen atom, a methyl group et al.

Preferred compounds of the invention are as follows:
(Z)-(3,4-difluorophenyl) {4-[(6-fluoro-1H,1'H-spiro[furo[3,4-c]pyridine-3,4'-piperidin]-1'-yl)methyl]phenyl}methanone O-methyloxime,
(E)-(3,4-difluorophenyl){5-[(6-fluoro-1H,1'H-spiro[furo[3,4-c]pyridine-3,4'-piperidin]-1'-yl)methyl]-2-pyridinyl}methanone O-methyloxime,
(E)-(3,4-difluorophenyl)[5-(1H, 1'H-spiro [furo[3,4-c]pyridine-3,4'-piperidin]-1'-ylmethyl)pyridin-2-yl]methanone O-(2-fluoroethyl)oxime,
(E)-(3,4-difluorophenyl)[2-(1H,1'H-spiro [furo[3,4-c]pyridine-3,4'-piperidin]-1'-ylmethyl)pyrimidin-5-yl]methanone O-(2-fluoroethyl)oxime,
(E)-(3,4-difluorophenyl)[5-(1H,1'H-spiro [furo[3,4-c]pyridine-3,4'-piperidin]-1'-ylmethyl)pyrimidin-2-yl]methanone O-ethyloxime,
N- {1'-[(6-{(E)-(3,4-difluorophenyl)[(ethyloxy)imino]methyl}-3-pyridinyl)methyl]-3H-spiro[furo[3,4-c]pyridine-1,4'-piperidin]-6-yl} acetamide,
(E)-(3,4-difluorophenyl)[5-(1H,1'H-spiro[furo[3,4-c]pyridine-3,4'-piperidin]-1'-ylmethyl)pyridin-2-yl]methanone O-(2-hydroxy-2-methylpropyl)oxime,
1'-({6-[(E)-(3,4-difluorophenyl)(ethoxyimino)methyl]pyridin-3-yl}methyl)-5-methyl-3,5-dihydro-6H-spiro[furo[3,4-c]pyridine-1,4'-piperidin]-6-one,
1'-({6-[(E)-(3,4-difluorophenyl)(ethoxyimino)methyl]pyridin-3-yl}methyl)-3,5-dihydro-6H-spiro[furo[3,4-c]pyridine-1,4'-piperidin]-6-one,
1'-({5-[(E)-(3,4-difluorophenyl)(ethoxyimino)methyl]pyridin-2-yl}methyl)-5-methyl-3,5-dihydro-6H-spiro[furo[3,4-c]pyridine-1,4'-piperidin]-6-one,
1'-({6-[(E)-(3,4-difluorophenyl)(hydroxyimino)methyl]pyridin-3-yl}methyl)-5-methyl-3,5-dihydro-6H-spiro[furo[3,4-c]pyridine-1,4'-piperidin]-6-one,
{[((1E)-(3,4-difluorophenyl){5-[(5-methyl-6-oxo-5,6-dihydro-1'H,3H-spiro[furo[3,4-c]pyridine-1,4'-piperidin]-1'-yl)methyl]pyridin-2-yl}methylene)amino]oxy}acetonitrile,
1'-[(6- {(E)-(3,4-difluorophenyl)[(2-hydroxy-2-methylpropoxy)imino]methyl}pyridin-3-yl)methyl]-5-methyl-3,5-dihydro-6H-spiro[furo[3,4-c]pyridine-1,4'-piperidin]-6-one,
(Z)-(3,4-difluorophenyl) {4-[(6-fluoro-1H,1'H-spiro[furo[3,4-c]pyridine-3,4'-piperidin]-1'-yl)methyl]phenyl}methanone O-(2-methoxyethyl)oxime,
methyl {[((1Z)-(3,4-difluorophenyl){4-[(6-fluoro-1H,1'H-spiro[furo[3,4-c]pyridine-3,4'-piperidin]-1'-yl)methyl]phenyl}methylene)amino]oxy} acetate,
(Z)-(3,4-difluorophenyl){4-[(6-fluoro-1H,1'H-spiro[furo[3,4-c]pyridine-3,4'-piperidin]-1'-yl)methyl]phenyl}methanone O-(2-hydroxyethyl)oxime,
2-{[((1Z)-(3,4-difluorophenyl){4-[(6-fluoro-1H,1'H-spiro[furo[3,4-c]pyridine-3,4'-piperidin]-1'-yl)methyl]phenyl} methylene)amino] oxy} -N,N-dimethylacetamide,
2-{[((1Z)-(3,4-difluorophenyl){4-[(6-fluoro-1H,1'H-spiro[furo[3,4-c]pyridine-3,4'-piperidin]-1'-yl)methyl]phenyl}methylene)amino]oxy}-N-methylacetamide,
(E)-(3,4-difluorophenyl) {5-[(5-oxido-1H,1'H-spiro[furo[3,4-c]pyridine-3,4'-piperidin]-1'-yl)methyl]-2-pyridinyl}methanone O-ethyloxime,
(Z)-(5-chloropyridin-2-yl)[4-(1H,1'H-spiro[furo[3,4-c]pyridine-3,4'-piperidin]-1'-ylmethyl)phenyl]methanone O-ethyloxime,
(Z)-(3,4-difluorophenyl)[3-methyl-4-(1H,1'H-spiro [furo[3,4-c]pyridine-3,4'-piperidin]-1'-ylmethyl)phenyl]methanone O-methyloxime,
[({(1E)-(3,4-difluorophenyl)[5-(1H,1'H-spiro[furo[3,4-c]pyridine-3,4'-piperidin]-l'-ylmethyl)-2-pyridinyl]methylidene} amino)oxy]acetonitrile,
1'-{[4-((Z)-(5-chloro-2-pyridinyl) {[(2-hydroxy-2-methylpropyl)oxy]imino}methyl)phenyl]methyl}-5-methyl-3,5-dihydro-6H-spiro[furo[3,4-c]pyridine-1,4'-piperidin]-6-one,
[({(1E)-(3,4-difluorophenyl)[3-(methyloxy)-4-(1H,1'H-spiro[furo[3,4-c]pyridine-3,4'-piperidin]-1'-ylmethyl)phenyl]methylidene}amino)oxy]acetonitrile,
[({(1Z)-(3,4-difluorophenyl)[3-(methyloxy)-4-(1H,1'H-spiro[furo[3,4-c]pyridine-3,4'-piperidin]-1'-ylmethyl)phenyl]methylidene} amino)oxy] acetonitrile,
1'-[(4- {(Z)-(6-chloro-3-pyridinyl)[(ethyloxy)imino]methyl}phenyl)methyl]-5-methyl-3,5-dihydro-6H-spiro[furo[3,4-c]pyridine-1,4'-piperidin]-6-one,
1'-({6-[(E)-[(cyclopropyloxy)imino](3,4-difluorophenyl)methyl]-3-pyridinyl}methyl)-5-methyl-3,5-dihydro-6H-spiro[furo[3,4-c]pyridine-1,4'-piperidin]-6-one,
(E)-(3,4-difluorophenyl)[5-(1H,1'H-spiro [furo[3,4-c]pyridine-3,4'-piperidin]-1'-ylmethyl)-2-pyrimidinyl]methanone O-(2-hydroxy-2-methylpropyl)oxime,
(E)-(3,4-difluorophenyl){5-[(6-fluoro-1H,1'H-spiro[furo[3,4-c]pyridine-3,4'-piperidin]-1'-yl)methyl]-2-pyrimidinyl}methanone O-(2-hydroxy-2-methylpropyl)oxime,
(E)-(3,4-difluorophenyl)[5-(1H,1'H-spiro[furo[3,4-c]pyridine-3,4'-piperidin]-1'-ylmethyl)-2-pyridinyl]methanone oxime,
(R)-(E)-(3,4-difluorophenyl) {5-[1-(1H,1'H-spiro[furo[3,4-c]pyridine-3,4'-piperidin]-1'-yl)ethyl]-2-pyridinyl}methanone O-(2-hydroxy-2-methylpropyl)oxime,
(S)-(E)-(3,4-difluorophenyl){5-[1-(1H,1'H-spiro[furo[3,4-c]pyridine-3,4'-piperidin]-1'-yl)ethyl]-2-pyridinyl}methanone O-(2-hydroxy-2-methylpropyl)oxime, or
1'-[(6- {(E)-(3,4-difluorophenyl)[(fluoromethoxy)imino]methyl}-3-pyridinyl)methyl]-5-methyl-3,5-dihydro-6H-spiro[furo[3,4-c]pyridine-1,4'-piperidin]-6-one et al; more preferably,
(E) or (Z)-(3,4-difluorophenyl){4-[(6-fluoro-1H,1'H-spiro[furo[3,4-c]pyridine-3,4'-piperidin]-1'-yl)methyl]phenyl}methanone oxime,
(E) or (Z)-(3,4-difluorophenyl)[5-(1H,1'H-spiro[furo[3,4-c]pyridine-3,4'-piperidin]-1'-ylmethyl)pyrimidin-2-yl]methanone O-ethyloxime,
1'-({6-[(E) or (Z)-(3,4-difluorophenyl)(ethoxyimino)methyl]pyridin-3-yl}methyl)-5-methyl-3,5-dihydro-6H-spiro[furo[3,4-c]pyridine-1,4'-piperidin]-6-one,
(Z)-(3,4-difluorophenyl)[4-(1H,1'H-spiro[furo [3,4-c]pyridine-3,4'-piperidin]-1'-ylmethyl)phenyl]methanone O-(2-fluoroethyl)oxime,
(E)-(3,4-difluorophenyl){5-[1-(1H,1H'-spiro[furo[3,4-c]pyridine-3,4'-piperidin]-1'-yl)ethyl]pyridin-2-yl}methanone O-(2-fluoroethyl)oxime,
{[((1E)-(3,4-difluorophenyl){5-[(5-methyl-6-oxo-5,6-dihydro-1'H,3H-spiro[furo[3,4-c]pyridine-1,4'-piperidin]-1'-yl)methyl]pyridin-2-yl}methylene)amino]oxy} acetonitrile,
2-{[((1Z)-(3,4-difluorophenyl){4-[(6-fluoro-1H,1'H-spiro[furo[3,4-c]pyridine-3,4'-piperidin]-1'-yl)methyl]phenyl} methylene)amino] oxy} -N,N-dimethylacetamide,
(E)-(3,4-difluorophenyl) {5-[(5-oxido-1H,1'H-spiro[furo[3,4-c]pyridine-3,4'-piperidin]-1'-yl)methyl]-2-pyridinyl}methanone O-ethyloxime,
(E)-(3,4-difluorophenyl){5-[1-(1H,1'H-spiro[furo[3,4-c]pyridine-3,4'-piperidin]-1'-yl)ethyl]-2-pyridinyl}methanone O-(2-hydroxy-2-methylpropyl)oxime, or
1'-[(6- {(E)-(3,4-difluorophenyl)[(fluoromethoxy)imino]methyl}-3-pyridinyl)methyl]-5-methyl-3,5-dihydro-6H-spiro[furo[3,4-c]pyridine-1,4'-piperidin]-6-one.

### Production Methods for Compounds of Formula (I)

The compounds of formula (I) can be produced, for example, according to the following production methods, however, the invention should not be limited to them.

### Production Method 1-1:

Production Method 1-1 is method for obtaining a compound of formula (I) by reacting a compound of formula (II) and a compound of formula (III).

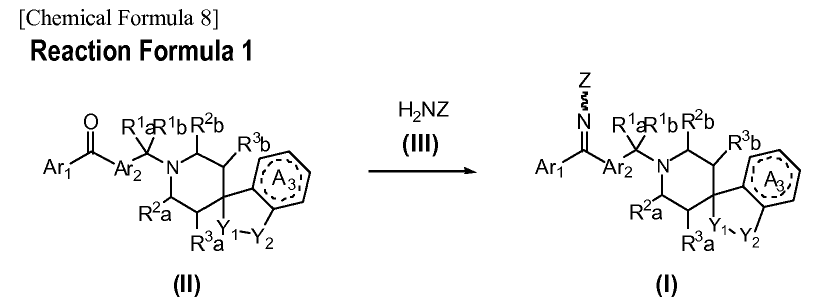

[Ar₁, Ar₂, A₃, R^{1a}, R^{1b}, R^{2a} , R^{2b} , R^{3a} , R^{3b}, Z, Y₁ and Y₂ have the same meanings as above.]

Specifically, a compound of formula (II) is reacted with a compound of formula (III) through oximation or hydrazonation in a known method to give a compound of formula (I).

The amount of the compound of formula (III) to be used is, for example, from 1.0 to 5.0 mols per mol of the compound of formula (II), preferably from 1.0 to 1.5 mols.

### 1) Reaction Condition for Oximation:

Examples of the reaction solvent are a lower alcohol such as methanol, ethanol, n-butanol, isopropyl alcohol, or pyridine et al.

The reaction temperature is, for example, from 0 to 100°C, preferably from 10 to 30°C, and the reaction is completed generally from 0.5 to 24 hours.

Examples of the compound of formula (III) are hydroxylamine hydrochloride, O-methylhydroxylamine hydrochloride, O-ethylhydroxylamine hydrochloride et al.

### 2) Reaction Condition for Hydrazonation:

The reaction solvent includes, for example, a mixed solvent of a lower alcohol such as methanol, ethanol, n-butanol, isopropyl alcohol et al, and acetic acid. The blend ratio by volume is recommendably such that acetic acid accounts for from 0.1 to 2.0 or so relative to alcohol of 10.

The reaction temperature is, for example, from 0 to 150°C, preferably from 60 to 120°C, and the reaction is completed generally from 0.5 to 24 hours.

The compound of formula (III) includes acetohydrazide, methoxycarbonylhydrazine, N-methylacetylhydrazide et al.

The reaction liquid containing the compound of formula (I) obtained according to the above method contains remaining reagents and side products, and therefore, the compound of formula (I) may be isolated through extraction or purification in a conventional known manner. (The same shall apply to the production methods to be mentioned hereinunder.)

### Production Method 1-2:

Production Method 1-2 is method for obtaining a compound of formula (Ia) through condensation of a compound of formula (I) where Z is a hydroxyl group, or that is a compound of formula (IIa) with a compound of formula (IIIa).

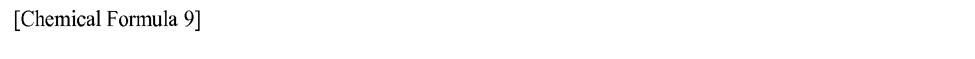

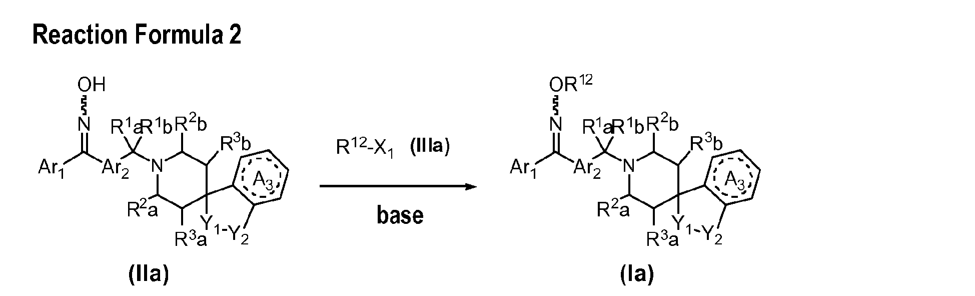

[In the formula, X₁ represents a leaving group such as a halogen atom, a benzenesulfonyloxy group, a p-toluenesulfonyloxy group, a methanesulfonyloxy group et al; Ar₁, Ar₂, A₃, R^{1a}, R^{1b}, R^{2a}, R^{2b}, R^{3a}, R^{3b}, R¹², Y₁ and Y₂ have the same meanings as above.]

Specifically, a compound of formula (IIa) is condensed with a compound of formula (IIIa) in a reaction solvent in the presence of a base to give a compound of formula (Ia).

The amount of the compound of formula (IIIa) to be used is, for example, from 1.0 to 2.0 mols per mol of the compound of formula (IIa), preferably from 1.0 to 1.5 mols.

The reaction solvent includes, for example, diethyl ether, tetrahydrofuran (hereinafter referred to as "THF"), 1,4-dioxane (hereinafter referred to as "dioxane"), dimethylformamide (hereinafter referred to as "DMF"), dimethylsulfoxide (hereinafter referred to as "DMSO") et al.

The base includes, for example, inorganic bases such as sodium carbonate, sodium hydrogencarbonate, potassium carbonate, potassium hydrogencarbonate, lithium carbonate et al; organic bases such as triethylamine, diisopropylethylamine, pyridine et al. The amount of the base to be used is, for example, from 1.0 to 5.0 mols per mol of the compound of formula (IIa), preferably from 1.1 to 1.5 mols.

The reaction temperature is, for example, from 0 to 100°C, preferably from 0 to 65°C; and the reaction is completed generally from 0.5 to 24 hours.

The compound of formula (IIIa) includes, for example, the following:

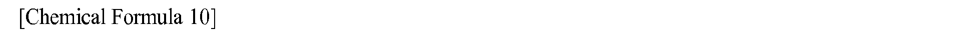

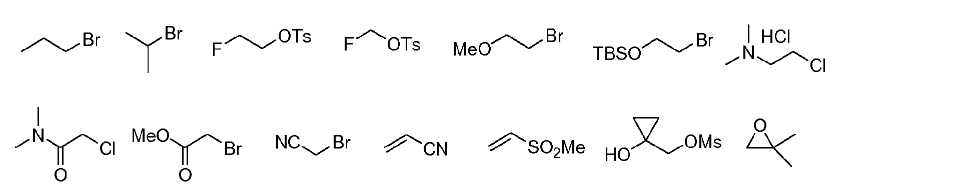

### Production Method 1-3:

Production Method 1-3 is method for producing a compound of formula (I), starting from a compound of formula (IVb).

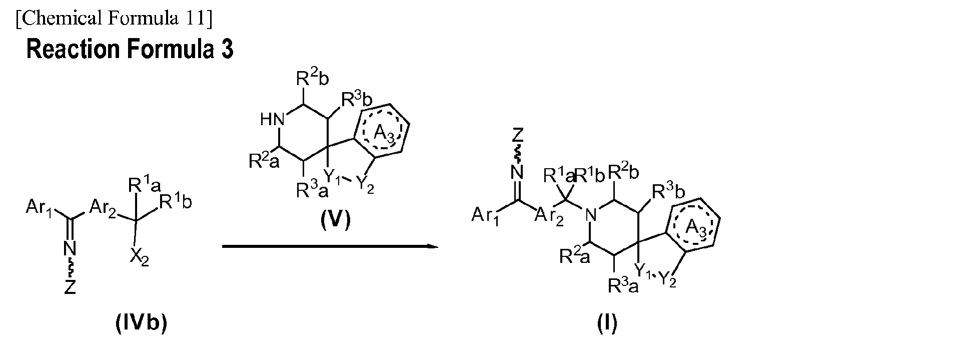

[In the formula, X₂ has the same meaning as that of X₁; and Ar₁, Ar₂, A₃, Z, R^{1a}, R^{1b}, R^{2a}, R^{2b}, R^{3a}, R^{3b}, Y₁ and Y₂ have the same meanings as above.]

A compound of formula (IVb) is reacted with a compound of formula (V) in a reaction solvent preferably in the presence of a base to give a compound of formula (I).

The amount of the compound of formula (V) to be used is, for example, from 1.0 to 1.5 mols per mol of the compound of formula (IVb), preferably from 1.0 to 1.3 mols.

The base includes, for example, inorganic bases such as sodium carbonate, sodium hydrogencarbonate, potassium carbonate, potassium hydrogencarbonate, lithium carbonate et al; organic bases such as triethylamine, diisopropylethylamine, pyridine et al. The amount of the base to be used is, for example, from 1.0 to 5.0 mols per mol of the compound of formula (IVb), preferably from 1.1 to 1.5 mols.

The reaction solvent includes, for example, halogenohydrocarbons such as methylene chloride, chloroform, carbon tetrachloride et al; ethers such as diethyl ether, THF, dioxane et al; and DMF, DMSO et al.

The reaction temperature is, for example, from 0 to 100°C, preferably from 10 to 40°C; and the reaction is completed generally from 1 to 24 hours.

The compound of formula (V) may be produced according to the methods described in WO2004/069798, WO2004/064762; or may be produced according to the methods described in Examples. Concretely, for example, the compound includes the following:

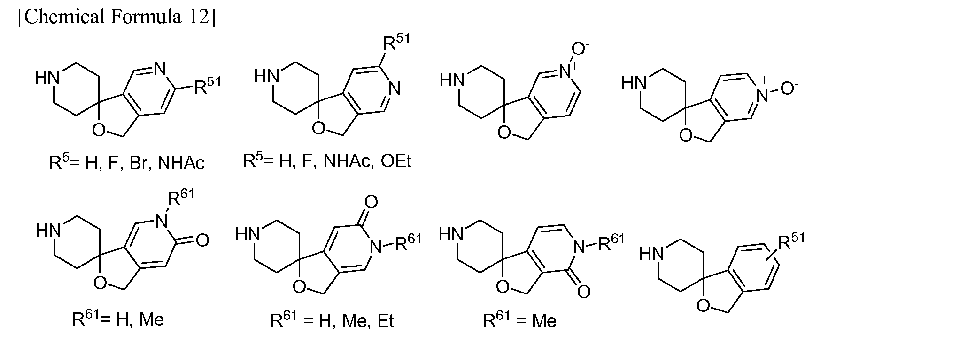

### Production Method 2-1:

Production Method 2-1 is method for producing a compound of formula (II).

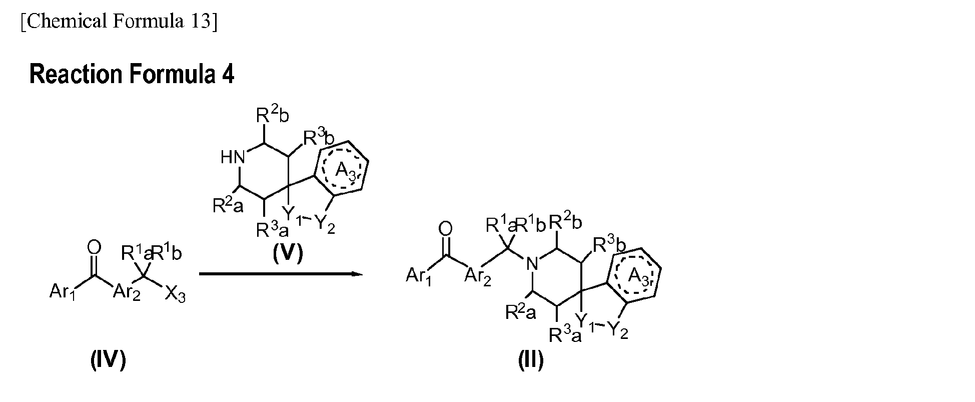

[In the formula, X₃ has the same meaning as that of X₁; and Ar₁, Ar₂, A₃, R^{1a}, R^{1b}, R^{2a}, R^{2b}, R^{3a}, R^{3b}, Y₁ and Y₂ have the same meanings as above.]

A compound of formula (IV) is condensed with a compound of formula (V) in a reaction solvent to give a compound of formula (II).

The amount of the compound of formula (V) to be used is, for example, from 1.0 to 2.0 mols per mol of the compound of formula (IV), preferably from 1.0 to 1.5 mols.

The reaction solvent includes, for example, halogenohydrocarbons such as chloroform, methylene chloride, carbon tetrachloride et al; ethers such as THF, diethyl ether, dioxane et al; and DMF, DMSO et al.

Preferably, the reaction is attained in the presence of a base, for example, an inorganic base such as sodium carbonate, sodium hydrogencarbonate, potassium carbonate, potassium hydrogencarbonate, lithium carbonate et al, or an organic base such as triethylamine, diisopropylethylamine, pyridine et al.

The amount of the base, when used, is, for example, from 1.0 to 5.0 mols per mol of the compound of formula (V), preferably from 1.1 to 2.0 mols.

The reaction temperature is, for example, from 0 to 100°C, preferably from 10 to 30°C; and the reaction is completed generally from 0.5 to 24 hours.

The compound of formula (IIa) may be produced according to Production Method 1-1, starting from a compound of formula (II).

### Production Method 2-2:

Production Method 2-2 is method for producing a compound of formula (II), starting from a compound of formula (IVa).

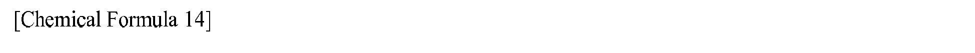

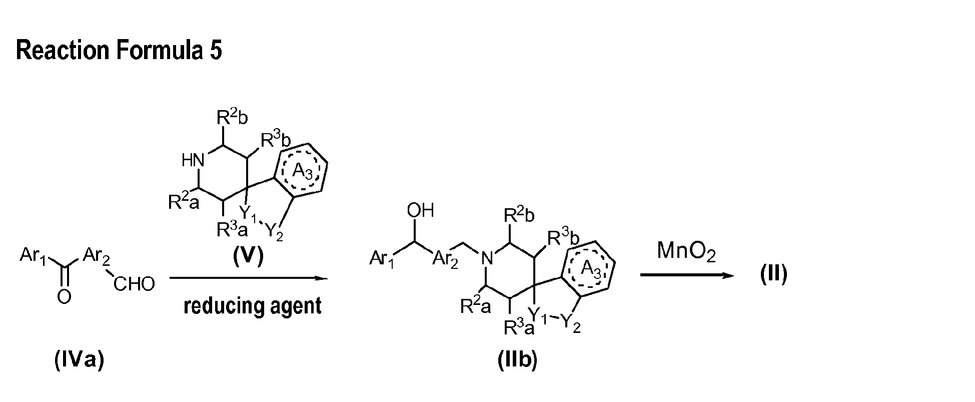

[In the formula, Ar₁, Ar₂, A₃, R^{1a}, R^{1b}, R^{2a}, R^{2b}, R^{3a}, R^{3b}, Y₁ and Y₂ have the same meanings as above.]

Specifically, a compound of formula (IVa) is reacted with a compound of formula (V) for reductive amination in a reaction solvent in the presence of a reducing agent to give a compound of formula (IIb). Next, the compound of formula (IIb) is oxidized to give a compound of formula (II).

The reaction of a compound of formula (V) with a compound of formula (IVa) may be attained generally in an equimolar ratio of the two or by using a small excessive amount of any one of the two.

The reducing agent includes, for example, sodium cyanoborohydride, sodium triacetoxyborohydride, zinc biscyanoborohydride, nickel biscyanoborohydride et al.

The amount of the reducing agent is, for example, from 1.0 mol to an excessive molar amount relative to 1 mol of the compound of formula (IVa), preferably from 1.0 to 5.0 mols.

The reaction solvent includes alcohols such as methanol, ethanol, propanol et al; ethers such as diethyl ether, THF, dioxane et al; halogenohydrocarbons such as methylene chloride, chloroform, dichloroethane et al; aromatic hydrocarbons such as benzene, toluene, chlorobenzene, xylene et al; DMF, acetonitrile et al; and their mixed solvents.

The reaction temperature is, for example, generally from -20 to 100°C, preferably from O°C to room temperature; and the reaction is completed generally from 5 minutes to 24 hours, preferably from 1 to 6 hours.

With the reductive amination, the carbonyl group in formula (IVa) is also reduced to give an alcohol (IIb), in which, therefore, the alcohol moiety is oxidized with manganese dioxide to give a compound of formula (II).

Specifically, the compound of formula (IIb) is oxidized in a known method using manganese dioxide in a halogenohydrocarbon such as methylene chloride, chloroform, carbon tetrachloride et al.

The amount of manganese dioxide to be used is, for example, from 1.0 to 10 mols per mol of the compound of formula (IIb), preferably from 3.0 to 5.0 mols.

The reaction temperature is, for example, from 0 to 60°C, preferably from 10 to 40°C; and the reaction is completed generally from 1 to 24 hours.

### Production Method 3-1:

Production Method 3-1 is method for producing a compound of formula (IVb).

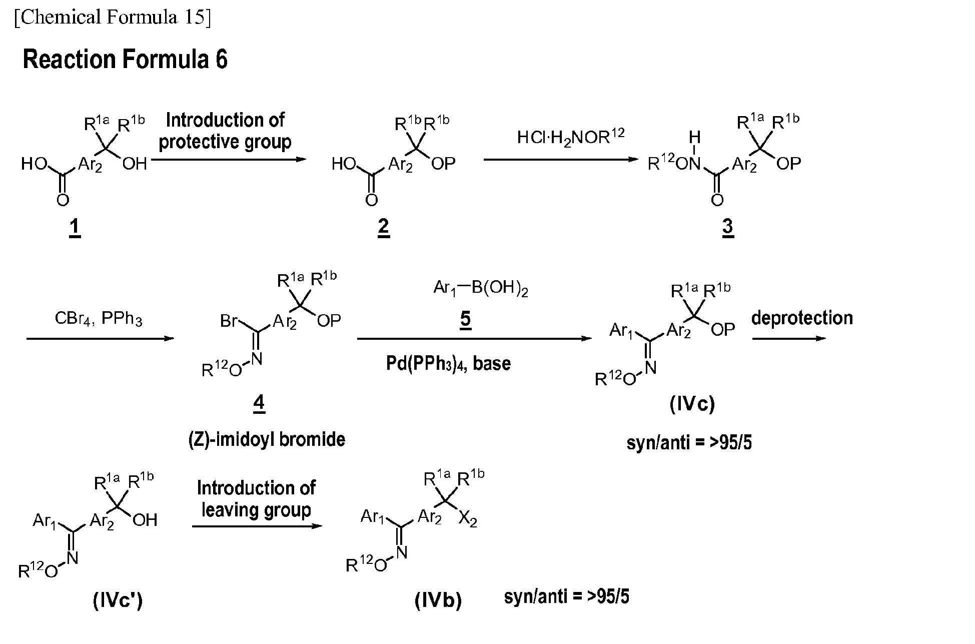

[In the formula, P represents a protective group for hydroxyl group; Ar₁, Ar₂, R¹², R^{1a}, R^{1b} and X₂ have the same meanings as above.]

The hydroxyl group in a compound 1 is protected in a known method to give a compound 2. Regarding its type, the protective group includes, for example, an acetyl group, a t-butyldimethylsilyl group et al.

Then the compound 2 is reacted with an O-alkylhydroxylamine (for example, methylhydroxylamine hydrochloride, ethylhydroxylamine hydrochloride, 2-fluoroethylhydroxylamine hydrochloride, [2-(aminooxy)ethoxy](t-butyl)dimethylsilane hydrochloride et al) according to Production Method 1-1, to give a compound 3. Next, the compound 3 is reacted under reflux in a solvent such as acetonitrile, in the presence of tetrabromomethane and triphenyl phosphine to give a compound 4.

The amount of tetrabromomethane to be used is, for example, from 1.5 to 3.0 mols per mol of the compound 3, preferably 1.5 mols; and the amount of triphenyl phosphine to be used is, for example, from 1.5 to 2.0 mols per mol of the compound 3, preferably 2.0 mols.

The obtained compound 4 is reacted with a compound 5 in a solvent such as toluene, in the presence of tetrakis(triphenylphosphine)palladium and a base, to give a compound of formula (IVc). In this, the compound (IVc) is obtained, keeping the stereochemistry of the imidoyl bromide 4, but depending on the type of Ar₁ and Ar₂, the (E)/(Z) expression of the compound is not uniform. Accordingly, for convenience, the case where Ar₁ and the oxime substituent are on the same side of the double bond is defined as syn, and the case where they are on the opposite side is as anti.

The base includes, for example, sodium carbonate, potassium carbonate, sodium hydrogencarbonate, sodium hydroxide, potassium hydroxide et al.

The amount of the compound 5 to be used is, for example, from 1.5 to 2.0 mols per mol of the compound 4, preferably 2.0 mols.

Regarding the amount of tetrakis(triphenylphosphine)palladium and the base to be used, the amount of tetrakis(triphenylphosphine)palladium is, for example, from 0.05 to 0.10 mols per mol of the compound 4, preferably 0.05 mols; and the amount of the base is, for example, from 1.5 to 2.0 mols, preferably 2.0 mols.

The reaction temperature is, for example, from 45 to 100°C, preferably 80°C; and the reaction is completed generally from 8 to 24 hours.

Then the hydroxyl-protective group P in the compound of formula (IVc) is removed in a known method to give a compound of formula (IVc'), and then the hydroxyl group in the compound of formula (IVc') is converted into a leaving group X (for example, methanesulfonyloxy group, p-toluenesulfonyloxy group, halogen atom et al) in a known method to give a compound of formula (IVb).

The compound 1 includes, for example, the following:

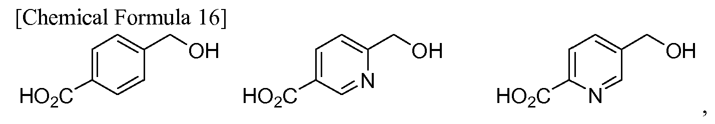

and the compound 5 includes, for example, the following:

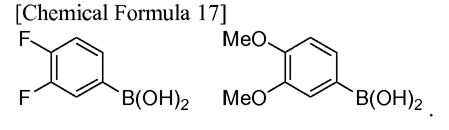

### Production Method 3-2:

Production Method 3-2 is method for producing a compound of formula (IV) or a compound of formula (IVb).

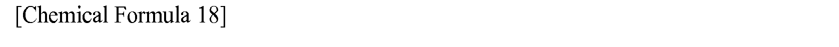

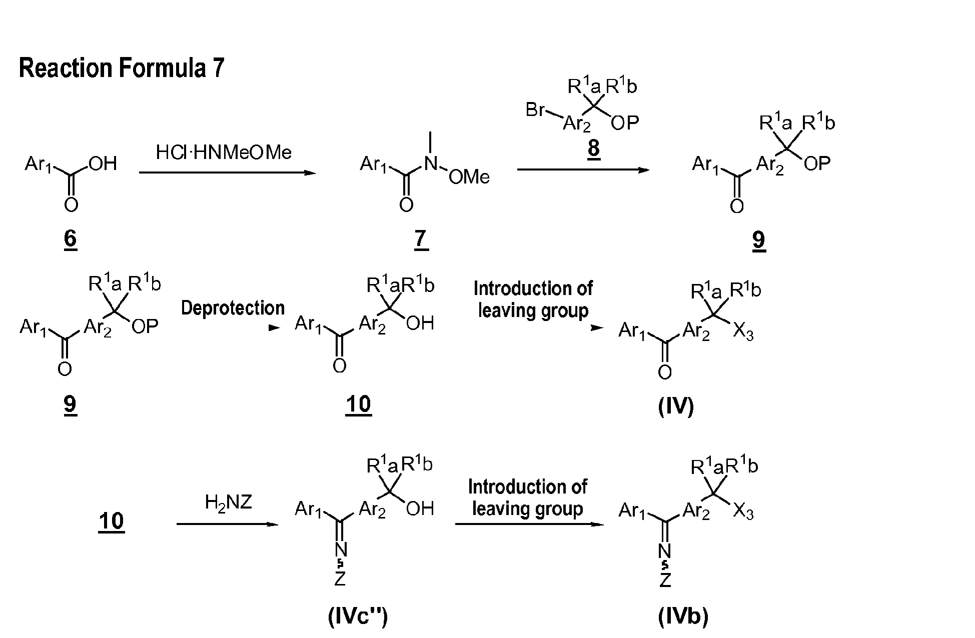

[In the formula, Ar₁, Ar₂, R^{1a}, R^{1b}, X₃ and P have the same meanings as above.]

Specifically, a compound 6 is condensed with N-methoxy-N-methylamine hydrochloride, for example, at 25°C to give a compound 7. Then the compound 7 is reacted with a compound 8 in the presence of a base such as n-butyllithium, isopropylmagnesium chloride et al, at -78 to 0°C to obtain a compound 9. The protective group in the compound 9 is removed in a known method to give a compound 10, and the hydroxyl group in the compound 10 is converted into a leaving group (for example, through reaction with mesyl chloride/triethylamine) to give a compound of formula (IV). Alternatively, the compound 10 is reacted according to Production Method 1-1 to give a compound of formula (IVc"), and the hydroxyl group is converted into a leaving group (for example, through reaction with mesyl chloride/triethylamine) to give a compound of formula (IVb).

The compound 6 includes, for example, the following:

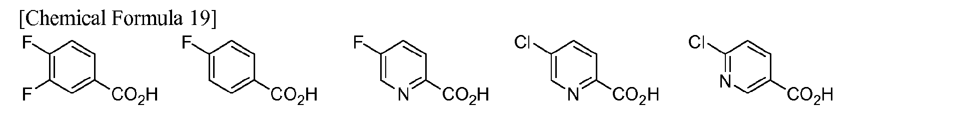

and the compound 8 includes, for example, the following:

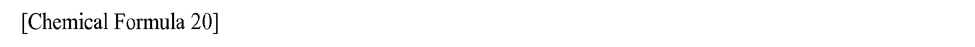

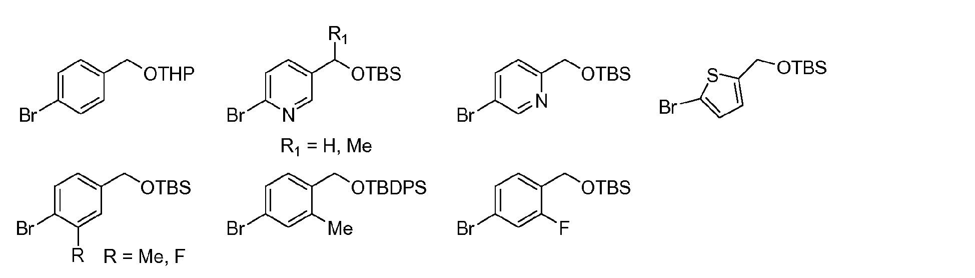

### Production Method 3-3:

Production Method 3-3 is an alternative method for producing the compound of formula (IV).

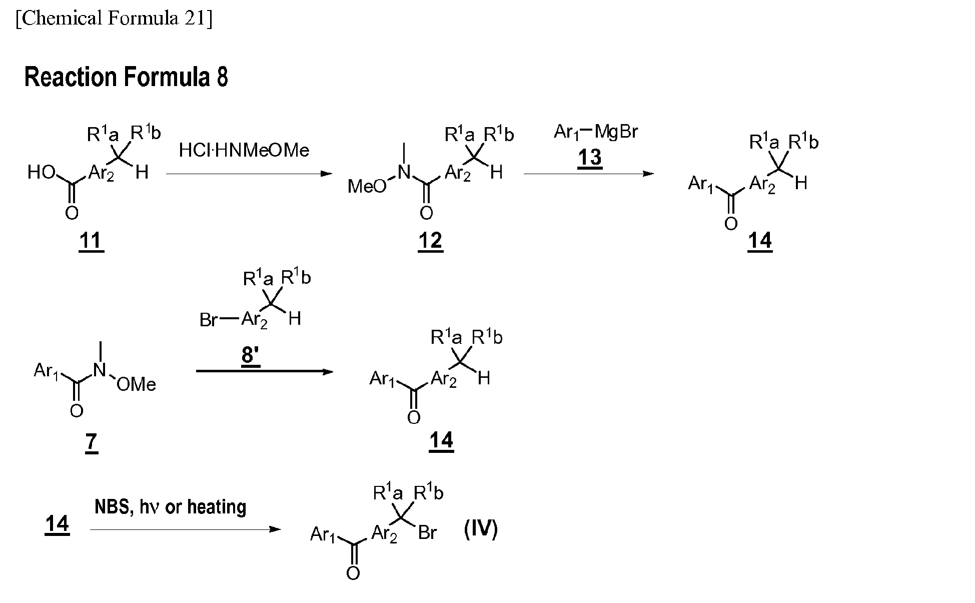

[In the formula, Ar₁, Ar₂, R^{1a} and R^{1b} have the same meanings as above.]

A compound 11 is reacted with N-methoxy-N-methylamine hydrochloride to give a compound 12, and the compound 12 is reacted with a Grignard reagent 13 at 0 to 25°C to give a compound 14.

Alternatively, a compound 7 is reacted with a compound 8' in the presence of a base such as n-butyllithium, isopropylmagnesium chloride et al, at -78 to 0°C to give a compound 14.

The obtained compound 14 is reacted with N-bromosuccinimide (NBS), for example, in carbon tetrachloride with irradiation with light to give a compound of formula (IV).

In place of irradiation with light, the system may be heated. In the case, the temperature is, for example, 30°C to the reflux temperature of the solvent. Further, the irradiation with light may be combined with heating.

The compound 11 includes, for example, the following:

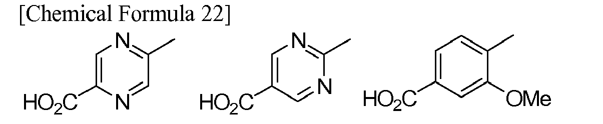

The compound 8' includes, for example, the following:

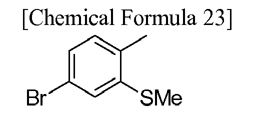

The compound 13 includes, for example, phenylmagnesium bromide, 4-fluorophenylmagnesium bromide, 3,4-difluorophenylmagnesium bromide et al.

### Production Method 3-4:

Production Method 3-4 is method for producing a compound of formula (IVa).

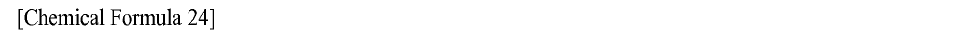

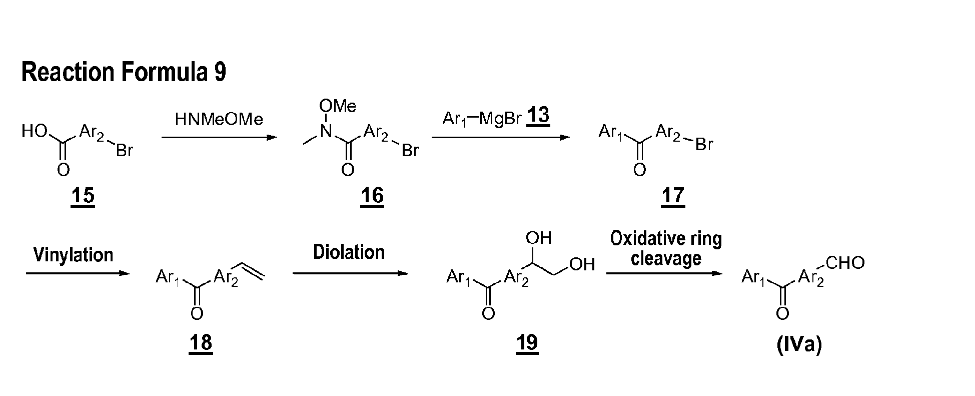

[In the formula, Ar₁ and Ar₂ have the same meanings as above.]

A compound 15 is condensed with N-methoxy-N-methylamine hydrochloride to give a compound 16, and the compound 16 is reacted with a Grignard reagent 13 to give a compound 17. Then, the compound 17 is cross-coupled (vinylation) with potassium vinyltrifluoroborate, using [1,1'-bis(diphenylphosphino)ferrocene]palladium(II) dichloride, to give a compound 18. The compound 18 is diolated with osmium tetraoxide and N-methylmorpholine-N-oxide, to give a compound 19. The diol moiety of the compound 19 is oxidatively cleaved in a known method using sodium periodate to give a compound of formula (IVa).

The compound 15 may be a commercial reagent, or may be prepared according to the methods described in Examples.

In the above production methods where the reactant substances have an amino group, an imino group, a hydroxyl group, a carboxyl group, an oxo group or a carbonyl group not participating in the reaction, the amino group, the hydroxyl group, the carboxyl group, the oxo group and the carbonyl group may be suitably protected with an amino-protective group, a hydroxy-protective group, a carboxyl-protective group, or an oxo or carbonyl-protective group, then the reaction in the described production method is attained, and after the reaction, the protective group may be removed.

The introduction and the removal of the protective group, through differing depending on the type of the protective group and the stability of the product compound, may be attained, for example, through solvolysis with acid or base, for example, according to methods described in literature [see Protective Groups in Organic Synthesis, T. W. Greene, John Wiley & Sons, 1981], concretely, for example, according to a method of processing with from 0.01 mol to a large excessive amount of an acid, preferably trifluoroacetic acid, formic acid, hydrochloric acid et al, or with from an equimolar amount to a large excessive amount of a base, preferably potassium hydroxide, calcium hydroxide et al; or through chemical reduction with a metal hydride complex, or through catalytic reduction with a palladium-carbon catalyst or a Raney-nickel catalyst et al.

Not specifically limited, the amino and imino-protective group may be any one having its function, and includes, for example, an aralkyl group such as a benzyl group, a p-methoxybenzyl group, a 3,4-dimethoxybenzyl group, an o-nitrobenzyl group, a p-nitrobenzyl group, a benzhydryl group, a trityl group et al; a lower alkanoyl group such as a formyl group, an acetyl group, a propionyl group, a butyryl group, a pivaloyl group et al; an arylalkanoyl group such as a benzoyl group, a phenylacetyl group, a phenoxyacetyl group et al; a lower alkoxycarbonyl group such as a methoxycarbonyl group, an ethoxycarbonyl group, a propyloxycarbonyl group; a tert-butoxycarbonyl group et al; an alkyloxycarbonyl group such as a benzyloxycarbonyl group, a p-nitrobenzyloxycarbonyl group, a phenethyloxycarbonyl group et al; a lower alkylsilyl group such as a trimethylsilyl group, a tert-butyldimethylsilyl group et al; a tetrahydropyranyl group; a trimethylsilylethoxymethyl group; a lower alkylsulfonyl group such as a methylsulfonyl group, an ethylsulfonyl group et al; an arylsulfonyl group such as a benzenesulfonyl group, a toluenesulfonyl group et al; and especially preferred are an acetyl group, a benzoyl group, a tert-butoxycarbonyl group, a trimethylsilylethoxymethyl group, a methylsulfonyl group et al.

Not specifically limited, the hydroxyl-protective group may be any one having its function, and includes, for example, a lower alkyl group such as a methyl group, an ethyl group, a propyl group, an isopropyl group, a tert-butyl group et al; a lower alkylsilyl group such as a trimethylsilyl group, a tert-butyldimethylsilyl group et al; a lower alkoxymethyl group such as a methoxymethyl group, a 2-methoxyethoxymethyl group et al; a tetrahydropyranyl group; a trimethylsilylethoxymethyl group; an aralkyl group such as a benzyl group, a p-methoxybenzyl group, a 2,3-dimethoxybenzyl group, an o-nitrobenzyl group, a p-nitrobenzyl group, a trityl group et al; an acyl group such as a formyl group, an acetyl group et al. Especially preferred are a methyl group, a methoxymethyl group, a tetrahydropyranyl group, a trityl group, a trimethylsilylethoxymethyl group, a tert-butyldimethylsilyl group, an acetyl group et al.

Not specifically limited, the carboxyl-protective group may be any one having its function, and includes, for example, a lower alkyl group such as a methyl group, an ethyl group, a propyl group, an isopropyl group, a tert-butyl group et al; a lower haloalkyl group such as a 2,2,2-trichloroethyl group et al; a lower alkenyl group such as a 2-propenyl group et al; an aralkyl group such as a benzyl group, a p-methoxybenzyl group, a p-nitrobenzyl group, a benzhydryl group, a trityl group et al. Especially preferred are a methyl group, an ethyl group, a tert-butyl group, a 2-propenyl group, a benzyl group, a p-methoxybenzyl group, a benzhydryl group et al.

Not specifically limited, the carbonyl-protective group may be any one having its function, and includes, for example, acetals and ketals such as ethylene ketal, dimethyl ketal, S,S'-dimethyl ketal et al.

The compounds of formula (I) or the compounds of formula (Ia) obtained in the manner as above may be readily isolated and purified in any conventional known separation method, for example, solvent extraction, recrystallization, column chromatography, or preparative thin-layer chromatography et al.

The compounds may be formed into pharmaceutically-acceptable salts thereof in an ordinary manner, or on the contrary, the salts may be converted into free compounds in an ordinary manner.

The effect of the compounds of the invention as an MCH receptor antagonist is shown, for example, by the following pharmacological test example.

### Pharmacological Test Example: MCH binding inhibition test

A human MCH-1R encoding cDNA sequence [FEBS Letters, Vol. 398, 253 (1996); Biochimica et Biophisica Acta, Vol. 1401, 216 (1998)] was cloned to a plasmid vector pEF/myc/cyto (Invitrogen Corporation). The obtained expression vector was transfected to host cells CHO-K1 (American Type Culture Collection) using Lipofectamine Plus Reagent (Life Technology Inc.) to provide MCH-1R expression cells.

Membrane samples prepared from the MCH-1R expression cells were incubated with each test compound and 50 pM of [¹²⁵I]MCH (NEN Co.), in an assay buffer (50 mM Tris buffer comprising 10 mM magnesium chloride, 2 mM ethylenediamine tetraacetate, 0.01 % bacitracin and 0.2 % bovine serum albumin; pH 7.4) at 25°C for an hour, followed by filtration through a glass filter CF/C (Wattman Co.). After washing the glass filter with 50 mM Tris buffer (pH 7.4) comprising 10 mM magnesium chloride, 2 mM ethylenediamine tetraacetate and 0.04 % Tween-20, the radioactive activity on the glass filter was measured. The non-specific binding was measured in the presence of 1 µM human MCH and 50 % inhibition concentration (IC₅₀ value) of each test compound to the specific [¹²⁵I]MCH binding was determined. The results are shown in Table 1.

**[Table 1]**

| Example No. | Structure | IC₅₀ (nM) | Example No. | Structure | IC₅₀ (nM) |
|---|---|---|---|---|---|
| 1-1 | 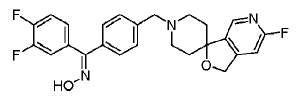 | 0.26 | 2-6 | 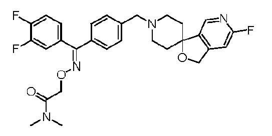 | 0.27 |
| 1-3-2 | 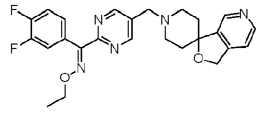 | 8.90 | 2-10-2 | 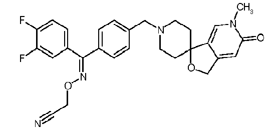 | 9.03 |
| 1-3-3 | 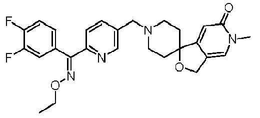 | 6.75 | 3-1-11 | 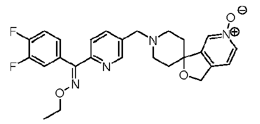 | 4.70 |
| 2-1-2 | 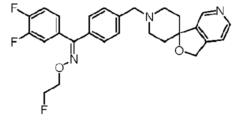 | 0.07 | 3-1-13 | 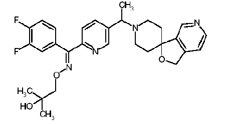 | 4.01 |
| 2-1-3 | 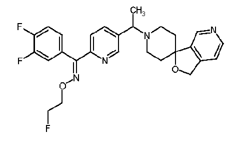 | 1.55 | 3-1-15 | 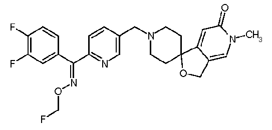 | 8.37 |

As in the above, the compounds of the invention strongly inhibit the binding of MCH to MCH-1R, and therefore exhibit an excellent effect as an MCH-1R antagonist.

Accordingly, the compounds of the invention are useful as a preventive or a remedy for various MCH-associated diseases, for example, metabolic disorders such as obesity, diabetes, hormone disorder, hyperlipidemia, gout, fatty liver; cardiovascular disorders such as stenocardia, acute or congestive heart failure, myocardial infarction, coronary atherosclerosis, hypertension, renal diseases, electrolyte abnormality; central nervous system or peripheral nervous system disorders such as bulimia, emotional disturbance, depression, anxiety, epilepsy, delirium, dementia, schizophrenia, attention-deficit hyperactivity disorder, memory impairment, sleep disorders, cognitive failure, dyskinesia, paresthesias, smell disorders, morphine tolerance, drug dependence, alcoholism; reproductive disorders such as infertility, preterm labor and sexual dysfunction; and other digestive disorders, respiratory disorders, cancer or pigmentation; especially as a preventive or a remedy for bulimia, obesity, diabetes, fatty liver, depression, anxiety.

### Pharmaceutical Composition Comprising Compound of Formula (I)

The compound of the invention can be orally or parenterally administered, and can be formulated into preparations suitable to the administration thereof, which may be used as pharmaceutical compositions for prevention or treatment for the above-mentioned diseases.

In its clinical use, the compound of the invention may be formulated into various preparations along with a pharmaceutically-acceptable carrier added thereto generally in accordance with the administration route thereof, and the thus-formulated pharmaceutical compositions may be administered. Various conventional additives known in the field of pharmaceutical preparations can be used. For example, gelatin, lactose, white sugar, titanium oxide, starch, crystalline cellulose, hydroxypropylmethyl cellulose, carboxymethyl cellulose, corn starch, microcrystalline wax, white petrolatum, magnesium aluminate metasilicate, anhydrous calcium phosphate, citric acid, trisodium citrate, hydroxypropyl cellulose, sorbitol, sorbitan fatty acid esters, polysorbate, sucrose fatty acid esters, polyoxyethylene, hardened castor oil, polyvinylpyrrolidone, magnesium stearate, light silicic anhydride, talc, vegetable oils, benzyl alcohol, gum arabic, propylene glycol, polyalkylene glycol, cyclodextrin and hydroxypropylcyclodextrin et al.

Preparations to be formed with those additives include, for example, solid preparations such as tablets, capsules, granules, powders, suppositories et al; and liquid preparations such as syrups, elixirs, injections et al. These may be formulated according to conventional methods known in the field of pharmaceutical preparations. The liquid preparations may also be in such a form that may be dissolved or suspended in water or in any other suitable medium in their use. Especially for injections, if desired, the preparations may be dissolved or suspended in physiological saline or glucose liquid, and a buffer or a preservative may be optionally added thereto.

The pharmaceutical compositions may contain the compound of the invention in an amount of from 1 to 99.9 % by weight, preferably from 1 to 60 % by weight of the composition. The compositions may further contain any other therapeutically-effective compounds.

In case where the compounds of the invention are used for prevention or treatment for the above-mentioned diseases, the dose and the dosing frequency may be varied, depending on the sex, the age, the body weight and the disease condition of the patient and on the type and the range of the intended remedial effect. In general, when orally administered, the dose may be from 0.001 to 50 mg/kg of body weight/day, and it may be administered at a time or in several times. The dose is preferably from about 0.01 to about 25 mg/kg/day, more preferably from about 0.05 to about 10 mg/kg/day.

As combination therapy, the compounds of the invention can be used in combination with drugs effective for hypertension, obesity-associated hypertension, hypertension-associated diseases, hypertrophy, left ventricular hypertrophy, metabolic disorders, obesity, obesity-associated diseases and the like (hereafter referred to as "co-drug"). Such drugs can be administered simultaneously, separately or in succession, for prevention or treatment of the above-mentioned diseases. When a compound of the invention is used simultaneously with one, two or more of co-drugs, they may be formulated into a medical preparation suited for single administration form. However, in combination therapy, a composition containing the compound of the invention and co-drugs may be administered to the object of medication in different packages, either simultaneously, separately or successively. They may be administered at time intervals.

The dose of the co-drug may be determined in accordance with the clinically adopted dose thereof, which can be suitably selected according to the individual object of medication, the administration route, the specific disease, the combination of drugs, and the like. The form of the co-drug for administration is not specifically limited, it may be combined with the compound of the invention when they are administered.

The administration mode includes, for example, the following: (1) A compound of the invention is combined with a co-drug to give a single preparation for single administration; (2) a compound of the invention and a co-drug are separately formulated into different two preparations, and the two preparations are simultaneously administered in one administration route; (3) a compound of the invention and a co-drug are separately formulated into different two preparations, and they are administered at different times in one and the same administration route; (4) a compound of the invention and a co-drug are separately formulated into different two preparations, and they are administered at the same time in two different administration routes; (5) a compound of the invention and a co-drug are separately formulated into different two preparations, and they are administered at different times in different administration routes (for example, a compound of the invention and a co-drug are administered in that order, or in an order contrary to this). The blend ratio of the compound of the invention and the co-drug may be suitably determined depending on the administration object, the administration route, and the disease for the administration.

The co-drug usable in the invention include, for example, remedy for diabetes, remedy for hyperlipidemia, remedy for hypertension, anti-obesity drug. Two or more such co-drugs may be combined in an adequate ratio and used.

The remedy for diabetes include, for example, 1) PPAR-γ agonists such as glitazones (e.g., ciglitazone, darglitazone, englitazone, isaglitazone (MCC-555) et al), pioglitazone, rosiglitazone, troglitazone, BRL49653, CLX-0921, 5-BTZD, GW-0207, LG-100641, LY-300512et al; 2) biguanides such as metformin, buformin, phenformin et al; 3) protein tyrosine phosphatase 1B inhibitors; 4) sulfonylureas such as acetohexamide, chloropropamide, diabinese, glibenclamide, glipizide, glyburide, glimepiride, gliclazide, glipentide, gliquidone, glisolamide, trazamide, tolubutamide et al; 5) meglitinides such as repaglinide, nateglinide et al; 6) α-glucoside hydroxylase inhibitors such as acarbose, adiposine, camiglibose, emiglitate, miglitol, voglibose, pradimicin-Q, salbostatin, CKD-711, MDL-25, 673, MDL-73, 945, MOR14 et al; 7) α-amylase inhibitors such as tendamistat, trestatin, A13688 et al; 8) insulin secretion promoters such as linogliride, A-4166 et al; 9) fatty acid oxidation inhibitors such as clomoxir, etomoxir et al; 10) A2 antagonists such as midaglizole, isaglidole, deriglidole, idazoxan, earoxan, fluparoxan et al; 11) insulin or insulin mimetics such as biota, LP-100, novalapid, insulindetermir, insulin lispro, insulin glargine, insulin zinc, Lys-Pro-insulin, GLP-1 (73-7), GLP1 amide (7-36) et al; 12) non-thiazolidinediones such as JT-501, farglitazar et al; 13) PPARα/γ dual-agonists such as MK-0767, CLX-0940, GW-1536, GW-1929, GW-2433, KRP-297, L-796449, LR-90 and SB219994 et al.

The remedy for hyperlipidemia include, for example, 1) bile acid absorption promoters such as cholesterylamine, colesevelem, colestipol, crosslinked dextran dialkylaminoalkyl derivatives, Colestid^{™}, LoCholest^{™}, Questran^{™} et al; 2) HMG-CoA reductase inhibitors such as atorvastatin, itavastatin, fluvastatin, lovastatin, pravastatin, rivastatin, rosuvastatin, simvastatin, ZD-4522 et al; 3) HMG-CoA synthase inhibitors; 4) cholesterol absorption inhibitors such as snatol ester, β-sitosterol, sterol glucoside, ezetimibe et al; 5) acyl-coenzyme A·cholesterol acyl transferase inhibitors such as avasimibe, eflucimibe, KY-505, SMP-709 et al; 6) CETP inhibitors such as JTT705, torcetrapib, CP532632, BAY-63-2149, SC-591, SC-795 et al; 7) squalane synthesis inhibitors; 8) antioxidants such as probucol; 9) PPAR-α agonists such as beclofibrate, benzafibrate, ciprofibrate, clofibrate, etofibrate, fenofibrate, gemcabene, gemfibrozil, GW-7647, BM-170744, LY-518674, fibric acid derivatives (e.g., Atromid^{™}, Lopid^{™}, Tricor^{™}) et al; 10) FXR receptor antagonists such as GW-4064, SR-103912 et al; 11) LXR receptor agonists such as GW3965, T9013137, XTCO-179628 et al; 12) lipoprotein synthesis inhibitors such as niacin; 13) renin-angiotensin system inhibitors; 14) microsome-triglyceride transport inhibitors; 15) bile acid resorption inhibitors such as BARA1453, SC435, PHA384640, S-435, AZD7706 et al; 16) PPAR-δ agonists such as GW501516, GW590735 et al; 17) triglyceride synthesis inhibitors; 18) MTTP inhibitors such as LAB687, CP346086 et al; 19) low-density lipoprotein receptor inducers; 20) squalane epoxidase inhibitors; 21) platelet agglutination inhibitors; 22) 5-lipoxygenase activated protein inhibitors such as MK-591.

The remedy for hypertension include, for example, 1) thiazide diuretics such as chlorothialidon, chlorothiazide, dichlorofenamide, hydrofluorothiazide, indapamide, hydrochlorothiazide et al; loop diuretics such as bumetanide, ethacrynic acid, flosemide, tolusemide et al; sodium diuretics such as amyloride, triamterene et al; aldosterone antagonist diuretics such as spironolactone, epilenone et al; 2) β-adrenaline blockers such as acebutolol, atenolol, betaxolol, bevantolol, bisoprolol, bopindolol, carteolol, carvedilol, celiprolol, esmolol, indenolol, metaprolol, nadolol, nebivolol, penbutolol, pindolol, probanolol, sotalol, tertatolol, tilisolol, timolol et al; 3) calcium channel blockers such as amlodipine, aranidipine, azelnidipine, barnidipine, benidipine, bepridil, cinaldipine, clevidipine, diltiazem, efonidipine, felodipine, gallopamil, isradipine, lacidipine, lemildipine, lercanidipine, nicardipine, nifedipine, nilvadipine, nimodepine, nisoldipine, nitrendipine, manidipine, pranidipine, verapamil et al; 4) angiotensin converting enzyme inhibitors such as benazepril, captopril, cilazapril, delapril, enalapril, fosinopril, imidapril, rosinopril, moexipril, quinapril, quinaprilat, ramipril, perindopril, perindoropril, quanipril, spirapril, tenocapril, trandolapril, zofenopril et al; 5) neutral endopeptidase inhibitors such as omapatrilat, cadoxatril, ecadotril, fosidotril, sampatrilat, AVE7688, ER4030 et al; 6) endotheline antagonists such as tezosentan, A308165, YM62899 et al; 7) vasodilators such as hydralazine, clonidine, minoxidil, nicotinyl alcohol et al; 8) angiotensin II antagonists such as candesartan, eporsartan, iribesartan, losartan, pratosartan, tasosartan, telmisartan, valsartan, EXP-3137, FI6828K, RNH6270 et al; 9) α/β adrenaline blockers such as nipradilol, arotinolol, amoslalol et al; 10) α1 blockers such as terazosin, urapidil, prazosin, bunazosin, trimazosin, doxazosin, naphthopidil, indolamin, WHIP164, XEN010 et al; 11) α2 agonists such as lofexidine, tiamenidine, moxonidine, rilmenidine, guanobenz et al; and 12) aldosterone inhibitors.

The anti-obesity drugs include, for example, 1) 5HT (serotonin) transporter inhibitors such as paroxetine, fluoxetine, fenfluramine, fluvoxamine, sertraline, imipramine et al; 2) norepinephrine transporter inhibitors such as GW320659, desipramine, talsupram, nomifensin et al; 3) cannabinoid-1 receptor 1 (CB-1) antagonists/inverse-agonists such as limonabant (Sanofi Synthelabo), SR-147778 (Sanofi Synthelabo), BAY-65-2520 (Bayer), SLV-319 (Solvey), as well as compounds disclosed in USP 5,532,237, USP 4,973,587, USP 5,013,837, USP 5,081,122, USP 5,112,820, USP 5,292,736, USP 5,624,941, USP 6,028,084, WO96/33159, WO98/33765, WO98/43636, WO98/43635, WO01/09120, WO01/96330, WO98/31227, WO98/41519, WO98/37061, WO00/10967, WO00/10968, WO97/29079, WO99/02499, WO01/58869, WO02/076949, WO01/64632, WO01/64633, WO01/64634, WO03/006007, WO03/007887 and EP-658546 et al; 4) ghrelin antagonists such as compounds disclosed in WO01/87355, WO02/08250 et al; 5) histamine(H3) antagonists/inverse-agonists such as thioperamide, 3-(1H-imidazol-4-yl)propylN-(pentenyl)carbonate, clobenpropit, iodofenpropit, imoproxyfen, GT2395, A331440, compounds disclosed in WO02/15905, O-[3-(1H-imidazol-4-yl)propanol] carbamate, piperazine-containing H3-receptor antagonists (Lazewska, D. et al., Pharmazie, 56: 927-32 (2001), benzophenone derivatives (Sasse, A. et al., Arch. Pharm. (Weinheim) 334: 45-52 (2001)), substituted N-phenylcarbamates (Reidemeister, S. et al., Pharmazie, 55: 83-6 (2000)), proxyfen derivatives (Sasse, A. et al., J. Med. Chem., 43: 3335-43 (2000)) et al; 6) MCH-1R antagonists such as T-226296 (Takeda), SNP-7941 (Synaptic), other compounds disclosed in WO01/82925, WO01/87834, WO02/051809, WO02/06245, WO02/076929, WO02/076947, WO02/04433, WO02/51809, WO02/083134, WO02/094799, WO03/004027 and JP-A-2001-226269 et al; 7) MCH-2R agonists/antagonists; 8) NPY1 antagonists such as isopropyl 3-chloro-5-(1-(6-[2-(5-ethyl-4-methyl-thiazol-2-yl)-ethyl]-4-morpholinyl-4-yl-piridin-2-ylamino)-ethyl)phenyl]carbamate, BIBP3226, BIBO3304, LY-357897, CP-671906, GI-264879, and other compounds disclosed in USP 6,001,836, WO96/14307, WO01/23387, WO99/51600, WO01/85690, WO01/85098, WO01/85173 and WO01/89528 et al; 9) NPY5 antagonists such as 152804, GW-569180A, GW-594884A, GW-587081X, GW-548118X, FR235,208, FR226928, FR240662, FR252384, 1229U91, GI-264879A, CGP71683A, LY-377897, LY366377, PD-160170, SR-120562A, SR-120819A, JCF-104, H409/22, and other compounds disclosed in USP 6,140,354, USP 6,191,160, USP 6,258,837, USP 6,313,298, USP 6,337,332, USP 6,329,395, USP 340,683, USP 6,326,375, USP 6,329,395, USP 6,337,332, USP 6,335,345, EP-01010691, EP-01044970, WO97/19682, WO97/20820, WO97/20821, WO97/20822, WO97/20823, WO98/27063, WO00/107409, WO00/185714, WO00/185730, WO00/64880, WO00/68197, WO00/69849, WO01/09120, WO01/14376, WO01/85714, WO1/85730, WO01/07409, WO01/02379, WO01/02379, WO01/23388, WO01/23389, WO01/44201, WO01/62737, WO01/62738, WO01/09120, WO02/20488, WO02/22592, WO02/48152, WO02/49648, WO02/094789, and compounds disclosed in Norman et al., J. Med. Chem., 43:4288-4312(2000) et al; 10) leptins such as human recombinant leptin (PEG-OB, Hoffman La Roche), recombinant methionylleptin (Amgen) et al; 11) leptin derivatives such as compounds disclosed in USP 5,552,524, USP 5,552,523, USP 5,552,522, USP 5,521,283, WO96/23513, WO96/23514, WO96/23515, WO96/23516, WO96/23517, 96/23518, WO96/23519 and WO96/23520 et al; 12) opioid antagonists such as nalmefen (Revex^{™}), 3-methoxynaltorexone, naloxone, naltorexone, compounds disclosed in WO00/21509 et al; 13) orexin antagonists such as SB-334867A, and other compounds disclosed in WO01/96302, WO01/68609, WO02/51232, WO02/51838 and WO03/023561 et al; 14) bombesin receptor subtype-3 agonists; 15) cholecystokinin A (CCK-A) agonists such as AR-R15849, GI-181771, JMV-180, A-71378, A-71623, SR-146131, and other compounds disclosed in USP 5,739,106 et al; 16) CNTF (ciliary neurotrophic factors) such as GI-181771 (Glaxo-Smith Kline), SR146131 (Sanofi Synthelabo), butabindide, PD170,292, PD 149164 (Pfizer) et al; 17) CNTF derivatives such as axokine (Regeneron), and other compounds disclosed in WO94/09134, WO98/22128, WO99/43813 et al; 18) growth hormone secretion receptor agonists such as NN703, hexarelin, MK-0677, SM-130686, CP-424,391, L-692,429, L-163,255, and compounds disclosed in USP 6,358,951, US Patent Application Nos. 2002/049196, 2002/022637, WO01/56592, WO02/32888 et al; 19) serotonin receptor-2C agonists such as BVT933, DPCA37215, IK264, PNU22394, WAY161503, R-1065, YM348, and other compounds disclosed in USP 3,914,250, WO02/36596, WO02/48124, WO02/10169, WO01/66548, WO02/44152, WO02/51844, WO02/40456 and WO02/40457 et al; 20) melanocortin-3 receptor agonists; 21) melanocortin-4 receptor agonists such as CHIR86036 (Chiron), ME-10142, ME-10145 (Melacure), and other compounds disclosed in WO99/64002, WO00/74679, WO01/991752, WO01/74844, WO01/70708, WO01/70337, WO01/91752, WO02/059095, WO02/059107, WO02/059108, WO02/059117, WO02/12166, WO02/11715, WO02/12178, WO02/15909, WO02/068387, WO02/068388, WO02/067869, WO03/007949 and WO03/009847 et al; 22) monoamine resorption inhibitors such as sibutramine (Meridia™/Reductil™) and its salts, and other derivatives disclosed in USP 4,746,680, USP 4,806,570, USP 5,436,272, US Patent Application No. 2002/0006964, WO01/27068 and WO01/62341 et al; 23) serotonin re-uptake inhibitors such as dexfenfluramine, fluoxetine, and other compounds disclosed in USP 6,365,633, WO01/27060 and WO01/162341 et al; 24) glucagon-like peptide-1 agonists; 25) topiramate (Topimax™); 26) phytopharm compound 57 (e.g., CP644,673); 27) acetyl CoA carboxylase-2 (ACC2) inhibitors; 28) β-adrenalin receptor-3 agonists such as AD9677/TAK677 (Dai-Nippon Pharmaceutical/Takeda Chemical), CL-316,243, SB418790, BRL-37344, L-796568, BMS-196085, BRL-35135A, CGP12177A, BTA-243, W427353, Trecadrine, Zeneca D7114, SR59119A, and other compounds disclosed in USP 5,705,515, USP 5,451,677, WO01/74782 and WO02/32897 et al; 29) diacylglycerol acyltransferase-1 inhibitors; 30) diacylglycerol acyltransferase-2 inhibitors, 31) fatty acid synthesis inhibitors such as carulenin, C75; 32) phosphodiesterase inhibitors such as theophylline, pentoxiphylline zaprinast, sildenafil, amrinone, milrinone, cilostamide, rolipram and cilomilast et al; 33) thyroid hormone-β agonists such as KB-2611 (KaroBio BMS), and other compounds disclosed in WO02/15845, JP-A-2000-256190 et al; 34) UCP (uncoupling protein)-1, 2, or 3 activators such as phytanic acid, 4-[(E)-2-(5,6,7,8-tetrahydro-5,5,8,8-tetramethyl-2-naphthalenyl)-1-propenyl]benzoic acid (TTNPB), retinoic acid, and other compounds disclosed in WO99/00123 et al; 35) acylestrogens such as oleoylestrone, and other compounds disclosed in del Mar-Grasa, M. et al., Obesity Research, 9:202-9 (2001) et al; 36) glucocorticoid antagonists; 37) 11-β-hydroxysteroid dehydrogenase-1 inhibitors such as BVT3498, BVT2733, and other compounds disclosed in WO01/90091, WO01/90090, WO01/90092 et al; 38) stearoyl-CoA desaturase-1 inhibitors; 39) dipeptidyl peptidase-IV inhibitors such as isoleucine thiazolidide, valine pyrrolidide, NVP-DPP728, AF237, P93/01, TSL225, TMC-2A/2B/2C, FE999011, P9310/K364, VIP0177, SDZ274-444, and other compounds disclosed in WO03/004498, WO03/004496, EP1258476, WO02/083128, WO02/062764, WO03/000250, WO03/002530, WO03/002531, WO03/002553, WO03/002593, WO03/000180 and WO03/000181 et al; 40) lipase inhibitors such as tetrahydroliptatin (Orlistat/Xenical™), Triton WR1339, RHC80267, lipstatin, teasaponin, diethylumbelliferyl phosphate, FL-386, WAY-121898, Bay-N-3176, valilactone, esteracin, ebelactone A, ebelactone B, RHC80267, and other compounds disclosed in WO01/77094, USP 4,598,089, USP 4,452,813, USP 5,512,565, USP 5,391,571, USP 5,602,151, USP 4,405,644, USP 4,189,438 and USP 4,242,453 et al; 41) fatty acid transporter inhibitors; 42) dicarboxylate transporter inhibitors; 43) glucose transporter inhibitors; 44) phosphate transporter inhibitors.

Those combined drugs are obtained by combining a compound of the invention with one, two or more of the above co-drugs. Furthermore, the combined drugs are useful for prevention or treatment of metabolic disorders, when combined with one, two or more drugs selected from the group consisting of remedy for diabetes and remedy for hyperlipidemia. Combinations containing, in particular, remedy for hypertension and anti-obesity agent are useful for prevention or treatment of metabolic disorders with synergistic effect, when remedy for diabetes and/or remedy for hyperlipidemia are added thereto.

On the other hand, the compound of the invention may be combined with an antipsychotic. An antipsychotic, especially an atypical antipsychotic is known to have a side effect of body weight increase; and the compound of the invention, when combined with such an antipsychotic, is useful for retarding the side effect. The antipsychotic includes, for example, olanzapine, Risperidone, quetiapine, Ziprasidone, aripiprazole, Paliperidone, Clozapine et al. Using an antipsychotic, as combined with a compound of the invention, may improve the level of metabolic parameters such as the level of blood pressure, glucose and lipid level that may be elevated by the antipsychotic. The above-mentioned methods may apply to the conditions of dose, administration subject, administration route and administration form.

### BEST MODE FOR CARRYING OUT THE INVENTION

The invention is described more concretely with reference to the following Examples, however, the invention should not be limited to the examples. As silica gel for column, used was Wakogel^{™} C-200 (Wako Pure Chemical Industries); as a filled silica gel column, used was FLASH+^{™} cartridge, KP-Sil or FPNH, FLASH12+M, FLASH25+S, FLASH25+M or FLASH40+M (Biotage Japan); and as a preparative thin-layer chromatogram, used was Kieselgel 60F254 (Merck). For mass spectrometry, used was QuattroII (Micromass).

### EXAMPLES

Reference Example 1 - Production of ketoalcohol 10:
Reference Example 1-1:

### Production of 3,4-difluoro-N-methoxy-N-methylbenzamide

At 0°C, N,O-dimethylhydroxylamine hydrochloride (12.3 g), 1-hydroxybenzotriazole hydrate (14.5 g), N-[3-(dimethylamino)propyl]-N'-ethylcarboximide hydrochloride (18.2 g) and triethylamine (44.5 mL) were added to a chloroform solution (115 mL) of 3,4-difluorobenzoic acid (10.0 g), and stirred overnight at room temperature. Water was added to the reaction liquid, and extracted with chloroform. The organic layer was washed with saturated saline, and dried with anhydrous sodium sulfate. After the organic layer was concentrated under reduced pressure, the residue was purified through silica gel column chromatography (hexane/ethyl acetate = 8/2 to 7/3) to obtain the entitled compound (12.8 g) as a colorless oil.
ESI-MS Found: m/z 202[M+H]⁺

### Reference Example 1-2:

### Production of 4-fluoro-N-methoxy-N-methylbenzamide

In the same manner as in Reference Example 1-1 but using 4-fluorobenzoic acid (2.00 g), the entitled compound (2.09 g) was obtained as a colorless oil.
ESI-MS Found: m/z 184[M+H]⁺

### Reference Example 1-3:

### Production of 5-fluoro-N-methoxy-N-methylpyridine-2-carboxamide

In the same manner as in Reference Example 1-1 but using 5-fluoropyridine-2-carboxylic acid (4.50 g), the entitled compound (5.88 g) was obtained as a colorless oil.
ESI-MS Found: m/z 185[M+H]⁺

### Reference Example 1-4:

### Production of 5-chloro-N-methoxy-N-methylpyridine-2-carboxamide

In the same manner as in Reference Example 1-1 but using 5-chloropyridine-2-carboxylic acid (1.00 g), the entitled compound (1.03 g) was obtained as a colorless oil.
ESI-MS Found: m/z 201 [M+H]⁺

### Reference Example 1-5:

### Production of 6-chloro-N-methyl-N-(methyloxy)-3-pyridinecarboxamide

In the same manner as in Reference Example 1-1 but using 6-chloropyridine-3-carboxylic acid (5.00 g), the entitled compound (4.96 g) was obtained as a colorless oil.
ESI-MS Found: m/z 201 [M+H]⁺

### Reference Example 1-6:

### Production of 1-(6-bromopyridin-3-yl)ethanol

At -78°C, a hexane solution (29.3 mL) of 2.59 M n-butyllithium was added to a diethyl ether solution (225 mL) of 2,5-dibromopyridine (15.0 g), and stirred for 1 hour. To it was added dimethylacetamide (7.30 mL), and stirred at 0°C for 1 hour. At 0°C, sodium borohydride (4.79g) and methanol (50.0 mL) were added to it, and stirred for 1 hour. To the reaction liquid, added were aqueous acetic acid solution and aqueous 2 N sodium hydroxide solution, and then extracted with chloroform. The organic layer was washed with water, and dried with anhydrous sodium sulfate. After the organic layer was concentrated under reduced pressure, the residue was purified through silica gel column chromatography (hexane/ethyl acetate = 7/3 to 5/5) to obtain the entitled compound (7.62 g) as a brown oil.
ESI-MS Found: m/z 202[M+H]⁺

### Reference Example 1-7:

### Production of 2-bromo-5-(1-{[tert-butyl(dimethyl)silyl]oxy}ethyl)pyridine

At 0°C, t-butyldimethylsilyl chloride (4.93 g) was added to a DMF solution (20.0 mL) of the compound (6.00 g) obtained in Reference Example 1-6 and imidazole (4.05 g), and stirred overnight at room temperature. Water was added to the reaction liquid, and extracted with diethyl ether. The organic layer was washed with water, and then dried with anhydrous sodium sulfate. After the organic layer was concentrated under reduced pressure, the residue was purified through silica gel column chromatography (hexane/ethyl acetate = 98/2 to 95/5) to obtain the entitled compound (7.91 g) as a colorless oil. ESI-MS Found: m/z 316[M+H]⁺

### Reference Example 1-8:

### Production of [(4-bromo-3-fluorobenzyl)oxy](tert-butyl)dimethylsilane

In the same manner as in Reference Example 1-7 but using 4-bromo-3-fluorophenylmethanol (1.12 g), the entitled compound (1.68 g) was obtained as a colorless oil. ESI-MS Found: m/z 319[M+H]⁺

### Reference Example 1-9:

### Production of [(4-bromo-2-fluorobenzyl)oxy](tert-butyl)dimethylsilane

In the same manner as in Reference Example 1-7 but using 4-bromo-2-fluorophenylmethanol (2.08 g), the entitled compound (3.11 g) was obtained as a colorless oil. ESI-MS Found: m/z 319[M+H]⁺

### Reference Example 1-10:

### Production of (3,4-difluorophenyl){4-[(tetrahydro-2H-pyran-2-yloxy)methyl]phenyl}methanone

At -78°C, a hexane solution (19.0 mL) of 1.6 M n-butyllithium was added to a THF solution (50.0 mL) of 2-[(4-bromobenzyl)oxy]tetrahydro-2H-pyran (6.04 g), and stirred for 1 hour. To this was added, at -78°C, a THF solution (12.0 mL) of the amide (5.00 g) obtained in Reference Example 1-1, and stirred for 1 hour, then further stirred at 0°C for 1 hour. Aqueous ammonium chloride solution was added to the reaction liquid, and extracted with ethyl acetate. The organic layer was washed with saturated saline, and dried with anhydrous sodium sulfate. After the organic layer was concentrated under reduced pressure, the residue was purified through silica gel column chromatography (hexane/ethyl acetate = 95/5 to 8/2) to obtain the entitled compound (4.06 g) as a yellow oil.
ESI-MS Found: m/z 333[M+H]⁺

### Reference Example 1-11:

### Production of (3,4-difluorophenyl)[4-(hydroxymethyl)phenyl]methanone

At room temperature, p-toluenesulfonic acid hydrate (22.9 mg) was added to a methanol solution (60.0 mL) of the compound (4.00 g) obtained in Reference Example 1-10, and stirred for 5.5 hours. Aqueous sodium hydrogencarbonate solution was added to the reaction liquid, and extracted with ethyl acetate. The organic layer was washed with saturated saline, and dried with anhydrous sodium sulfate. After the organic layer was concentrated under reduced pressure, the residue was purified through silica gel column chromatography (hexane/ethyl acetate = 9/1 to 6/4) to obtain the entitled compound (2.75 g) as a white solid.
ESI-MS Found: m/z 249[M+H]⁺

### Reference Example 1-12:

Using various amides 7 obtained in the above Reference Example 1 and arylbromides 8 (including compounds known in literature), the following ketoalcohols 10 were obtained under the conditions shown in Reference Examples 1-10 and 1-11 and Tables 2-1 and 2-2.

### [Table 2]

**Table 2-1 - Production of Ketoalcohols 10**

| 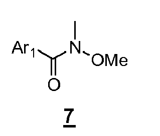 | | 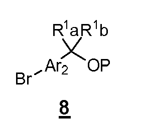 | | 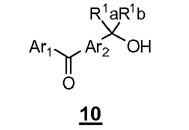 | ESI-MS |
|---|---|---|---|---|---|
| 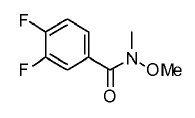 | a) | 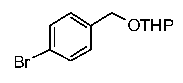 | d) | 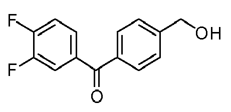 | 249 [M+H]⁺ |
| | a) | 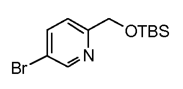 | e) | 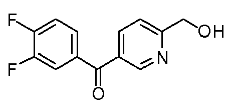 | 250 [M+H]⁺ |
| | b) | 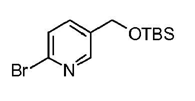 | e) | 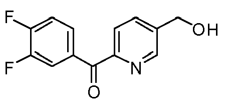 | 250 [M+H]⁺ |
| | b) | 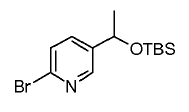 | e) | 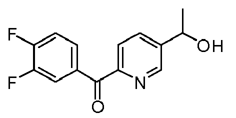 | 264 [M+H]⁺ |
| | c) | 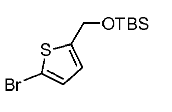 | e) | 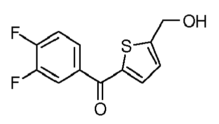 | 255 [M+H]⁺ |
| | a) | 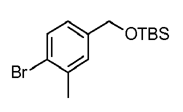 | e) | 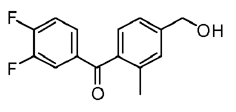 | 263 [M+H]⁺ |
| | a) | 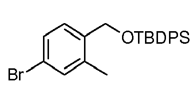 | e) | 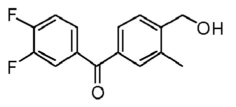 | 263 [M+H]⁺ |
| | a) | 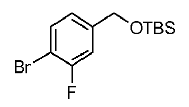 | e) | 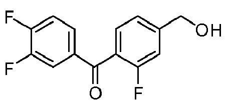 | 267 [M+H]⁺ |
| | a) | 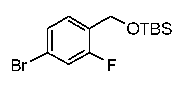 | e) | 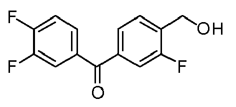 | 267 [M+H]⁺ |

| | | | | | |
|---|---|---|---|---|---|
| conditions: a) *n*-BuLi,THF, -78 °C, 1-2 h b) *n*-BuLi, toluene- hexane, -78 °C, 1 h, 0 °C, 18 h c) *i*-PrMgCI, THF, 0 °C, 1 h, r.t., 13 h d) *p*-TsOH, MeOH, r.t., 1 h e) TBAF, THF, 0 °C, 1-2 h | | | | | |

### [Table 3]

**Table 2-2 - Production of Ketoalcohols 10**

| 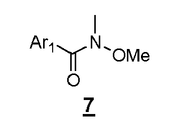 | | 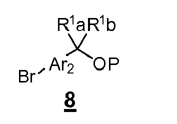 | | 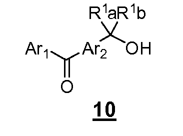 | ESI-MS |
|---|---|---|---|---|---|
| 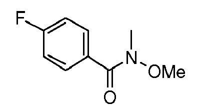 | a) | 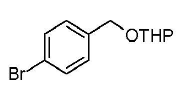 | b) | 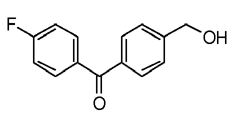 | 231 [M+Hl⁺ |
| 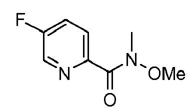 | a) | 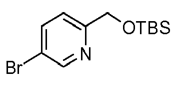 | c) | 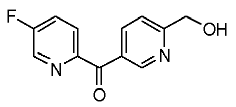 | 233 [M+H]⁺ |
| 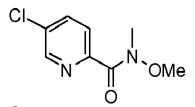 | a) | 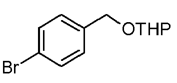 | b) | 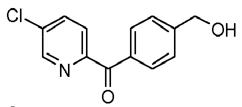 | 248 [M+H]⁺ |
| 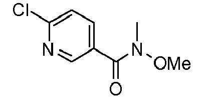 | a) | 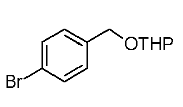 | b) | 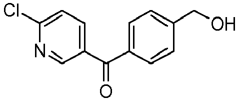 | 248 [M+H]⁺ |

| | | | | | |
|---|---|---|---|---|---|
| conditions: a) *n*-BuLi, THF, -78 °C, 1-2 h b) *p*-TsOH, MeOH, r.t., 1 h c) TBAF, THF, 0 °C, 1-2 h | | | | | |

### Reference Example 2: Production of bromide (IV)

### Reference Example 2-1:

### Production of N-methoxy-N,5-dimethylpyrazine-2-carboxamide

In the same manner as in Reference Example 1-1 but using 5-methylpyrazine-2-carboxylic acid (10.0 g), the entitled compound (13.2 g) was obtained as a yellow oil.
ESI-MS Found: m/z 182[M+H]⁺

### Reference Example 2-2:

### Production of (3,4-difluorophenyl)(5-methylpyrazin-2-yl)methanone

At -78°C, a THF solution (33.2 mL) of 0.5 M 3,4-difluorophenylmagnesium bromide was added to a THF solution (50.0 mL) of the compound (3.00 g) obtained in Reference example 2-1, and stirred for 2 hours. This was heated up to 0°C, and aqueous ammonium chloride solution and aqueous sodium hydrogencarbonate solution were added to it, and extracted with ethyl acetate. The organic layer was washed with saturated saline, and dried with anhydrous sodium sulfate. After the organic layer was concentrated under reduced pressure, the residue was purified through silica gel column chromatography (hexane/ethyl acetate = 8/2) to obtain the entitled compound (1.85 g) as a pale yellow solid.
ESI-MS Found: m/z 235[M+H]⁺

### Reference Example 2-3:

### Production of [5-(bromomethyl)pyrazin-2-yl](3,4-difluorophenyl)methanone

At room temperature, N-bromosuccinimide (495 mg) was added to a carbon tetrachloride solution (29.0 mL) of the compound (500 mg) obtained in Reference Example 2-2, and heated under reflux for 3.5 hours with irradiation with light. Using a Kiriyama funnel, this was filtered, and the filtrate was concentrated under reduced pressure. The residue was purified through preparative thin-layer silica gel chromatography (hexane/ethyl acetate = 7/3) to obtain the entitled compound (291 mg) as a yellow oil. ESI-MS Found: m/z 313[M+H]⁺

### Reference Example 2-4:

### Production of N-methoxy-N,2-dimethlpyrimidine-5-carboxamide

In the same manner as in Reference Example 1-1 but using 2-methylpyridine-5-carboxylic acid (3.00 g), the entitled compound (3.12 g) was obtained as a yellow oil.
ESI-MS Found: m/z 182[M+H]⁺

### Reference Example 2-5:

### Production of 4-bromo-1-methyl-2-(methylthio)benzene

At room temperature, sodium methyl sulfide (1.84 g) was added to a DMF solution (15.0 mL) of 4-bromo-2-fluorotoluene (3.00 mL), and stirred at 50°C for 18 hours. Aqueous sodium hydrogencarbonate solution was added to it, and extracted with diethyl ether. The organic layer was washed with water and saturated saline, and dried with anhydrous sodium sulfate. After the organic layer was concentrated under reduced pressure, the residue was purified through silica gel column chromatography (hexane/diethyl ether = 200/1 to 50/1) to obtain the entitled compound (2.51 g) as a pale yellow oil.
ESI-MS Found: m/z 218[M+H]⁺

### Reference Example 2-6:

Using various amides 12 obtained in the above Reference Example 2 or arylbromides 7 (including compounds known in literature), the following bromides (IV) were obtained under the conditions shown in Reference Examples 1-10, 2-2 and 2-3 and Table 3.

### [Table 4]

**Table 3 - Production of Bromides (IV)**

| 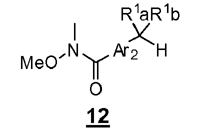 | | 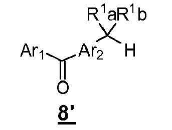 | | 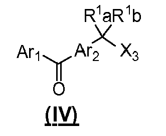 | ESI-MS |
|---|---|---|---|---|---|
| 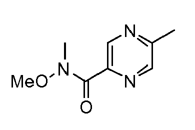 | a) | 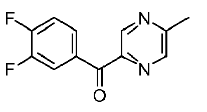 | c) | 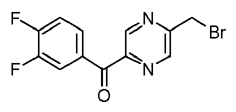 | 313 [M+H]⁺ |
| 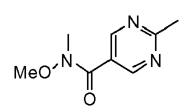 | a) | 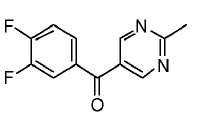 | c) | 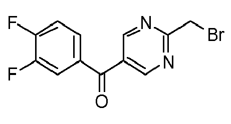 | 313 [M+H]⁺ |
| 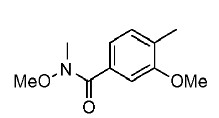 | a) | 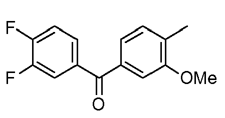 | c) | 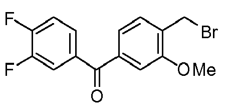 | 341 [M+H]⁺ |
| 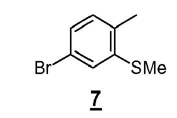 | b) | 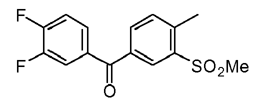 | c) | 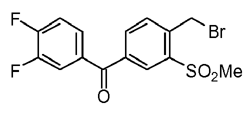 | 389 [M+H]⁺ |

| | | | | | |
|---|---|---|---|---|---|
| conditions: a) 3,4-Difluorophenyl magnesium bromide, THF, 0 °C, 1-3 h b) (1) *n*-BuLi, THF, -78 °C, 1 h, then 3,4-Difluoro-N-methoxy-N-methylbenzamide (2) mCPBA, CHCl₃, 0 °C, 4 h c) NBS, h ν , CHCl₃, reflux, 2-5 h or NBS, AIBN, CHCl₃, reflux, 2-5 h | | | | | |

### Reference Example 3 - Production of Aldehydes (IVa)

### Production Example 3-1:

### Production of 5-bromo-N-methoxy-N-methylpyrimidine-2-carboxamide

In the same manner as in Reference Example 1-1 but using 5-bromopyrimidine-2-carboxylic acid (21.6 g), the entitled compound (19.3 g) was obtained as a pale yellow solid. ESI-MS Found: m/z 246[M+H]⁺

### Reference Example 3-2:

### Production of (5-bromopyrimidin-2-yl)(3,4-difluorophenyl)methanone

In the same manner as in Reference Example 2-2 but using the compound (10.0 g) obtained in Reference Example 3-1, the entitled compound (11.7 g) was obtained as a pale yellow solid. ESI-MS Found: m/z 299[M+H]⁺

### Reference Example 3-3:

### Production of (3,4-difluorophenyl)(5-vinylpyrimidin-2-yl)methanone

Triethylamine (6.28 mL) was added to an isopropyl alcohol solution (223 mL) of potassium vinyltrifluoroborate (7.19 g), [1,1'-bis(diphenylphosphino)ferrocene]palladium(II) dichloride (654 mg) and the compound (11.0 g) obtained in Reference Example 3-2, and stirred at 80°C for 2 hours. The reaction liquid was concentrated under reduced pressure, and the residue was purified through silica gel column chromatography (hexane/ethyl acetate = 85/15 to 5/5). The resulting solid was washed with diisopropyl ether/hexane (5/5) mixed solvent to obtain the entitled compound (7.80 g) as a pale brown solid.
ESI-MS Found: m/z 247[M+H]⁺

### Reference Example 3-4:

### Production of (3,4-difluorophenyl)[5-(1,2-dihydroxyethyl)pyrimidin-2-yl]methanone

At room temperature, aqueous 0.1 M osmium tetraoxide solution (3.05 mL) was added to an acetonitrile (90.0 mL)/water (30.0 mL) mixed solution of the compound (7.51 g) obtained in Reference Example 3-3 and 4-methylmorpholine-N-oxide (7.37 g), and stirred overnight. After cooled to 0°C, aqueous sodium thiosulfate solution was added to the reaction liquid, and extracted with ethyl acetate. The organic layer was washed with aqueous 1 N hydrochloric acid solution, aqueous sodium hydrogencarbonate solution and saturated saline, and dried with anhydrous sodium sulfate. After the organic layer was concentrated under reduced pressure, the residue was purified through silica gel column chromatography (chloroform/methanol = 95/5 to 9/1) to obtain the entitled compound (6.62 g) as a brown solid.
ESI-MS Found: m/z 281[M+H]⁺

### Reference Example 3-5:

### Production of 2-(3,4-difluorobenzoyl)pyrimidine-5-carbaldehyde

At 0°C, an aqueous solution (50.0 mL) of sodium periodate (3.90 g) was added to a THF solution (150 mL) of the compound (4.26 g) obtained in Reference Example 3-4, and stirred overnight at room temperature. After cooled to 0°C, aqueous ammonium chloride solution was added to the reaction liquid, and extracted with ethyl acetate. The organic layer was washed with saturated saline, and dried with anhydrous sodium sulfate. The organic layer was concentrated under reduced pressure to obtain a crude product (3.78 g) of the entitled compound as a brown solid.
ESI-MS Found: m/z 249[M+H]⁺

### Reference Example 4 - Production of Amine (V):

### Reference Example 4-1:

### Production of 1'-benzyl-6-chloro-3H-spiro[furo[3,4-c]pyridine-1,4'-piperidin]-3-one

At -78°C, a hexane solution (500 mL) of 2.55 M n-butyllithium was dropwise added to a THF solution (900 mL) of 2,2,6,6-tetramethylpiperazine (162 mL). After stirred for 1 hour, a THF solution (450 mL) of 6-chloronicotinic acid (50.2 g) was dropwise added to it at -78°C over 1 hour. After further stirred for 2 hours, a THF solution (150 mL) of 1-benzyl-4-piperidone (177 mL) was dropwise added to it at -78°C. After stirred for 2 hours, water (500 mL) was added to it, and heated up to room temperature. The aqueous layer was separated, and the organic layer was extracted with aqueous 1 N sodium hydroxide solution. The obtained aqueous layer was extracted with diethyl ether, and the aqueous layer was made acidic (pH∼1) by adding concentrated hydrochloric acid. After stirred for 1 hour, the precipitated red brown solid was collected through filtration, washed with water, and dissolved in ethyl acetate. The organic layer was washed with aqueous saturated sodium hydrogencarbonate solution and dried with anhydrous sodium sulfate. After the organic layer was concentrated under reduced pressure, the residue was washed with diethyl ether to obtain the entitled compound (60.1 g) as a pale yellow solid. ESI-MS Found: m/z 329[M+H]⁺

### Reference Example 4-2:

### Production of 1'-benzyl-6-chloro-3H-spiro[furo[3,4-c]pyridine-1,4'-piperidin]-3-ol

At -78°C, a toluene solution (300 mL) of 1.0 M diisobutylaluminium hydride was dropwise added to a chloroform solution (600 mL) of the compound (54.8 g) obtained in Reference Example 4-1. After stirred for 1 hour, sodium sulfate 10-hydrate (300 g) was added to it, and stirred at room temperature for 10 hours. The solid was separated through filtration, and the filtrate was concentrated under reduced pressure. The residue was washed with a mixed solvent of diethyl ether/hexane (1/1) to obtain the entitled compound (46.2 g) as a pale yellow solid.
ESI-MS Found: m/z 331[M+H]⁺

### Reference Example 4-3:

### Production of 1'-benzyl-6-chloro-3H-spiro[furo[3,4-c]pyridine-1,4'-piperidine]

At 0°C, sodium borohydride (7.71 g) and boron trifluoride-ether complex (52.6 mL) were added to an acetonitrile solution (800 mL) of the compound (41.2 g) obtained in Reference Example 4-2. This was heated up to 60°C and stirred for 1 hour, and then again cooled to 0°C, and aqueous 1 N sodium hydroxide solution was added to it (pH∼10). This was extracted with ethyl acetate, and the organic layer was washed with saturated saline, and dried with anhydrous sodium sulfate. After concentrated under reduced pressure, methanol (200 mL) and 1 N hydrochloric acid (400 mL) were added to the residue, and stirred at 100°C for 10 hours. Methanol was concentrated under reduced pressure, and aqueous 5 N sodium hydroxide solution was added to it at 0°C (pH∼10). The precipitated solid was collected through filtration, and washed with water. This was dried under reduced pressure to obtain the entitled compound (40.0 g) as a pale yellow solid.
ESI-MS Found: m/z 315[M+H]⁺

### Reference Example 4-4:

### Production of 1'-benzyl-6-(benzyloxy)-3H-spiro[furo[3,4-c]pyridine-1,4'-piperidine]hydrochloride

At 0°C, sodium hydride (3.81 g) was added to a DMF solution (150 mL) of the compound (20.0 g) obtained in Reference Example 4-3 and benzyl alcohol (7.55 mL). This was heated up to 60°C and stirred for 12 hours, and then sodium hydride (3.81 g) was again added thereto at 0°C. This was heated up to 90°C, stirred for 5 hours, then cooled to 0°C, and aqueous 2 N hydrochloric acid solution (160 mL) was added thereto. The precipitated solid was collected through filtration, and washed with water. This was dried under reduced pressure to obtain the entitled compound (23.2 g) as a white solid.
ESI-MS Found: m/z 387[M+H]⁺

### Reference Example 4-5:

### Production of tert-butyl 6-oxo-5,6-dihydro-1'H,3H-spiro[furo[3,4-c]pyridine-1,4'-piperidine]-1'-carboxylate

At 0°C in a nitrogen atmosphere, 20 % palladium hydroxide-carbon (1.50 g) was added to a methanol solution (125 mL) of the compound (5.00 g) obtained in Reference Example 4-4 and cyclohexene (11.9 mL). This was heated up to 85°C, stirred for 2 hours, then again cooled to 0°C, triethylamine (4.93 mL) and di-t-butyl dicarbonate (3.09 g) were added thereto. After stirred for 12 hours at room temperature, the solid was separated through filtration. The filtrate was concentrated under reduced pressure, and the residue was dissolved in chloroform, and aqueous saturated sodium hydrogen carbonate solution was added thereto. Extracted with chloroform, the organic layer was washed with saturated saline, and dried with anhydrous sodium sulfate. After the organic layer was concentrated under reduced pressure, the residue was washed with a mixed solvent of diethyl ether/hexane (1/1) to obtain the entitled compound (3.38 g) as a white solid.
ESI-MS Found: m/z 307[M+H]⁺

### Reference Example 4-6:

### Production of tert-butyl 5-methyl-6-oxo-5,6-dihydro-1'H,3H-spiro[furo[3,4-c]pyridine-1,4'-piperidine]-1'-carboxylate

At 0°C, cesium fluoride (37.5 g) and iodomethane (6.16 mL) were added to a DMF solution (500 mL) of the compound (24.4 g) obtained in Reference Example 4-5. After stirred at room temperature for 12 hours, the reaction liquid was poured into saturated saline, and extracted with chloroform. The organic layer was dried with anhydrous sodium sulfate, and concentrated under reduced pressure. The residue was purified through silica gel column chromatography (chloroform/methanol = 80/1 to 20/1) to obtain the entitled compound (21.8 g) as a white solid.
ESI-MS Found: m/z 321[M+H]⁺

### Reference Example 4-7:

### Production of tert-butyl 5-ethyl-6-oxo-5,6-dihydro-1'H,3H-spiro[furo[3,4-c]pyridine-1,4'-piperidine]-1'-carboxylate and tert-butyl 6-ethoxy-1'H,3H-spiror[furor3,4-c]pyridine-1,4'-piperidine]-1'-carboxylate

In the same manner as in Reference Example 4-6 but using the compound (500 mg) obtained in Reference Example 4-5 and ethyl iodide, the entitled compound, N-ethyl form (210 mg) was obtained as a white solid and the entitled compound O-ethyl form (180 mg) was obtained as a white amorphous substance.
ESI-MS Found: m/z 335[M+H]⁺

### Reference Example 4-8:

### Production of 6-fluoro-1H-spiro[furo[3,4-c]pyridine-3,4'-piperidine]hydrochloride

20 % palladium hydroxide-carbon (21.1 g) was added to a methanol solution (300 mL) of 7-chloro-6-fluoro-1'-(phenylmethyl)-1H-spiro[furo[3,4-c]pyridine-3,4'-piperidine] (20.0 g). After stirred overnight in a hydrogen atmosphere (1 atmospheric pressure) at room temperature, the reaction system was purged with nitrogen. After filtered through Celite, the filtrate was concentrated under reduced pressure to obtain the entitled compound (14.1 g) as a white solid.
ESI-MS Found: m/z 209[M+H]⁺

### Reference Example 4-9:

### Production of tert-butyl 6-fluoro-1H,1'H-spiro[furo[3,4-c]pyridine-3,4'-piperidine]-1'-carboxylate

At 0°C, triethylamine (24.3 mL) and di-t-butyl dicarbonate (12.6 g) were added to a 1,4-dioxane (100 mL)/water (100 mL) mixed solution of the compound (14.1 g) obtained in Reference Example 4-8, and then stirred overnight at room temperature. Water was added to the reaction liquid, and extracted with ethyl acetate. The organic layer was washed with saturated saline, and dried with anhydrous sodium sulfate. After the organic layer was concentrated under reduced pressure, the precipitated solid was washed with hexane to obtain the entitled compound (15.4 g) as a white solid.
ESI-MS Found: m/z 309[M+H]⁺

### Reference Example 4-10:

### Production of tert-butyl 6-oxo-5,6-dihydro-1H,1'H-spiro[furo[3,4-c]pyridine-3,4'-piperidine]-1'-carboxylate

Aqueous 6 N hydrochloric acid solution (100 mL) was added to the compound (14.9 g) obtained in Reference Example 4-8, and heated under reflux for 2 hours. The reaction liquid was concentrated under reduced pressure, and to a 1,4-dioxane (60.0 mL)/water (60.0 mL) mixed solution of the residue, triethylamine (25.4 mL) and di-t-butyl dicarbonate (14.4 g) were added at 0°C. After stirred overnight at room temperature, water was added to the reaction liquid, and extracted with ethyl acetate. The organic layer was washed with saturated saline, and dried with anhydrous sodium sulfate. After the organic layer was concentrated under reduced pressure, the residue was purified through silica gel column chromatography (chloroform/methanol = 95/5) to obtain the entitled compound (18.2 g) as a white amorphous substance.
ESI-MS Found: m/z 307[M+H]⁺

### Reference Example 4-11:

### Production of tert-butyl 5-methyl-6-oxo-5,6-dihydro-1H,1'H-spiro[furo[3,4-c]pyridine-3,4'-piperidine]-1'-carboxylate

In the same manner as in Reference Example 4-6 but using the compound (3.00 g) obtained in Reference Example 4-10, the entitled compound (2.26 g) was obtained as a white solid. ESI-MS Found: m/z 321 [M+H]⁺

### Reference Example 4-12:

### Production of 1'-benzyl-4-chloro-3H-spiro[furo[3,4-c]pyridine-1,4'-piperidin]-3-one

In the same manner as in Reference Example 4-1 but using 2-chloronicotinic acid (10.0 g), the entitled compound (7.01 g) was obtained as a pale brown solid.
ESI-MS Found: m/z 329[M+H]⁺

### Reference Example 4-13:

### Production of 1'-benzyl-4-chloro-3H-spiro[furo[3,4-c]pyridine-1,4'-piperidin]-3-ol

In the same manner as in Reference Example 4-2 but using the compound (6.50 g) obtained in Reference Example 4-12, the entitled compound (6.50 g) was obtained as a white solid. ESI-MS Found: m/z 331 [M+H]⁺

### Reference Example 4-14:

### Production of 1'-benzyl-4-chloro-3H-spiro[furo[3,4-c]pyridine-1,4'-piperidine]

In the same manner as in Reference Example 4-3 but using the compound (6.50 g) obtained in Reference Example 4-13, the entitled compound (4.68 g) was obtained as a white solid. ESI-MS Found: m/z 315[M+H]⁺

### Reference Example 4-15:

### Production of 1'-benzyl-4-[(4-methoxybenzyl)oxy]-3H-spiro[furo[3,4-c]pyridine-1,4'-piperidine]

In the same manner as in Reference Example 4-4 but using the compound (3.29 g) obtained in Reference Example 4-14 and 4-methoxybenzyl alcohol, the entitled compound (3.37 g) was obtained as a brown oil.
ESI-MS Found: m/z 417[M+H]⁺

### Reference Example 4-16:

### Production of tert-butyl 4-oxo-4,5-dihydro-1'H,3H-spiro[furo[3,4-c]pyridine-1,4'-piperidinel-1'-carboxylate

10 % palladium-carbon (1.93 g) was added to an ethanol solution (36.0 mL) of the compound (1.51 g) obtained in Reference Example 4-15 and 1,4-cyclohexadiene (3.39 mL), and heated under reflux for 4 days. After filtered through Celite, the filtrate was concentrated under reduced pressure. To a 1,4-dioxane (9.00 mL)/water (9.00 mL) mixed solution of the residue, triethylamine (1.02 mL) and dit-butyl dicarbonate (792 mg) were added at 0°C, and stirred overnight at room temperature. After the reaction liquid was concentrated under reduced pressure, the residue was purified through silica gel column chromatography (chloroform/methanol = 95/5 to 9/1) to obtain the entitled compound (752 mg) as a white solid.
ESI-MS Found: m/z 307[M+H]⁺

### Reference Example 4-17:

### Production of tert-butyl 5-methyl-4-oxo-4,5-dihydro-1'H,3H-spiro[furo[3,4-c]pyridine-1,4'-piperidine]-1'-carboxylate

In the same manner as in Reference Example 4-6 but using the compound (500 mg) obtained in Reference Example 4-16, the entitled compound (466 mg) was obtained as a white solid. ESI-MS Found: m/z 321[M+H]⁺

### Reference Example 4-18:

### Production of 1'-benzyl-7-chloro-1H-spiro[furo[3,4-c]pyridine-3,4'-piperidine]-6-amine

Aqueous 25 % ammonia solution (125 mL) was added to an isopropyl alcohol solution (125 mL) of 7-chloro-6-fluoro-1'-(phenylmethyl)-1H-spiro[furo[3,4-c]pyridine-3,4'-piperidine] (50.0 g), and heated overnight under stirring at 140°C in a sealed tube. The reaction liquid was concentrated under reduced pressure, cooled to 0°C, and made acidic (pH ∼3) by adding aqueous 10 % phosphoric acid solution. The aqueous layer was extracted with diethyl ether, and aqueous 4 N sodium hydroxide solution was added to the aqueous layer (pH, ∼10). This was extracted with chloroform, then the organic layer was washed with saturated saline, dried with anhydrous sodium sulfate. After the organic layer was concentrated under reduced pressure, the precipitated solid was washed with diisopropyl ether to obtain the entitled compound (39.7 g) as a white solid.
ESI-MS Found: m/z 330[M+H]⁺

### Reference Example 4-19:

### Production of 1H-spiro[furo[3,4-c]pyridine-3,4'-piperidine]-6-amine hydrochloride

In the same manner as in Reference Example 4-8 but using the compound (10.0 g) obtained in Reference Example 4-18, the entitled compound (6.56 g) was obtained as a white solid.
ESI-MS Found: m/z 206[M+H]⁺

### Reference Example 4-20:

### Production of tert-butyl 6-amino-1H,1'H-spiro[furo[3,4-c]pyridine-3,4'-piperidine]-1'-carboxylate

In the same manner as in Reference Example 4-9 but using the compound (10.0 g) obtained in Reference Example 4-19, the entitled compound (10.6 g) was obtained as a white amorphous substance.
ESI-MS Found: m/z 306[M+H]⁺

### Reference Example 4-21:

### Production of tert-butyl 6-(acetylamino)-1H,1'H-spiro[furo[3,4-c]pyridine-3,4'-piperidine]-1'-carboxylate

At 0°C, acetic anhydride (4.85 mL) was added to a pyridine solution (86.0 mL) of the compound (10.5 g) obtained in Reference Example 4-20, then stirred overnight at room temperature. Water and aqueous sodium hydrogencarbonate solution were added to the reaction liquid, and extracted with diethyl ether. The organic layer was washed with water and saturated saline, and dried with anhydrous sodium sulfate. After the organic layer was concentrated under reduced pressure, the residue was purified through silica gel column chromatography (chloroform/methanol = 98/2 to 95/5) to obtain the entitled compound (12.1 g) as a white amorphous substance.
ESI-MS Found: m/z 348[M+H]⁺

### Reference Example 4-22:

### Production of N-1H-spiro[furo[3,4-c]pyridine-3,4'-piperidin]-6-ylacetamide hydrochloride

At 0°C, 4 N hydrogen chloride-ethyl acetate (20 mL) was added to the compound (1.00 g) obtained in Reference Example 4-21, and stirred for 30 minutes. The precipitated solid was washed with diethyl ether to obtain the entitled compound (808 mg) as a white solid.
ESI-MS Found: m/z 248[M+H]⁺

### Reference Example 4-23:

### Production of 1'-benzyl-6,7-dichloro-3H-spiro[furo[3,4-c]pyridine-1,4'-pipendine]-3-one

In the same manner as in Reference Example 4-1 but using 5,6-dichloronicotinic acid (5.00 g), the entitled compound (3.17 g) was obtained as a white solid.
ESI-MS Found: m/z 363[M+H]⁺

### Reference Example 4-24:

### Production of 1'-benzyl-6,7-dichloro-3H-spiro[furo[3,4-c]pyridine-1,4'-piperidine]-3-ol

In the same manner as in Reference Example 4-2 but using the compound (2.00 g) obtained in Reference Example 4-23, the entitled compound (2.01 g) was obtained as a white solid.
ESI-MS Found: m/z 365[M+H]⁺

### Reference Example 4-25:

### Production of 1'-benzyl-6,7-dichloro-3H-spiro[furo[3,4-c]pyridine-1,4'-piperidine]

In the same manner as in Reference Example 4-3 but using the compound (1.74 g) obtained in Reference Example 4-24, the entitled compound (1.46 g) was obtained as a white solid. ESI-MS Found: m/z 349[M+H]⁺

### Reference Example 4-26:

### Production of N-(1'-benzyl-7-chloro-3H-spiro[furo[3,4-c]pyridine-1,4'-piperidin]-6-yl)acetamide

Aqueous 25 % ammonia solution (4.00 mL) was added to an isopropyl alcohol solution (4.00 mL) of the compound (400 mg) obtained in Reference Example 4-25, and heated under stirring at 190°C for 3 days in a sealed tube. The reaction liquid was concentrated under reduced pressure, and to a pyridine solution (10.0 mL) of the residue, acetic anhydride (165 µL) was added at 0°C, and then stirred overnight at room temperature. Water and aqueous sodium hydrogencarbonate solution were added to the reaction liquid, and extracted with diethyl ether. The organic layer was washed with water and saturated saline, and dried with anhydrous sodium sulfate. After the organic layer was concentrated under reduced pressure, the residue was purified through preparative thin-layer silica gel chromatography (chloroform/methanol = 9/1) to obtain the entitled compound (325 mg) as a white amorphous substance. ESI-MS Found: m/z 372[M+H]⁺

### Reference Example 4-27:

### Production of N-3H-spiro[furo[3,4-c]pyridine-1,4'-piperidin]-6-ylacetamide hydrochloride

10 % palladium-carbon (287 mg) was added to an ethanol solution (2.5 mL) of the compound (100 mg) obtained in Reference Example 4-26 and 1,4-cyclohexadiene (250 µL), and heated under reflux for 3 days. After filtered through Celite, the filtrate was concentrated under reduced pressure to obtain the entitled compound (79.4 mg) as a white solid.
ESI-MS Found: m/z 248[M+H]⁺

### Reference Example 4-28:

### Production of 1'-benzyl-1H-spiro[furo[3,4-c]pyridine-3,4'-piperidin]-1-one

At -78°C, a hexane solution (500 mL) of 2.71 M n-butyllithium was dropwise added to a THF solution (4.00 L) ofN-phenyl-4-pyridinecarboxamide (131 g). After stirred for 2.5 hours, a THF solution (650 mL) of 1-benzyl-4-piperidone (125 g) was dropwise added to it at -78°C, and stirred for 1 hour. After warmed up to -20°C, aqueous 1 N sodium hydroxide solution (1.00 L) was added to it, and concentrated under reduced pressure. After extracted with ethyl acetate, the aqueous layer was made acidic (pH ∼1) by adding concentrated hydrochloric acid. After stirred for 1 hour, this was neutralized (pH ∼8) with aqueous 12 % potassium carbonate solution. The precipitated solid was collected through filtration, washed with water, and dissolved in chloroform. This was washed with aqueous saturated sodium hydrogencarbonate solution, and dried with anhydrous sodium sulfate. The organic layer was concentrated under reduced pressure to obtain the entitled compound (152 g) as a white solid.
ESI-MS Found: m/z 295[M+H]⁺

### Reference Example 4-29:

### Production of 1'-benzyl-1H-spiro[furo[3,4-c]pyridine-3,4'-piperidin]-1-ol

In the same manner as in Reference Example 4-2 but using the compound (150 g) obtained in Reference Example 4-28, the entitled compound (160 g) was obtained as a white solid.
ESI-MS Found: m/z 297[M+H]⁺

### Reference Example 4-30:

### Production of 1-benzyl-4-[4-(hydroxymethyl)pyridin-3-yl]piperidin-4-ol

At 0°C, sodium borohydride (19.8 g) was added to an ethanol solution (2.70 L) of the compound (155 g) obtained in Reference Example 4-29, and stirred at room temperature for 1.5 hours. Again cooled to 0°C, methanol (2.70 L) was added to it, and then aqueous 6 N hydrochloric acid solution (770 mL) was added thereto. Aqueous 4 N sodium hydroxide solution (1.10 L) was added to it (pH ∼10), and concentrated under reduced pressure. Methanol (500 mL) and THF (1.50 L) were added to the residue, and extracted with ethyl acetate. The organic layer was washed with saturated saline, and then dried with anhydrous sodium sulfate. After the organic layer was concentrated under reduced pressure, the precipitated solid was collected through filtration. Washed with diisopropyl ether, the entitled compound (134 g) was obtained as a white solid.
ESI-MS Found: m/z 299[M+H]⁺

### Reference Example 4-31:

### Production of 1'-benzyl-1H-spiro[furo[3,4-c]pyridine-3,4'-piperidine]

At 0°C, methanesulfonyl chloride (41.1 mL) was added to a chloroform solution (2.00 L) of the compound (132 g) obtained in Reference Example 4-30 and triethylamine (185 mL), and stirred at room temperature for 1.5 hours. After concentrated under reduced pressure, water (300 mL) was added to it, and extracted with ethyl acetate. Aqueous ammonium chloride solution was added to the organic layer, washed with saturated saline, and dried with anhydrous sodium sulfate. The organic layer was concentrated under reduced pressure, and the residue was purified through silica gel column chromatography (hexane, ethyl acetate/chloroform = 4/1, ethyl acetate/chloroform/methanol = 9/1/1) to obtain the entitled compound (110 g) as a brown oil.
ESI-MS Found: m/z 281[M+H]⁺

### Reference Example 4-32:

### Production of 1H-spiro[furo[3,4-c]pyridine-3,4'-piperidine]dihydrochloride

20 % palladium hydroxide-carbon (50.0 g) was added to a methanol solution (1.20 L) of the compound (89.6 g) obtained in Reference Example 4-31, and stirred overnight in a hydrogen atmosphere (1 atmospheric pressure) at room temperature, and then the reaction system was purged with nitrogen. After filtered through Celite, the filtrate was concentrated under reduced pressure to obtain a residue (56.6 g) as a pale yellow oil. Methanol (100 mL) and ethyl acetate (300 mL) were added to it, and at 0°C, 4 N hydrogen chloride-ethyl acetate (160 mL) was added thereto. After concentrated under reduced pressure, diisopropyl ether (100 mL) and ethyl acetate (300 mL) were added to it, and stirred at room temperature for 15 minutes. The precipitated solid was collected through filtration, and washed with ethyl acetate to obtain the entitled compound (70.2 g) as a white solid.
ESI-MS Found: m/z 191 [M+H]⁺

### Reference Example 4-33:

### Production of tert-butyl 1H,1'H-spiro[furo[3,4-c]pyridine-3,4'-piperidine]-1'-carboxylate

In the same manner as in Reference Example 4-9 but using the compound (2.00 g) obtained in Reference Example 4-32, the entitled compound (2.31 g) was obtained as a white solid.
ESI-MS Found: m/z 291 [M+H]⁺

### Reference Example 4-34:

### Production of tert-butyl 1H, 1'H-spiro[furo[3,4-c]pyridine-3,4'-piperidine]-1'-carboxylate 5-oxide

At room temperature, m-chloroperbenzoic acid (387 mg) was added to a chloroform solution (10 mL) of the compound (500 mg) obtained in Reference Example 4-33, and stirred overnight at room temperature. Aqueous saturated sodium hydrogencarbonate solution was added to the reaction liquid, and extracted with chloroform. The organic layer was dried with anhydrous sodium sulfate, and the organic layer was concentrated under reduced pressure. Diisopropyl ether was added to the residue, and the resulting solid was collected through filtration to obtain the entitled compound (405 mg) as a white solid.
ESI-MS Found: m/z 307[M+H]⁺

### Reference Example 4-35:

### Production of tert-butyl 1'H,3H-spiro[furo[3,4-c]pyridine-1,4'-piperidine-1'-carboxylate

In the same manner as in Reference Examples 4-28 to 4-33 but using 3-pyridinecarboxylic acid, the entitled compound was obtained as a white solid.
ESI-MS Found: m/z 291[M+H]⁺

### Reference Example 4-36:

### Production of tert-butyl 1'H,3H-spiro[furo[3,4-c]pyridine-1,4'-piperidine-1'-carboxylate 5-oxide

In the same manner as in Reference Example 4-34 but using the compound (500 mg) obtained in Reference Example 4-35, the entitled compound (435 mg) was obtained as a pale yellow solid. ESI-MS Found: m/z 307[M+H]⁺

### Reference Example 4-37:

### Production of 1,1-dimethylethyl 6-bromo-1H, 1'H-spiro[furo[3,4-c]pyridine-3,4'-piperidine]-1'-carboxylate

25 % hydrogen bromide-acetic acid (20.0 mL) was added to the compound (5.00 g) obtained in Reference Example 4-8, and heated overnight under reflux at 120°C. The reaction liquid was concentrated under reduced pressure, and to a 1,4-dioxane (20.0 mL) and water (20.0 mL) mixed solution of the residue, triethylamine (14.4 mL) and di-t-butyl dicarbonate (4.46 g) were added at 0°C. After stirred at 0°C for 30 minutes, aqueous sodium hydrogencarbonate solution was added to the reaction liquid, and extracted with ethyl acetate. The organic layer was washed with saturated saline, and dried with anhydrous sodium sulfate. After the organic layer was concentrated under reduced pressure, the residue was purified through silica gel column chromatography (hexane/ethyl acetate = 7/3) to obtain the entitled compound (2.53 g) as a white solid.
ESI-MS Found: m/z 369[M+H]⁺

### Reference Example 5 - Production of Ketone (II):

### Reference Example 5-1:

### Production of (3,4-difluorophenyl){4-[(6-fluoro-1H,1'H-spiro[furo[3,4-c]pyridine-3,4'-piperidin]-1'-yl)methyl]phenyl}methanone

At 0°C, methanesulfonyl chloride (780 µL) was added to an ethyl acetate solution (13.0 mL) of the compound (1.00 g) obtained in Reference Example 1-11 and triethylamine (1.98 mL), and stirred for 20 minutes. Aqueous sodium hydrogencarbonate solution was added to the reaction liquid, and extracted with ethyl acetate. The organic layer was washed with saturated saline, and dried with anhydrous sodium sulfate. The organic layer was concentrated under reduced pressure, and a crude product (1.33 g) of 4-(3,4-difluorobenzoyl)benzyl methanesulfonate was obtained as a white solid.
At 0°C, triethylamine (1.70 mL) was added to a chloroform solution (15.0 mL) of the compound (986 mg) obtained in Reference Example 4-8. To it was added a chloroform solution (5.00 mL) of the above-obtained mesylate (1.33 g), and stirred at room temperature for 3 hours. Aqueous sodium hydrogencarbonate solution was added to the reaction liquid, and extracted with chloroform. The organic layer was washed with saturated saline, and dried with anhydrous sodium sulfate. After the organic layer was concentrated under reduced pressure, the residue was purified through silica gel column chromatography (chloroform/methanol = 95/5) to obtain the entitled compound (1.16 g) as a colorless oil. ESI-MS Found: m/z 439[M+H]⁺

### Reference Example 5-1-1:

In the same manner as in Reference Example 5-1 but using various ketoalcohols 10 or bromides (IV) obtained in Reference Examples 1 and 2 and various amines (V) obtained in Reference Example 4, the following ketones (II) were obtained.

### [Table 5]

**Table 4-1 - Production of Ketones (II)**

| 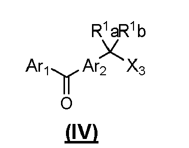 | 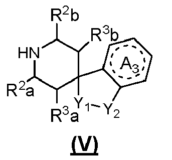 | | 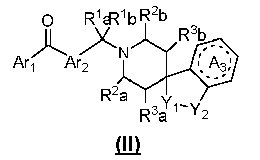 | ESI-MS |
|---|---|---|---|---|
| 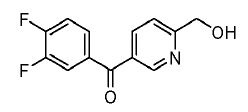 | 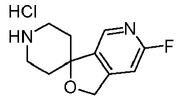 | a) | 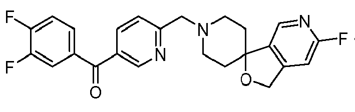 | 440 [M+H]⁺ |
| 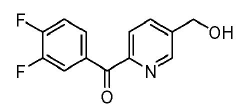 | | a) | 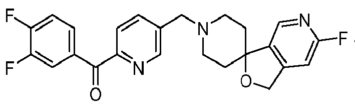 | 440 [M+H]⁺ |
| 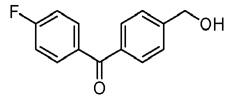 | | a) | 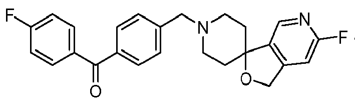 | 421 [M+H]⁺ |
| 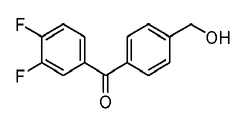 | 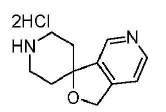 | a) | 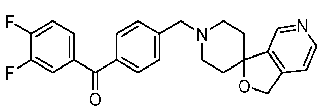 | 421 [M+H]⁺ |
| 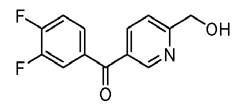 | | a) | 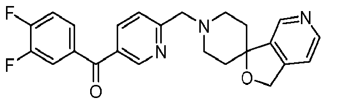 | 422 [M+H]⁺ |
| 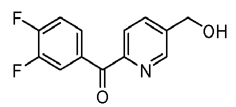 | | a) | 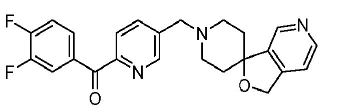 | 422 [M+H]⁺ |
| 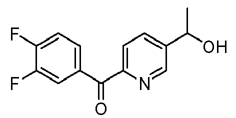 | | a) | 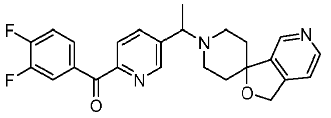 | 436 [M+H]⁺ |
| 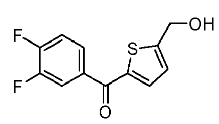 | | a) | 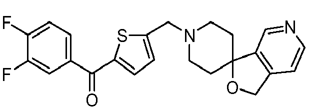 | 427 [M+H]⁺ |
| 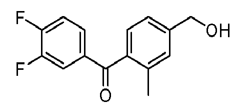 | | a) | 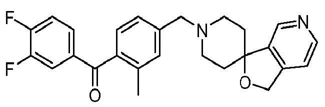 | 435 [M+H]⁺ |
| 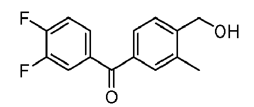 | | a) | 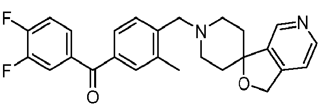 | 435 [M+H]⁺ |

| | | | | |
|---|---|---|---|---|
| conditions: a) Et₃N or *i*-Pr₂NEt, CHCl₃, 0 °C, 1 h, r.t., 1-5 d | | | | |

### [Table 6]

**Table 4-2 - Production of Ketones (ll)**

| 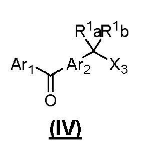 | | 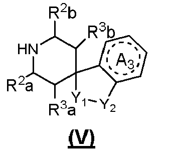 | | 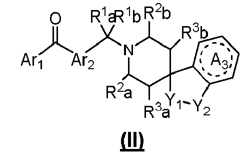 | ESI-MS |
|---|---|---|---|---|---|
| 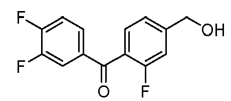 | a) | 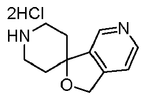 | b) | 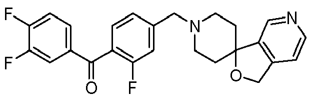 | 439 [M+H]⁺ |
| 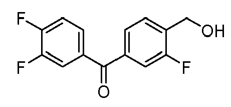 | a) | | b) | 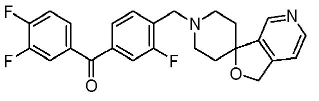 | 439 [M+H]⁺ |
| 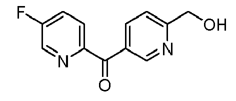 | a) | | b) | 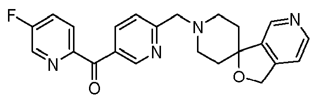 | 405 [M+H]⁺ |
| 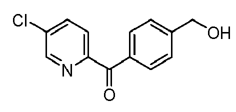 | a) | | b) | 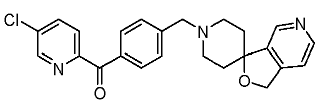 | 420 [M+H]⁺ |
| 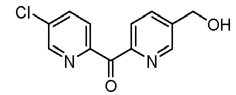 | a) | | b) | 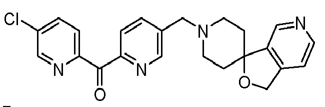 | 420 [M+H]⁺ |
| 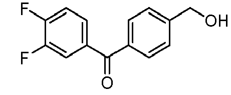 | a) | 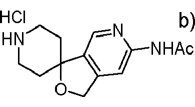 | b) | 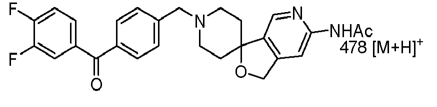 | 478 [M+H]⁺ |
| 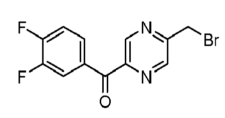 | | 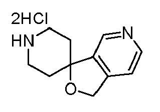 | b) | 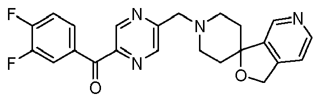 | 423 [M+H]⁺ |
| 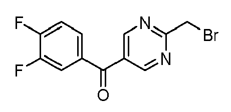 | | | b) | 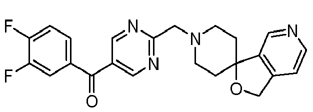 | 423 [M+H]⁺ |
| 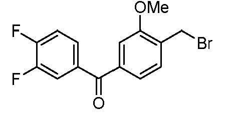 | | | b) | 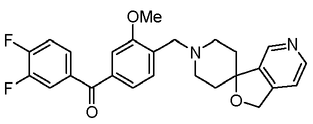 | 451 [M+H]⁺ |
| 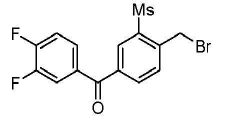 | | | b) | 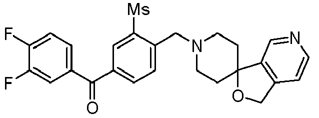 | 499 [M+H]⁺ |

| | | | | | |
|---|---|---|---|---|---|
| conditions: a) MsCl, Et₃N, EtOAc, 0 °C, 15 min b) Et₃N or *i*-Pr₂NEt, CHCl₃, 0 °C, 1-2 h, r.t., 1 d | | | | | |

### Reference Example 5-2:

### Production of 1'-({6-[(3,4-difluorophenyl)carbonyl]-3-pyridinyl}methyl)-5-methyl-3,5-dihydro-6H-spiro[furo[3,4-c]pyridine-1,4'-piperidine]-6-one

In the same manner as in Reference Example 5-1 but using (3,4-difluorophenyl)[5-(hydroxymethyl)-2-pyridinyl]methanone (39.8 mg) obtained in Reference Example 1-12, a crude product (52.4 mg) of the corresponding mesylate was obtained as a brown oil. At 0°C, 4 N hydrogen chloride-dioxane solution (5.00 mL) was added to the compound (51.3 mg) obtained in Reference Example 4-6, and stirred for 20 minutes, and then the reaction liquid was concentrated under reduced pressure. Chloroform (5.00 mL) was added to the residue, then at 0°C, a chloroform solution (3.00 mL) of triethylamine (107 µL) and the above mesylate (52. 4 mg) were dropwise added to it. This was stirred at 0°C for 1 hour, and then stirred overnight at room temperature. Aqueous sodium hydrogencarbonate solution was added to the reaction solution, and extracted with chloroform. The organic layer was washed with saturated saline, then dried with anhydrous sodium sulfate. After the organic layer was concentrated under reduced pressure, the residue was purified through silica gel column chromatography (chloroform/methanol = 30/1) to obtain the entitled compound (59.8 mg) as a white amorphous substance.
ESI-MS Found: m/z 452[M+H]⁺

### Reference Example 5-3:

In the same manner as in Reference Example 5-2 but using various ketoalcohols 10 obtained in Reference Example 1 and the amine precursor obtained in Reference Example 4-6, the following ketones (II) were obtained.

### [Table 7]

**Table 5 - Production of Ketones (II)**

| 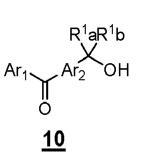 | | 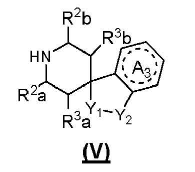 | | 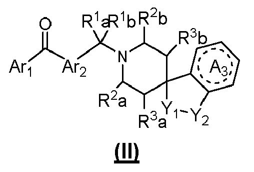 | ESI-MS |
|---|---|---|---|---|---|
| 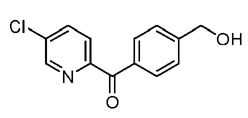 | a), b) | 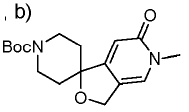 | c) | 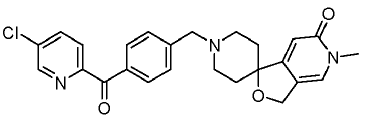 | 450 [M+H]⁺ |
| 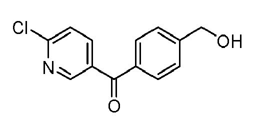 | a, b) | | c) | 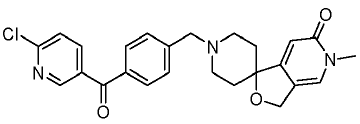 | 450 [M+H]⁺ |

| | | | | | |
|---|---|---|---|---|---|
| conditions: a) MsCl, Et₃N, EtOAc, 0 °C, 15 min b) 4 M HCI - EtOAc, 0 °C, 30 min c) Et₃N or *i*-Pr₂NEt, CHCl₃, 0 °C, 1-2 h, r.t., 1 d | | | | | |

### Reference Example 6 - Production of Ketones (II):

### Reference Example 6-1:

### Production of (3,4-difluorophenyl)[5-(1H,1'H-spiro[furo[3,4-c]pyridine-3,4'-piperidin]-1'-ylmethyl)pyrimidin-2-yl]methanone

At 0°C, triethylamine (8.55 mL) was added to a THF solution (100 mL) of the compound (4.00 g) obtained in Reference Example 4-32, and this was dropwise added to a THF solution (50.0 mL) of the compound (3.77 g) obtained in Reference Example 3-5 at 0°C. Methanol (50.0 mL) was added thereto, and stirred at room temperature for 1 hour. At room temperature, a methanol solution (100 mL) of 0.30 M zinc(II) chloride-sodium cyanotrihydroborate was added thereto, and stirred overnight. After cooled to 0°C, water was added to the reaction liquid, and concentrated under reduced pressure. Aqueous 2 N sodium hydroxide solution was added to the residue, and the organic layer was extracted with chloroform. The organic layer was dried with anhydrous sodium sulfate, and the organic layer was concentrated under reduced pressure. Chloroform (152 mL) was added to the obtained residue (6.93 g) of (3,4-difluorophenyl)[5-(1H,1H'-spiro[furo[3,4-c]pyridine-3,4'-piperidin]-1'-ylmethyl)pyrimidin-2-yl]methanol, and 85 % manganese dioxide (15.5 g) was added thereto at room temperature, and stirred overnight. After filtered through Celite, the residue was purified through silica gel column chromatography (chloroform/methanol= 98/2 to 95/5) to obtain the entitled compound (4.01 g) as a white amorphous substance.
ESI-MS Found: m/z 423[M+H]⁺

### Reference Example 6-2:

### Production of (3,4-difluorophenyl){5-[(6-fluoro-1H,1'H-spiro[furo[3,4-c]pyridine-3,4'-piperidin]-1'-yl)methyl]-2-pyrimidinyl}methanone

In the same manner as in Reference Example 6-1 but using the compound (66.0 mg) obtained in Reference Example 4-8 and the compound (67.0 mg) obtained in Reference Example 3-5, the entitled compound (34.8 mg) was obtained as a white amorphous substance.
ESI-MS Found: m/z 441 [M+H]⁺

### Reference Example 7 - Production of Oximes (IVc"):

### Reference Example 7-1:

### Production of (Z)-(3,4-difluorophenyl)[5-(hydroxymethyl)-2-pyridinyl]methanone O-(2-hydroxy-2-methylpropyl)oxime and (E)-(3,4-difluorophenyl)[5-(hydroxymethyl)-2-pyridinyl]methanone O-(2-hydroxy-2-methylpropyl)oxime

O-(2-hydroxy-2-methylpropyl)hydroxylamine hydrochloride (2.41 g) was added to a pyridine solution (25.0 mL) of (3,4-difluorophenyl)[5-(hydroxymethyl)-2-pyridinyl]methanone (1.36 g) obtained in Reference Example 1-12, and stirred overnight at room temperature. Pyridine was evaporated off under reduced pressure, aqueous sodium hydrogencarbonate solution was added thereto, and extracted with ethyl acetate. The organic layer was washed with saturated saline, and dried with anhydrous sodium sulfate. After the organic layer was concentrated under reduced pressure, the residue was purified through silica gel column chromatography (hexane/ethyl acetate = 100/0 to 0/100). This was subjected to separation of geometric isomers through high-performance liquid chromatography (YMC-CombiPrep™ pro C-18, H₂O (0.1% TFA)/CH₃CN (0.1% TFA)=90/10 to 50/50) to obtain the entitled compound (Z)-form (435 mg) as a white amorphous substance and the entitled compound (E)-form (1.17 g) as a pale yellow amorphous substance.

### Entitled Compound (Z)-form:

¹HNMR (400 MHz, CDCl₃, δ ppm): 1.21 (6H, s), 4.10 (2H, s), 4.83 (2H, s), 7.08-7.17 (2H, m), 7.31-7.37 (1H,m),7.50(1H,d,J=8.3Hz),7.88(1H,dd,J=8.3,2.2Hz),8.72(1H,d,J=1.5Hz).
ESI-MS Found: m/z 337[M+H]⁺

### Entitled Compound (E)-form:

¹HNMR (400 MHz, CDCl₃, δ ppm): 1.25 (6H, s), 4.15 (2H, s), 4.77 (2H, s), 7.15-7.33 (3H, m), 7.78-7.79 (2H, m), 8.56 (1H, s).
ESI-MS Found: m/z 337[M+H]⁺

### Reference Example 8 - Production of Oximes (IVc')

### Reference Example 8-1:

### Production of 4-[(acetyloxy)methyl]benzoic acid

Acetic anhydride (60.0 mL) and pyridine (2.00 mL) were added to a chloroform solution (300 mL) of 4-(hydroxymethyl)benzoic acid (18.3 g), and stirred at 80°C for 3 hours. The reaction liquid was concentrated under reduced pressure, and water (600 mL) was added to the residue and stirred at 90°C for 5 hours. The reaction liquid was cooled to 0°C, and the formed white solid was collected through filtration. The obtained solid was washed with water, and dissolved in ethyl acetate. The organic layer was washed with saturated saline, and dried with anhydrous sodium sulfate. The organic layer was concentrated under reduced pressure to obtain the entitled compound (21.0 g) as a white solid. ESI-MS Found: m/z 217[M+Na]⁺

### Reference Example 8-2:

### Production of 4-{[(2-fluoroethoxy)amino]carbonyl}benzyl acetate

At 0°C, O-(2-fluoroethyl)hydroxylamine hydrochloride (2.41 g), N-ethyldiisopropylamine (35.0 mL) and O-(7-azabenzotriazol-1-yl)-N,N,N',N'-tetramethyluronium hexafluorophosphate (16.7 g) were added to a DMF solution (160 mL) of the compound (7.77 g) obtained in Reference Example 8-1, and stirred overnight at room temperature. Water was added to the reaction liquid, and extracted with ethyl acetate. The organic layer was washed with saturated saline, and dried with anhydrous sodium sulfate. After the organic layer was concentrated under reduced pressure, the residue was purified through silica gel column chromatography (hexane/ethyl acetate = 50/50 to 0/100) to obtain the entitled compound (7.39 g) as a white solid.
ESI-MS Found: m/z 256[M+H]⁺

### Reference Example 8-3:

### Production of 4-{(Z)-bromo[(2-fluoroethoxy)imino]methyl}benzyl acetate

Carbon tetrabromide (14.9 g) and triphenyl phosphine (11.8 g) were added to an acetonitrile solution (150 mL) of the compound (8.41 g) obtained in Reference Example 8-2, and stirred at 80°C for 4 hours. The reaction liquid was cooled to room temperature, and the resulting white solid was filtered. After the filtrate was concentrated under reduced pressure, the residue was purified through silica gel column chromatography (hexane/ethyl acetate = 100/0 to 50/50) to obtain the entitled compound (8.76 g) as a yellow oil.
ESI-MS Found: m/z 318[M+H]⁺

### Reference Example 8-4:

### Production of 4-{(Z)-(3,4-difluorophenyl)[(2-fluoroethoxy)imino]methyl}benzyl acetate

3,4-Difluorophenylboronic acid (5.51 g), palladium acetate (653 mg), triphenyl phosphine (1.53 g) and aqueous 2 M sodium carbonate solution (29.0 mL) were added to a toluene solution (116 mL) of the compound (9.26 g) obtained in Reference Example 8-3, and stirred overnight at 80°C. Aqueous sodium hydrogencarbonate solution was added to the reaction liquid, and extracted with ethyl acetate. The organic layer was washed with saturated saline, and dried with anhydrous sodium sulfate. After the organic layer was concentrated under reduced pressure, the residue was purified through silica gel column chromatography (hexane/ethyl acetate = 100/0 to 0/100) to obtain the entitled compound (8.97 g) as a yellow oil.
ESI-MS Found: m/z 352[M+H]⁺

### Reference Example 8-5:

### Production of (Z)-(3,4-difluorophenyl)[4-(hydroxymethyl)phenyl]methanone O-(2-fluoroethyl)oxime

Potassium carbonate (6.91 g) was added to a methanol (200 mL)/water (50.0 mL) mixed solution of the compound (8.97 g) obtained in Reference Example 8-4, and stirred at room temperature for 1 hour. After methanol was evaporated off under reduced pressure, aqueous ammonium chloride solution was added thereto and extracted with ethyl acetate. The organic layer was washed with saturated saline, and dried with anhydrous sodium sulfate. After the organic layer was concentrated under reduced pressure, the residue was purified through silica gel column chromatography (chloroform/ethyl acetate = 100/0 to 70/30) to obtain the entitled compound (7.71 g) as a yellow oil.
ESI-MS Found: m/z 310[M+H]⁺

### Reference Example 8-6:

### Production of (Z)-[3,4-bis(methyloxy)phenyl][4-(hydroxymethyl)phenyl]methanone O-(2-fluoroethyl)oxime

In the same manner as in Reference Examples 8-4 and 8-5 but using the compound obtained in Reference Example 8-3 and 3,4-dimethoxyboronic acid, the entitled compound was obtained as a pale yellow oil.
ESI-MS Found: m/z 334[M+H]⁺

### Reference Example 8-7:

### Production of {5-[(Z)-bromo(ethoxyimino)methyl]pyridin-2-yl}methyl acetate

In the same manner as in Reference Examples 8-1 to 8-3 but using 6-(hydroxymethyl)pyridine-3-carboxylic acid, the entitled compound was obtained as a pale yellow white solid.
ESI-MS Found: m/z 301 [M+H]⁺

### Reference Example 8-8:

### Production of (E)-(3,4-difluorophenyl)[6-(hydroxymethyl)pyridin-3-yl]methanone O-ethyloxime

In the same manner as in Reference Examples 8-4 and 8-5 but using the compound obtained in Reference Example 8-7, the entitled compound was obtained as a colorless oil.
ESI-MS Found: m/z 293[M+H]⁺

### Reference Example 8-9:

### Production of {6-[(Z)-bromo(ethoxyimino)methyl]pyridin-3-yl}methyl acetate

In the same manner as in Reference Examples 8-1 to 8-3 but using 5-(hydroxymethyl)pyridine-2-carboxylic acid, the entitled compound was obtained as a yellow oil.
ESI-MS Found: m/z 301[M+H]⁺

### Reference Example 8-10:

### Production of (E)-(3,4-difluorophenyl)[5-(hydroxymethyl)pyridin-2-yl]methanone O-ethyloxime

In the same manner as in Reference Examples 8-4 and 8-5 but using the compound obtained in Reference Example 8-9, the entitled compound was obtained as a yellow oil.
ESI-MS Found: m/z 293[M+H]⁺

### Example 1-1:

### Production of (E)-(3,4-difluorophenyl){4-[(6-fluoro-1H,1'H-spiro[furo[3,4-c]pyridine-3,4'-piperidin]-1'-yl)methyl]phenyl}methanone oxime and (Z)-(3,4-difluorophenyl){4-[(6-fluoro-1H,1'H-spiro[furo[3,4-c]pyridine-3,4'-piperidin]-1'-yl)methyl]phenyl}methanone oxime

At 0°C, hydroxylamine hydrochloride (41.4 mg) was added to a pyridine solution (1.20 mL) of the compound (52.0 mg) obtained in Reference Example 5-1, and stirred overnight at room temperature. Aqueous sodium hydrogencarbonate solution was added to the reaction liquid, and extracted with diethyl ether. The organic layer was washed with water and saturated saline, and then dried with anhydrous sodium sulfate. After the organic layer was concentrated under reduced pressure, the residue was purified through preparative thin-layer silica gel chromatography (chloroform/methanol = 95/5) to obtain the entitled compound (E)-form (17.5 mg) and the entitled compound (Z)-form (27.4 mg) each as a white amorphous substance.

### Entitled Compound (E)-form:

¹HNMR (400 MHz, DMSO-d6, δ ppm): 1.67-1.71 (2H, m), 1.96-2.01 (2H, m), 2.32-2.38 (2H, m), 2.75-2.78 (2H, m), 3.57 (2H, s), 4.99 (2H, s), 7.09-7.11 (2H, m), 7.27 (2H, d, J=8.0 Hz), 7.37-7.46 (2H, m), 7.43 (2H, d, J=8.0 Hz), 8.19 (1H, s), 11.55 (1H, s).
ESI-MS Found: m/z 454[M+H]⁺

### Entitled Compound (Z)-form:

¹HNMR (400 MHz, DMSO-d6, δ ppm): 1.65-1.68 (2H, m), 1.90-1.98 (2H, m), 2.28-2.33 (2H, m), 2.70-2.72 (2H, m), 3.52 (2H, s), 4.97 (2H, s), 7.10-7.12 (2H, m), 7.32 (2H, d, J=8.8 Hz), 7.35 (2H, d, J=8.8 Hz), 7.40-7.45 (1H, m), 7.48-7.55 (1H, m), 8.18 (1H, s), 11.51 (1H, s).
ESI-MS Found: m/z 454[M+H]⁺

In the same manner as in Example 1-1 but using various ketones obtained in Reference Example 5, the compounds of Examples 1-1-1 to 1-1-5 were obtained.

### Example 1-1-1:

### (Z)-(3,4-difluorophenyl){6-[(6-fluoro-1H,1'H-spiro[furo[3,4-c]pyridine-3,4'-piperidin]-1'-yl)methyl]pyridin-3-yl}methanone oxime

¹HNMR (400 MHz, CDCl₃, δ ppm): 1.83-1.86 (2H, m), 2.10-2.18 (2H, m), 2.61-2.66 (2H, m), 2.96-2.99 (2H, m), 3.84 (2H, s), 5.06 (2H, s), 6.77-6.78 (1H, m), 7.09-7.18 (2H, m), 7.35-7.40 (1H, m), 7.59 (1H, d, J=8.0 Hz), 7.76 (1H, dd, J=8.0, 2.0 Hz), 7.95 (1H, s), 8.64 (1H, d, J=2.0 Hz). ESI-MS Found: m/z 455[M+H]⁺

### (E)-(3,4-difluorophenyl){6-[(6-fluoro-1H,1'H-spiro[furo[3,4-c]pyridine-3,4'-piperidin]-1'-yl)methyl]pyridin-3-yl}methanone oxime

¹HNMR (400 MHz, CDCl₃, δ ppm): 1.80-1.83 (2H, m), 2.05-2.10 (2H, m), 2.54-2.60 (2H, m), 2.88-2.91 (2H, m), 3.78 (2H, s), 5.04 (2H, s), 6.76-6.77 (1H, m), 7.13-7.17 (1H, m), 7.24-7.28 (1H, m), 7.30-7.37 (1H, m), 7.46 (1H, d, J=8.4 Hz), 7.71 (1H, dd, J=8.0, 2.0 Hz), 7.96 (1H, s), 8.73 (1H, d, J=2.0 Hz). ESI-MS Found: m/z 455[M+H]⁺

### Example 1-1-2:

### (E)-(3,4-difluorophenyl)[5-(1H,1'H-spiro[furo[3,4-c]pyridine-3,4'-piperidin]-1'-ylmethyl)-2-pyridinyllmethanone oxime

¹HNMR (400 MHz, CDCl₃, δ ppm): 1.80-1.83 (2H, m), 2.04-2.09 (2H, m), 2.47-2.53 (2H, m), 2.85-2.88 (2H, m), 3.61 (2H, s), 5.08 (2H, s), 7.18-7.28 (3H, m), 7.34-7.39 (1H, m), 7.84 (2H, s), 8.50-8.51 (3H, m), 11.50 (1H, brs).
ESI-MS Found: m/z 437[M+H]⁺

### Example 1-1-3:

### (Z)-(3,4-difluorophenyl)[3-(methylsulfonyl)-4-(1H,1'H-spiro[furo[3,4-c]pyridine-3,4'-piperidin]-1'-ylmethyl)phenyl]methanone oxime

¹HNMR (400 MHz, CDCl₃, δ ppm): 1.79-1.93 (4H, m), 2.56-2.62 (2H, m), 2.90-2.93 (2H, m), 3.46 (3H, s), 4.04 (2H, s), 5.06 (2H, s), 7.06-7.13 (2H, m), 7.20 (1H, d, J=5.1 Hz), 7.34 (1H, dd, J=11.1, 8.9 Hz), 7.57-7.62 (2H, m), 8.14 (1H, s), 8.38 (1H, s), 8.51 (1H, d, J=5.1 Hz), 9.39 (1H, s).
ESI-MS Found: m/z 514[M+H]⁺

### (E)-(3,4-difluorophenyl)[3-(methylsulfonyl)-4-(1H,1'H-spiro[furo[3,4-c]pyridine-3,4'-piperidin]-1'-ylmethyl)phenyl]methanone oxime

¹HNMR (400 MHz, CDCl₃, δ ppm): 1.78-1.93 (4H, m), 2.52-2.61 (2H, m), 2.83-2.89 (2H, m), 3.42 (3H, s), 4.01 (2H, s), 5.05 (2H, s), 7.09-7.12 (1H, m), 7.20-7.33 (3H, m), 7.49-7.58 (2H, m), 8.26 (1H, s), 8.43 (1H, s), 8.47 (1H, d, J=5.1 Hz), 10.35 (1H, s).
ESI-MS Found: m/z 514[M+H]⁺

### Example 1-1-4:

### 1'-({6-[(Z)-(3,4-difluorophenyl)(hydroxyimino)methyl]pyridin-3-yl}methyl)-5-methyl-3,5-dihydro-6H-spiro[furo[3,4-c]pyridine-1,4'-piperidin]-6-one

¹HNMR (400 MHz, CDCl₃, δ ppm): 1.79-2.05 (4H, m), 2.52-2.62 (2H, m), 2.85-2.95 (2H, m), 3.54 (3H, s), 3.75 (2H, s), 4.84 (2H, s), 6.38 (1H, s), 7.17-7.28 (4H, m), 7.30-7.39 (2H, m), 8.00 (1H, s), 8.64 (1H, s).
ESI-MS Found: m/z 467[M+H]⁺

### 1'-({6-[(E)-(3,4-difluorophenyl)(hydroxyimino)methyl]pyridin-3-yl}methyl)-5-methyl-3,5-dihydro-6H-spiro[furo[3,4-c]pyridine-1,4'-piperidin]-6-one

¹HNMR (400 MHz, CDCl₃, δ ppm): 1.76-1.79 (2H, m), 1.92-2.00 (2H, m), 2.46-2.53 (2H, m), 2.86-2.90 (2H, m), 3.55 (3H, s), 3.66 (2H, s), 4.83 (2H, s), 6.53 (1H, s), 7.19-7.26 (4H, m), 7.36 (1H, ddd, J=10.9, 7.8, 1.9 Hz), 7.80 (1H, d, J=7.8 Hz), 7.91 (1H, s), 8.50 (1H, d, J=1.9 Hz).
ESI-MS Found: m/z 467[M+H]⁺

### Example 1-1-5:

### N-[1'-({4-(Z)-(3,4-difluorophenyl)(hydroxyimino)methyl]phenyl}methyl)-1H-spiro[furo[3,4-c]pyridine-3,4'-piperidin]-6-yl]acetamide

¹HNMR (400 MHz, DMSO-d6, δ ppm): 1.77-1.81 (2H, m), 1.98-2.03 (2H, m), 2.05 (3H, s), 2.43-2.49 (2H, m), 2.82-2.85 (2H, m), 3.61 (2H, s), 5.04 (2H, s), 7.16-7.20 (1H, m), 7.21-7.28 (1H, m), 7.34-7.39 (1H, m), 7.36 (2H, d, J=8.0 Hz), 7.41 (2H, d, J=8.0 Hz), 8.02 (1H, s), 8.14 (1H, s), 8.85 (1H, s).
ESI-MS Found: m/z 493[M+H]⁺

### Example 1-2:

### Production of (E)-(3,4-difluorophenyl){4-[(6-fluoro-1H,1'H-spiro[furo[3,4-c]pyridine-3,4'-piperidin]-1'-yl)methyl]phenyl}methanone O-methyloxime and (Z)-(3,4-difluorophenyl){4-[(6-fluoro-1H,1'H-spiro[furo[3,4-c]pyridine-3,4'-piperidin]-1'-yl)methyl]phenyl}methanone O-methyloxime

At 0°C, O-methylhydroxylamine hydrochloride (46.2 mg) was added to a pyridine solution (1.00 mL) of the compound (48.4 mg) obtained in Reference Example 5-1, and stirred overnight at room temperature. Aqueous sodium hydrogencarbonate solution was added to the reaction solution, and extracted with diethyl ether. The organic layer was washed with water and saturated saline, and dried with anhydrous sodium sulfate. After the organic layer was concentrated under reduced pressure, the residue was purified through preparative thin-layer silica gel chromatography (chloroform/methanol = 95/5 × 2) to obtain the entitled compound (E)-form (14.8 mg) as a colorless oil and the entitled compound (Z)-form (20.5 mg) as a white amorphous substance.

### Entitled Compound (E)-form:

¹HNMR (400 MHz, CDCl₃, δ ppm): 1.82-1.85 (2H, m), 2.00-2.05 (2H, m), 2.44-2.50 (2H, m), 2.87-2.89 (2H, m), 3.62 (2H, s), 3.98 (3H, s), 5.05 (2H, s), 6.77-6.78 (1H, m), 7.07-7.13 (1H, m), 7.17-7.21 (1H, m), 7.30 (2H, d, J=8.0 Hz), 7.35-7.40 (1H, m), 7.45 (2H, d, J=8.0 Hz), 8.00 (1H, s).
ESI-MS Found: m/z 468[M+H]⁺

### Entitled Compound (Z)-form:

¹HNMR (400 MHz, CDCl₃, δ ppm): 1.79-1.82 (2H, m), 1.96-2.02 (2H, m), 2.40-2.46 (2H, m), 2.81-2.84 (2H, m), 3.59 (2H, s), 3.99 (3H, s), 5.03 (2H, s), 6.76-6.77 (1H, m), 7.08-7.11 (1H, m), 7.19-7.28 (2H, m), 7.34 (2H, d, J=8.0 Hz), 7.42 (2H, d, J=8.0 Hz), 7.99 (1H, s).
ESI-MS Found: m/z 468[M+H]⁺

In the same manner as in Example 1-2 but using various ketones obtained in Reference Example 5, the compounds of Examples 1-2-1 to 1-2-7 were obtained.

### Example 1-2-1:

### (Z)-(3,4-difluorophenyl){6-[(6-fluoro-1H,1'H-spiro[furo[3,4-c]pyridine-3,4'-piperidin]-1'-yl)methyl]pyridin-3-yl}methanone O-methyloxime

¹HNMR (400 MHz, CDCl₃, δ ppm): 1.83-1.86 (2H, m), 2.06-2.12 (2H, m), 2.55-2.61 (2H, m), 2.90-2.93 (2H, m), 3.79 (2H, brs), 3.99 (3H, s), 5.06 (2H, s), 6.77-6.78 (1H, m), 7.10-7.18 (2H, m), 7.38-7.43 (1H, m), 7.54-7.56 (1H, m), 7.66-7.68 (1H, m), 8.00 (1H, s), 8.55-8.56 (1H, m).
ESI-MS Found: m/z 469[M+H]⁺

### (E)-(3,4-difluorophenyl){6-[(6-fluoro-1H,1'H-spiro[furo[3,4-c]pyridine-3,4'-piperidin]-1'-yl)methyl]pyridin-3-yl}methanone O-methyloxime

¹HNMR (400 MHz, CDCl₃, δ ppm): 1.80-1.83 (2H, m), 2.02-2.08 (2H, m), 2.51-2.57 (2H, m), 2.84-2.88 (2H, m), 3.76 (2H, brs), 4.01 (3H, s), 5.04 (2H, s), 6.76-6.77 (1H, m), 7.07-7.11 (1H, m), 7.21-7.31 (2H, m), 7.43-7.46 (1H, m), 7.76-7.78 (1H, m), 7.98 (1H, s), 8.61-8.62 (1H, m).
ESI-MS Found: m/z 469[M+H]⁺

### Example 1-2-2:

### (Z)-(3,4-difluorophenyl){5-[(6-fluoro-1H,1'H-spiro[furo[3,4-c]pyridine-3,4'-piperidin]-1'-yl)methyl]-2-pyridinyl}methanone O-methyloxime

¹HNMR (400 MHz, CDCl₃, δ ppm): 1.82-1.85 (2H, m), 1.98-2.06 (2H, m), 2.48-2.54 (2H, m), 2.84-2.87 (2H, m), 3.64 (2H, s), 3.99 (3H, s), 5.05 (2H, s), 6.78-6.79 (1H, m), 7.07-7.14 (1H, m), 7.17-7.20 (1H, m), 7.35-7.41 (1H, m), 7.52 (1H, d, J=8.0 Hz), 7.85 (1H, d, J=8.0 Hz), 8.00 (1H, s), 8.675-8.679 (1H, m).
ESI-MS Found: m/z 469[M+H]⁺

### (E)-(3,4-difluorophenyl){5-[(6-fluoro-1H,1'H-spiro[furo[3,4-c]pyridine-3,4'-piperidin]-1'-yl)methyl]-2-pyridinyl}methanone O-methyloxime

¹HNMR (400 MHz, CDCl₃, δ ppm): 1.79-1.83 (2H, m), 1.94-2.02 (2H, m), 2.43-2.49 (2H, m), 2.79-2.82 (2H, m), 3.61 (2H, s), 4.04 (3H, s), 5.03 (2H, s), 6.76-6.77 (1H, m), 7.12-7.16 (1H, m), 7.19-7.25 (1H, m), 7.27-7.32 (1H, m), 7.74-7.79 (2H, m), 7.98 (1H, s), 8.53-8.54 (1H, m).
ESI-MS Found: m/z 469[M+H]⁺

### Example 1-2-3:

### (E)-(4-fluorophenyl){4-[(6-fluoro-1H,1'H-spiro[furo[3,4-c]pyridine-3,4'-piperidin]-1'-yl)methyl]phenyl}methanone O-methyloxime

¹HNMR (400 MHz, CDCl₃, δ ppm): 1.81-1.85 (2H, m), 1.98-2.06 (2H, m), 2.43-2.49 (2H, m), 2.87-2.90 (2H, m), 3.62 (2H, brs), 3.98 (3H, s), 5.05 (2H, s), 6.77-6.78 (1H, m), 6.99-7.04 (2H, m), 7.32 (2H, d, J=8.0 Hz), 7.43-7.49 (4H, m), 8.00 (1H, s).
ESI-MS Found: m/z 450[M+H]+

### (Z)-(4-fluorophenyl){4-[(6-fluoro-1H,1'H-spiro[furo[3,4-c]pyridine-3,4'-piperidin]-1'-yl)methyl]phenyl}methanone O-methyloxime

¹HNMR (400 MHz, CDCl₃, δ ppm): 1.79-1.82 (2H, m), 1.96-2.02 (2H, m), 2.38-2.46 (2H, m), 2.82-2.86 (2H, m), 3.59 (2H, brs), 3.98 (3H, s), 5.03 (2H, s), 6.76-6.77 (1H, m), 7.10-7.14 (2H, m), 7.32-7.38 (4H, m), 7.43 (2H, d, J=8.0 Hz), 7.99 (1H, s).
ESI-MS Found: m/z 450[M+H]⁺

### Example 1-2-4:

### (Z)-(3,4-difluorophenyl)[5-(1H,1'H-spiro[furo[3,4-c]pyridine-3,4'-piperidin]-1'-ylmethyl)-2-thienyl]methanone O-methyloxime

¹HNMR (400 MHz, CDCl₃, δ pom): 1.81-1.84 (2H, m), 2.04-2.11 (2H, m), 2.47-2.53 (2H, m), 2.91-2.94 (2H, m), 3.83 (2H, s), 4.15 (3H, s), 5.06 (2H, s), 6.91 (1H, d, J=4.0 Hz), 7.01 (1H, d, J=4.0 Hz), 7.18-7.25 (2H, m), 7.27-7.31 (1H, m), 7.35-7.40 (1H, m), 8.47 (1H, s), 8.52 (1H, d, J=4.8 Hz).
ESI-MS Found: m/z 456[M+H]⁺

### (E)-(3,4-difluorophenyl)[5-(1H,1'H-spiro[furo[3,4-c]pyridine-3,4'-piperidin]-1'-ylmethyl)-2-thienyl]methanone O-methyloxime

¹HNMR (400 MHz, CDCl₃, δ pom): 1.80-1.83 (2H, m), 2.02-2.10 (2H, m), 2.47-2.53 (2H, m), 2.91-2.94 (2H, m), 3.76 (2H, s), 3.94 (3H, s), 5.06 (2H, s), 6.66 (1H, d, J=3.6 Hz), 6.80 (1H, d, J=3.6 Hz), 7.13-7.16 (1H, m), 7.19 (1H, d, J=4.8 Hz), 7.20-7.29 (2H, m), 8.47 (1H, s), 8.52 (1H, d, J=4.8 Hz).
ESI-MS Found: m/z 456[M+H]⁺

### Example 1-2-5:

### (Z)-(3,4-difluorophenyl)[2-methyl-4-(1H,1'H-spiro[furo[3,4-c]pyridine-3,4'-piperidin]-1'-ylmethyl)phenyl]methanone O-methyloxime

¹HNMR (400 MHz, CDCl₃, δ ppm): 1.80-1.87 (2H, m), 2.03-2.16 (5H, m), 2.43-2.61 (2H, m), 2.86-3.00 (2H, m), 3.64 (2H, brs), 3.96 (3H, s), 5.07 (2H, s), 7.01-7.20 (5H, m), 7.26-7.41 (2H, m), 8.47 (1H, s), 8.52 (1H, d, J=4.9 Hz).
ESI-MS Found: m/z 464[M+H]⁺

### (E)-(3,4-difluorophenyl)[2-methyl-4-(1H,1'H-spiro[furo[3,4-c]pyridine-3,4'-piperidin]-1'-ylmethyl)phenyl]methanone O-methyloxime

¹HNMR (400 MHz, CDCl₃, δ ppm): 1.78-1.84 (2H, m), 1.96-2.20 (5H, m), 2.38-2.52 (2H, m), 2.82-2.93 (2H, m), 3.59 (2H, brs), 4.02 (3H, s), 5.06 (2H, s), 7.11-7.26 (6H, m), 7.46-7.55 (1H, m), 8.45-8.54 (2H, m).
ESI-MS Found: m/z 464 [M+H]⁺

### Example 1-2-6:

### (E)-(3,4-difluorophenyl)[3-methyl-4-(1H,1'H-spiro[furo[3,4-c]pyridine-3,4'-piperidin]-1'-ylmethyl)phenyl]methanoneO-methyloxime

¹HNMR (400 MHz, CDCl₃, δ ppm): 1.78-1.86 (2H, m), 1.97-2.09 (2H, m), 2.41 (3H, s), 2.46-2.55 (2H, m), 2.83-2.92 (2H, m), 3.57 (2H, s), 3.97 (3H, s), 5.08 (2H, s), 7.06-7.20 (5H, m), 7.35-7.44 (2H, m), 8.44-8.54 (2H, m).
ESI-MS Found: m/z 464 [M+H]⁺

### (Z)-(3,4-difluorophenyl)[3-methyl-4-(1H,1'H-spiro[furo[3,4-c]pyridine-3,4'-piperidin]-1'-ylmethyl)phenyl]methanone O-methyloxime

¹HNMR (400 MHz, CDCl₃, δ ppm): 1.75-1.82 (2H, m), 1.94-2.03 (2H, m), 2.39 (3H, s), 2.42-2.51 (2H, m), 2.78-2.86 (2H, m), 3.54 (2H, s), 3.98 (3H, s), 5.06 (2H, s), 7.06-7.10 (1H, m), 7.18-7.32 (6H, m), 8.43-8.53 (2H, m).
ESI-MS Found: m/z 464 [M+H]⁺

### Example 1-2-7:

### (E)-(5-fluoropyridin-2-yl)[6-(1H,1'H-spiro[furo[3,4-c]pyridine-3,4'-piperidin]-1'-ylmethyl)pyridin-3-yl]methanone O-methyloxime

¹HNMR (400 MHz, CDCl₃, δ ppm): 1.81-1.85 (2H, m), 2.09-2.13 (2H, m), 2.57-2.61 (2H, m), 2.90-2.94 (2H, m), 3.80 (2H, brs), 4.02 (3H, s), 5.08 (2H, s), 6.94-6.97 (1H, m), 7.19-7.20 (1H, m), 7.56-7.58 (1H, m), 7.69-7.71 (1H, m), 7.98-8.02 (1H, m), 8.24-8.25 (1H, m), 8.46-8.47 (1H, m), 8.52-8.54 (1H, m), 8.60-8.61 (1H, m).
ESI-MS Found: m/z 434[M+H]⁺

### (Z)-(5-fluoropyridin-2-yl)[6-(1H,1'H-spiro[furo[3,4-c]pyridine-3,4'-piperidin]-1'-ylmethyl)pyridin-3-yl]methanone O-methyloxime

¹HNMR (400 MHz, CDCl₃, δ ppm): 1.79-1.83 (2H, m), 2.08-2.12 (2H, m), 2.53-2.57 (2H, m), 2.85-2.87 (2H, m), 3.77 (2H, brs), 4.03 (3H, s), 5.06 (2H, s), 7.03-7.05 (1H, m), 7.18-7.20 (1H, m), 7.46-7.52 (1H, m), 7.77-7.79 (1H, m), 7.84-7.88 (1H, m), 8.26-8.27 (1H, m), 8.45-8.46 (1H, m), 8.51-8.52 (1H, m), 8.63-8.64 (1H, m).
ESI-MS Found: m/z 434[M+H]⁺

### Example 1-3:

### (E)-(3,4-difluorophenyl){4-[(6-fluoro-1H,1'H-spiro[furo[3,4-c]pyridine-3,4'-piperidin]-1' yl)methyl]phenyl}methanone O-ethyloxime and (Z)-(3,4-difluorophenyl){4-[6-fluoro-1H,1'H-spiro[furo[3,4-c]pyridine-3,4'-piperidin]-1'-yl)methyl]phenyl}methanone O-ethyloxime

The same process as in Example 1-2 was carried out, using the compound (48.4 mg) obtained in Reference Example 5-1 and O-ethylhydroxylamine hydrochloride (46.2 mg). This was subjected to separation of geometric isomers through CHIRALPAK AD-H (hexane/ethanol/diethylamine = 80/20/0.02) to obtain the entitled compound (E)-form (22.4 mg, faster) as a colorless oil and the entitled compound (Z)-form (29.5 mg, later) as a white amorphous substance.

### Entitled Compound (E)-form:

¹HNMR (400 MHz, CDCl₃, δ ppm): 1.31 (3H, t, J=6.8 Hz), 1.82-1.85 (2H, m), 1.99-2.07 (2H, m), 2.44-2.50 (2H, m), 2.87-2.90 (2H, m), 3.62 (2H, brs), 4.24 (2H, q, J=6.8 Hz), 5.05 (2H, s), 6.77-6.78 (1H, m), 7.06-7.13 (1H, m), 7.16-7.20 (1H, m), 7.32 (2H, d, J=8.0 Hz), 7.35-7.40 (1H, m), 7.44 (2H, d, J=8.0 Hz), 8.00 (1H, s).
ESI-MS Found: m/z 482[M+H]⁺

### Entitled Compound (Z)-form:

¹HNMR (400 MHz, CDCl₃, δ ppm): 1.31 (3H, t, J=6.8 Hz), 1.79-1.82 (2H, m), 1.96-2.03 (2H, m), 2.40-2.45 (2H, m), 2.81-2.84 (2H, m), 3.59 (2H, s), 4.25 (2H, q, J=6.8 Hz), 5.03 (2H, s), 6.76-6.77 (1H, m), 7.08-7.12 (1H, m), 7.18-7.24 (1H, m), 7.24-7.29 (1H, m), 7.34 (2H, d, J=8.0 Hz), 7.42 (2H, d, J=8.0 Hz), 7.99 (1H, s).
ESI-MS Found: m/z 482[M+H]⁺

In the same manner as in Example 1-3 but using various ketones obtained in Reference Example 5, the compounds of Examples 1-3-1 to 1-3-4 were obtained.

### Example 1-3-1:

### (E)-(5-chloropyridin-2-yl)[4-(1H,1'H-spiro[furo[3,4-dpyridine-3,4'-piperidin]-1' ylmethyl)phenyl]methanone O-ethyloxime

¹HNMR (400 MHz, CDCl₃, δ ppm): 1.34 (3H, t, J=7.1 Hz), 1.79-1.89 (2H, m), 2.03-2.06 (2H, m), 2.43-2.50 (2H, m), 2.87-2.92 (2H, m), 3.62 (2H, s), 4.30 (2H, q, J=7.0 Hz), 5.07 (2H, s), 7.18-7.19 (1H, m), 7.38-7.44 (4H, m), 7.66-7.75 (2H, m), 8.47-8.54 (3H, m).
ESI-MS Found: m/z 463[M+H]⁺

### (Z)-(5-chloropyridin-2-yl)[4-(1H,1'H-spiro[furo[3,4-c]pyridine-3,4'-piperidin]-1'-ylmethyl)phenyl]methanone O-ethyloxime

¹HNMR (400 MHz, CDC₃, δ ppm): 1.29 (3H, t, J=7.1 Hz), 1.76-1.80 (2H, m), 1.97-2.05 (2H, m), 2.39-2.45 (2H, m), 2.80-2.85 (2H, m), 3.58 (2H, s), 4.25 (2H, q, J=7.2 Hz), 5.05 (2H, s), 7.17-7.18 (1H, m), 7.32-7.35 (2H, m), 7.41-7.43 (2H, m), 7.53-7.55 (1H, m), 7.76-7.78 (1H, m), 8.45-8.51 (2H, m), 8.67-8.68 (1H, m).
ESI-MS Found: m/z 463[M+H]⁺

### Example 1-3-2:

### (Z)-(3,4-difluorophenyl)[5-(1H,1'H-spiro[furo[3,4-c]pyridine-3,4'-piperidin]-1'-ylmethyl)pyrimidin-2-yl]methanone O-ethyloxime

¹HNMR (400 MHz, CDCl₃, δ ppm): 1.27 (3H, t, J=6.8 Hz), 1.83-1.86 (2H, m), 2.05-2.10 (2H, m), 2.55-2.61 (2H, m), 2.86-2.89 (2H, m), 3.67 (2H, s), 4.23 (2H, q, J=6.8 Hz), 5.08 (2H, s), 7.07-7.16 (2H, m), 7.20 (1H, d, J=4.8 Hz), 7.38-7.43 (1H, m), 8.47 (1H, s), 8.53 (1H, d, J=4.8 Hz), 8.90 (2H, s).
ESI-MS Found: m/z 466[M+H]⁺

### (E)-(3,4-difluorophenyl)[5-(1H,1'H-spiro[furo[3,4-c]pyridine-3,4'-piperidin]-1'-ylmethyl)pyrimidin-2-yl]methanone O-ethyloxime

¹HNMR (400 MHz, CDCl₃, δ ppm): 1.34 (3H, t, J=6.8 Hz), 1.79-1.82 (2H, m), 1.97-2.05 (2H, m), 2.48-2.54 (2H, m), 2.79-2.82 (2H, m), 3.63 (2H, s), 4.41 (2H, q, J=6.8 Hz), 5.06 (2H, s), 7.15-7.26 (2H, m), 7.19 (1H, d, J=4.8 Hz), 7.30-7.35 (1H, m), 8.45 (1H, s), 8.52 (1H, d, J=4.8 Hz), 8.79 (2H, s).
ESI-MS Found: m/z 466[M+H]⁺

### Example 1-3-3:

### 1'-({6-[(Z)-(3,4-difluorophenyl)(ethoxyimino)methyl]pyridin-3-yl}methyl)-5-methyl-3,5-dihydro-6H-spiro[furo[3,4-c]pyridine-1,4'-piperidin]-6-one

¹HNMR (400 MHz, CDCl₃, δ ppm): 1.26 (3H, t, J=7.0 Hz), 1.74-1.77 (2H, m), 1.83-1.90 (2H, m), 2.40-2.46 (2H, m), 2.78-2.81 (2H, m), 3.50 (3H, s), 3.59 (2H, s), 4.21 (2H, q, J=7.0 Hz), 4.80 (2H, s), 6.33 (1H, s), 7.06 (1H, dd, J=8.1, 17.4 Hz), 7.12-7.15 (2H, m), 7.31-7.37 (1H, m), 7.54 (1H, d, J=7.8 Hz), 7.81 (1H, dd, J=1.7, 8.1 Hz), 8.60 (1H, s).
ESI-MS Found: m/z 495[M+H]⁺

### 1'-({6-[(E)-(3,4-difluorophenyl)(ethoxyimino)methyl]pyridin-3-yl}methyl)-5-methyl-3,5-dihydro-6H-spiro[furo[3,4-c]pyridine-1,4'-piperidin]-6-one

¹HNMR (400 MHz, CDCl₃, δ ppm): 1.29 (3H, t, J=7.1 Hz), 1.70-1.73 (2H, m), 1.80-1.85 (2H, m), 2.35-2.41 (2H, m), 2.72-2.76 (2H, m), 3.49 (3H, s), 3.55 (2H, s), 4.27 (2H, q, J=7.1 Hz), 4.78 (2H, s), 6.31 (1H, s), 7.10-7.20 (3H, m), 7.25-7.30 (1H, m), 7.71-7.77 (2H, m), 8.46 (1H, s).
ESI-MS Found: m/z 495[M+H]⁺

### Example 1-3-4:

### 1'-[(4-{(Z)-(6-chloro-3-pyridinyl)[(ethyloxy)imino]methyl}phenyl)methyl]-5-methyl-3,5-dihydro-6H-spiro[furo[3,4-c]pyridine-1,4'-piperidin]-6-one

¹HNMR (400 MHz, CDCl₃, δ ppm): 1.26 (3H, t, J=7.0 Hz), 1.70-1.87 (4H, m), 2.32-2.38 (2H, m), 2.73-2.78 (2H, m), 3.49 (3H, s), 3.54 (2H, s), 4.22 (2H, q, J=7.0 Hz), 4.78 (2H, s), 6.32 (1H, s), 7.13 (1H, s), 7.31 (2H, d, J=8.2 Hz), 7.36-7.39 (3H, m), 7.66 (1H, dd, J=8.2, 2.3 Hz), 8.38 (1H, d, J=2.3 Hz).
ESI-MS Found: m/z 494[M+H]⁺

### Example 1-4:

### 1'-({6-[(Z)-[(cyclopropyloxy)imino](3,4-difluorophenyl)methyl]-3-pyridinyl}methyl)-5-methyl-3,5-dihydro-6H-spiro[furo[3,4-c]pyridine-1,4'-piperidin]1-6-one and 1'-({6-[(E)-[(cyclopropyloxy)imino](3,4-difluorophenyl)methyl]-3-pyridinyl}methyl)-5-methyl-3,5-dihydro-6H-spiro[furo[3,4-c]pyridine-1,4'-piperidin]-6-one

In the same manner as in Example 1-2 but using the compound (50.0 mg) obtained in Reference Example 5-2 and (aminooxy)cyclopropane hydrochloride (48.2 mg), the entitled compound (Z)-form (14.7 mg) and the entitled compound (E)-form (24.5 mg) were obtained each as a white amorphous substance.

### Entitled Compound (Z)-form:

¹HNMR (400 MHz, CDCl₃, δ ppm): 0.61-0.66 (2H, m), 0.71-0.74 (2H, m), 1.73-1.77 (2H, m), 1.82-1.88 (2H, m), 2.37-2.45 (2H, m), 2.76-2.80 (2H, m), 3.50 (3H, s), 3.57 (2H, s), 4.06-4.11 (1H, m), 4.80 (2H, s), 6.32 (1H, s), 7.07 (1H, dd, J=8.7, 18.1 Hz), 7.13-7.16 (2H, m), 7.35 (1H, ddd, J=11.4, 7.8, 1.9 Hz), 7.45 (1H, d, J=7.8 Hz), 7.78 (1H, d, J=7.8 Hz), 8.58 (1H, s).
ESI-MS Found: m/z 507[M+H]⁺

### Entitled Compound (E)-form:

¹HNMR (400 MHz, CDCl₃, δ ppm): 0.64-0.71 (2H, m), 0.75-0.77 (2H, m), 1.69-1.86 (4H, m), 2.34-2.40 (2H, m), 2.70-2.75 (2H, m), 3.49 (3H, s), 3.54 (2H, s), 4.12-4.15 (1H, m), 4.78 (2H, s), 6.31 (1H, s), 7.06 (1H, s), 7.13-7.20 (3H, m), 7.73 (1H, d, J=8.0 Hz), 7.77 (1H, d, J=8.2 Hz), 8.47 (1H, s). ESI-MS Found: m/z 507[M+H]⁺

### Example 1-5:

### Production of N'-((3,4-difluorophenyl){4-[(6-fluoro-1H,1'H-spiro[furo[3,4-c]pyridine-3,4'-piperidin]-]1' yl)methyl]phenyl}methylene)acetohydrazide

Acetohydrazide (72.0 mg) and trifluoroacetic acid (5 drops) were added to a toluene solution (10.0 mL) of the compound (50.0 mg) obtained in Reference Example 5-1, and stirred with heating under reflux for 2 days. Aqueous saturated sodium hydrogencarbonate solution was added to the reaction liquid, and extracted with ethyl acetate. The organic layer was washed with saturated saline, and dried with anhydrous sodium sulfate. After the organic layer was concentrated under reduced pressure, the residue was purified through high-performance liquid chromatography (YMC-Pack™ pro C-18, H₂O (0.1% TFA)/CH₃CN (0.1% TFA)=90/10 0 to 50/50) to obtain the entitled compound (10.4 mg) as a colorless oil.
¹HNMR (400 MHz, CDCl₃, δ ppm):
Major Isomer: 1.78-1.90 (2H, m), 1.97-2.10 (2H, m), 2.43 (3H, s), 2.43-2.54 (2H, m), 2.78-2.94 (2H, m), 3.65 (2H, s), 5.06 (2H, s), 6.78 (1H, d, J=2.0 Hz), 7.07-7.15 (1H, m), 7.16-7.25 (2H, m), 7.35-7.50 (1H, m), 7.58 (2H, d, J=7.8 Hz), 8.02 (1H, s), 8.39 (1H, s).
Minor Isomer: 1.78-1.90 (2H, m), 1.97-2.10 (2H, m), 2.44 (3H, s), 2.43-2.54 (2H, m), 2.78-2.94 (2H, m), 3.61 (2H, s), 5.03 (2H, s), 6.75-6.78 (1H, m), 7.00-7.05 (1H, m), 7.07-7.25 (2H, m), 7.35-7.50 (3H, m), 7.99 (1H, s), 8.28 (1H, s).
ESI-MS Found: m/z 495[M+H]⁺

### Example 1-6:

### Production of N'-{(1E)-(3,4-difluorophenyl)[4-(1H,1'H-spiro[furo[3,4-c]pyridine-3,4'-piperidin]-1'-ylmethyl)phenyl]methylidene}-N-methylacetohydrazide and N'-{(1Z)-(3,4-difluorophenyl)[4-(1H,1'H-spiro[furo[3,4-c]pyridine-3,4'-piperidin]-1'-ylmethyl)phenyl]methylidene}-N-methylacetohydrazide

N-methylacetohydrazide (0.128 mL) and acetic acid (0.160 mL) were added to an ethanol solution (1.00 mL) of (3,4-difluorophenyl)[4-(1H,1'H-spiro[furo[3,4-c]pyridine-3,4'-piperidin]-1'-ylmethyl)phenyl]methanone (100 mg) obtained in Reference Example 5-1-1, and irradiated with microwaves (160°C 3 hours). Aqueous saturated sodium hydrogencarbonate solution was added to the reaction liquid, then extracted with ethyl acetate. The organic layer was washed with saturated saline, and dried with anhydrous sodium sulfate. The organic layer was concentrated under reduced pressure, and to a THF solution (1.00 mL) of the residue, added at 0°C were triethylamine (0.084 mL) and acetyl chloride (0.034 mL), and then stirred overnight at 0°C to room temperature. Aqueous saturated sodium hydrogencarbonate solution was added to the reaction liquid, and extracted with ethyl acetate. The organic layer was washed with saturated saline, and dried with anhydrous sodium sulfate. After the organic layer was concentrated under reduced pressure, the residue was purified through silica gel column chromatography (KP-NH, FLASH12+M, hexane/ethyl acetate = 1/1) to obtain a mixture of geometric isomers. This was further purified through high-performance chromatography (CHIRALPAK^{™} AD-H, hexane/ethanol/diethylamine = 70/30/0.03) to obtain the entitled compound (Z)-form (47.0 mg, faster) as a colorless oil and the entitled compound (E)-form (24.5 mg, later) as a colorless oil. Entitled Compound (E)-form:
¹HNMR (400 MHz, CDCl₃, δ ppm): 1.80-1.90 (2H, m), 2.00-2.15 (2H, m), 2.39 (3H, s), 2.45-2.55 (2H, m), 2.73 (3H, s), 2.78-2.85 (2H, m), 3.65 (2H, s), 5.08 (2H, s), 7.08-7.18 (1H, m), 7.18-7.28 (4H, m), 7.42-7.52 (3H, m), 8.47 (1H, s), 8.52 (1H, d, J=4.9 Hz).
ESI-MS Found: m/z 491[M+H]⁺

### Entitled Compound (Z)-form:

¹HNMR (400 MHz, CDCl₃, δ ppm): 1.76-1.85 (2H, m), 2.00-2.10 (2H, m), 2.38 (3H, s), 2.42-2.52 (2H, m), 2.79 (3H, s), 2.80-2.90 (2H, m), 3.63 (2H, s), 5.06 (2H, s), 7.04-7.10 (1H, m), 7.10-7.18 (1H, m), 7.20-7.34 (2H, m), 7.36-7.42 (2H, m), 7.46-7.52 (2H, m), 8.46 (1H, s), 8.51 (1H, d, J=4.9 Hz).
ESI-MS Found: m/z 491[M+H]⁺

### Example 1-7:

### Production of N-((3,4-difluorophenyl){4-[(6-fluoro-1H,1'H-spiro[furo[3,4-c]pyridine-3,4'-piperidin]-1'-yl)methyl]phenyl}methylene)methanesulfonamide

Titanium tetrachloride (30.0 mg) was added to a dichloroethane solution (10.0 mL) of the compound (135 mg) obtained in Reference Example 5-1, methanesulfonamide (29.0 mg) and triethylamine (0.086 mL), and in a nitrogen atmosphere, stirred with heating under reflux for 13 hours. At 0°C, aqueous saturated sodium hydrogencarbonate solution was added to it, and extracted with ethyl acetate. The organic layer was washed with saturated saline, and dried with anhydrous sodium sulfate. After the organic layer was concentrated under reduced pressure, the residue was purified through preparative thin-layer silica gel chromatography (hexane/ethyl acetate/ethanol = 5/4/1) to obtain the entitled compound (49.1 mg) as a colorless amorphous substance.
¹HNMR (400 MHz, CDCl₃, δ ppm): 1.78-1.87 (2H, m), 1.95-2.07 (2H, m), 2.40-2.53 (2H, m), 2.77-2.90 (2H, m), 3.23 (3H, s), 3.65 (2H, s), 5.04 (2H, s), 6.75-6.80 (1H, m), 7.23-7.52 (7H, m), 8.00 (1H, s).
ESI-MS Found: m/z 516[M+H]⁺

### Example 2-1:

### Production of (E)-(3,4-difluorophenyl)[5-(1H,1'H-spiro[furo[3,4-c]pyridine-3,4'-piperidin]-1'-ylmethyl)pyridin-2-yl]methanone O-(2-fluoroethyl)oxime

At 0°C, a DMF solution (1.50 mL) of potassium carbonate (38.2 mg) and 2-fluoroethyl 4-methylbenzenesulfonate (40.3 mg) was added to a DMF solution (500 µL) of the compound (E)-form (40.1 mg) obtained in Example 1-1-2, and stirred overnight at room temperature. Aqueous sodium hydrogencarbonate solution was added to the reaction liquid, and extracted with diethyl ether. The organic layer was washed with water and saturated saline, and dried with anhydrous sodium sulfate. After the organic layer was concentrated under reduced pressure, the residue was purified through preparative thin-layer silica gel chromatography (chloroform/methanol = 9/1) to obtain the entitled compound (35.2 mg) as a colorless oil.
¹HNMR (400 MHz, CDCl₃, δ pom): 1.79-1.82 (2H, m), 1.98-2.05 (2H, m), 2.45-2.51 (2H, m), 2.81-2.83 (2H, m), 3.62 (2H, s), 4.43-4.45 (1H, m), 4.50-4.52 (1H, m), 4.64-4.66 (1H, m), 4.76-4.78 (1H, m), 5.06 (2H, s), 7.16-7.26 (3H, m), 7.30-7.35 (1H, m), 7.76-7.80 (2H, m), 8.45 (1H, s), 8.51 (1H, d, J=4.8 Hz), 8.55 (1H, brs).
ESI-MS Found: m/z 483[M+H]⁺

In the same manner as in Examples 1-1 and 2-1 but using various ketones obtained in Reference Example 5, the compounds of Examples 2-1-1 to 2-1-13 were obtained.

### Example 2-1-1:

### (Z)-(3,4-difluorophenyl)[5-(1H,1'H-spiro[furo[3,4-c]pyridine-3,4'-piperidin]-1'-ylmethyl)pyridin-2-yl]methanone O-(2-fluoroethyl)oxime

¹HNMR (400 MHz, CDC1₃, δ ppm): 1.82-1.85 (2H, m), 2.03-2.08 (2H, m), 2.50-2.56 (2H, m), 2.86-2.89 (2H, m), 3.65 (2H, s), 4.38-4.40 (1H, m), 4.45-4.47 (1H, m), 4.62-4.64 (1H, m), 4.74-4.76 (1H, m), 5.08 (2H, s), 7.08-7.15 (1H, m), 7.17-7.20 (2H, m), 7.35-7.40 (1H, m), 7.62 (1H, d, J=8.0 Hz), 7.86-7.88 (1H, m), 8.47 (1H, s), 8.52 (1H, d, J=4.8 Hz), 8.67-8.68 (1H, m).
ESI-MS Found: m/z 483[M+H]⁺

### Example 2-1-2:

### (Z)-(3,4-difluorophenyl)[4-(1H,1'H-spiro[furo[3,4-c]pyridine-3,4'-piperidin]-1'-ylmethyl)phenyl]methanone O-(2-fluoroethyl)oxime

¹HNMR (400 MHz, CDCl₃, δ ppm): 1.76-1.84 (2H, m), 1.99-2.06 (2H, m), 2.40-2.50 (2H, m), 2.80-2.87 (2H, m), 3.60 (2H, s), 4.41 (2H, dt, J=28.5, 4.2 Hz), 4.69 (2H, dt, J=47.6, 4.2 Hz), 5.05 (2H, s), 7.10-7.16 (1H, m), 7.16-7.20 (1H, m), 7.21-7.30 (2H, m), 7.33-7.38 (2H, m), 7.40-7.44 (2H, m), 8.45 (1H, s), 8.51 (1H, d, J=4.9Hz).
ESI-MS Found: m/z 482[M+H]

### Example 2-1-3:

### (E)-(3,4-difluorophenyl){5-[1-(1H,1'H-spiro[furo[3,4-c]pyridine-3,4'-piperidin]-1'-yl)ethyl]pyridin-2-yl}methanone O-(2-fluoroethyl)oxime ((R) or (S), CHIRALPAK AD-H (hexane/isopropyl alcoyol/diethylamine = 50/50/0.05), faster)

¹HNMR (400 MHz, CDCl₃, δ ppm): 1.44 (3H, d, J=6.8 Hz), 1.73-1.85 (2H, m), 1.91-2.06 (2H, m), 2.39-2.48 (2H, m), 2.71-2.74 (1H, m), 2.94-2.97 (1H, m), 3.62 (1H, q, J=6.8 Hz), 4.43-4.45 (1H, m), 4.50-4.52 (1H, m), 4.64-4.66 (1H, m), 4.76-4.78 (1H, m), 5.03 (2H, s), 7.18 (1H, d, J=4.8 Hz), 7.19-7.26 (1H, m), 7.31-7.36 (2H, m), 7.77 (2H, s), 8.44 (1H, s), 8.51 (1H, d, J=4.8 Hz), 8.57 (1H, s).
ESI-MS Found: m/z 497[M+H]⁺

### (E)-(3,4-difluorophenyl){5-[1-(1H,1'H-spiro[furo[3,4-c]pyridine-3,4'-piperidin]-1'-yl)ethyl]pyridin-2-yl}methanone O-(2-fluoroethyl)oxime ((R) or (S), CHIRALPAK AD-H (hexane/isopropyl alcoyol/diethylamine = 50/50/0.05), slower)

¹HNMR (400 MHz, CDCl₃, δ ppm): 1.44 (3H, d, J=6.8 Hz), 1.73-1.85 (2H, m), 1.91-2.06 (2H, m), 2.39-2.48 (2H, m), 2.71-2.74 (1H, m), 2.94-2.97 (1H, m), 3.62 (1H, q, J=6.8 Hz), 4.43-4.45 (1H, m), 4.50-4.52 (1H, m), 4.64-4.66 (1H, m), 4.76-4.78 (1H, m), 5.03 (2H, s), 7.18 (1H, d, J=4.8 Hz), 7.19-7.26 (1H, m), 7.31-7.36 (2H, m), 7.77 (2H, s), 8.44 (1H, s), 8.51 (1H, d, J=4.8 Hz), 8.57 (1H, s).
ESI-MS Found: m/z 497[M+H]⁺

### Example 2-1-4:

### (Z)-(3,4-difluorophenyl)[2-fluoro-4-(1H,1'H-spiro[furo[3,4-c]pyridine-3,4'-piperidin]-1'-ylmethyl)phenyl]methanone O-(2-fluoroethyl)oxime

¹HNMR (400 MHz, CDCl₃, δ ppm): 1.80-1.87 (2H, m), 2.01-2.13 (2H, m), 2.45-2.56 (2H, m), 2.85-2.92 (2H, m), 3.63 (2H, s), 4.43 (2H, dt, J=27.8, 4.4 Hz), 4.67 (2H, dt, J=47.6, 4.4 Hz), 5.08 (2H, s), 7.08-7.25 (6H, m), 7.39-7.45 (1H, m), 8.47-8.54 (2H, m).
ESI-MS Found: m/z 500[M+H]⁺

### Example 2-1-5:

### (E)-(3,4-difluorophenyl)[2-fluoro-4-(1H,1'H-spiro[furo[3,4-c]pyridine-3,4'-piperidin]1-1'-ylmethyl)phenyl]methanone O-(2-fluoroethyl)oxime

¹HNMR (400 MHz, CDCl₃, δ ppm): 1.77-1.85 (2H, m), 1.98-2.10 (2H, m), 2.42-2.52 (2H, m), 2.79-2.88 (2H, m), 3.60 (2H, brs), 4.45 (2H, dt, J=28.8, 4.1 Hz), 4.71 (2H, dt, J=47.8, 4.1 Hz), 5.06 (2H, s), 7.11-7.25 (5H, m), 7.38-7.47 (2H, m), 8.45-8.54 (2H, m).
ESI-MS Found: m/z 500[M+H]⁺

### Example 2-1-6:

### (E)-(3,4-difluorophenyl)[3-fluoro-4-(1H,1'H-spiro[furo[3,4-c]pyridine-3,4'-piperidin]-1'-ylmethyl)phenyl]methanone O-(2-fluoroethyl)oxime

¹HNMR (400 MHz, CDCl₃, δ ppm): 1.80-1.87 (2H, m), 2.01-2.12 (2H, m), 2.51-2.61 (2H, m), 2.88-2.96 (2H, m), 3.70 (2H, s), 4.42 (2H, dt, J=28.5, 4.3 Hz), 4.69 (2H, dt, J=47.3, 4.3 Hz), 5.07 (2H, s), 7.08-7.21 (5H, m), 7.35-7.41 (1H, m), 7.50-7.57 (1H, m), 8.46 (1H, s), 8.51 (1H, d, J=4.9 Hz).
ESI-MS Found: m/z 500[M+H]⁺

### Example 2-1-7:

### (Z)-(3,4-difluorophenyl)[3-fluoro-4-(1H,1'H-spiro[furo[3,4-c]pyridine-3,4'-piperidin]-1'-ylmethyl)phenyl]methanone O-(2-fluoroethyl)oxime

¹HNMR (400 MHz, CDCl₃, δ ppm): 1.77-1.84 (2H, m), 1.98-2.08 (2H, m), 2.46-2.57 (2H, m), 2.82-2.90 (2H, m), 3.68 (2H, s), 4.42 (2H, dt, J=28.3, 4.1 Hz), 4.69 (2H, dt, J=47.3, 4.1 Hz), 5.05 (2H, s), 7.08-7.30 (6H, m), 7.37-7.44 (1H, m), 8.44 (1H, s), 8.51 (1H, d, J=3.9 Hz).
ESI-MS Found: m/z 500[M+H]⁺

### Example 2-1-8:

### (Z)-(3,4-difluorophenyl)[5-(1H,1'H-spiro[furo[3,4-c]pyridine-3,4'-piperidin]-1'-ylmethyl)pyrazin-2-yl]methanone O-(2-fluoroethyl)oxime

¹HNMR (400 MHz, CDCl₃, δ ppm): 1.83-1.86 (2H, m), 2.08-2.16 (2H, m), 2.61-2.67 (2H, m), 2.92-2.95 (2H, m), 3.85 (2H, s), 4.42-4.44 (1H, m), 4.49-4.51 (1H, m), 4.62-4.64 (1H, m), 4.74-4.76 (1H, m), 5.08 (2H, s), 7.11-7.21 (3H, m), 7.38-7.43 (1H, m), 8.46 (1H, s), 8.53 (1H, d, J=5.2 Hz), 8.82 (1H, d, J=1.6 Hz), 8.93 (1H, d, J=1.6 Hz).
ESI-MS Found: m/z 484[M+H]⁺

### Example 2-1-9:

### (E)-(3,4-difluorophenyl)[5-(1H,1'H-spiro[furo[3,4-c]pyridine-3,4'-piperidin]-1'-ylmethyl)pyrazin-2-yl]methanone O-(2-fluoroethyl)oxime

¹HNMR (400 MHz, CDCl₃, δ ppm): 1.80-1.83 (2H, m), 2.04-2.12 (2H, m), 2.56-2.62 (2H, m), 2.86-2.89 (2H, m), 3.82 (2H, s), 4.47-4.49 (1H, m), 4.54-4.56 (1H, m), 4.65-4.67 (1H, m), 4.77-4.79 (1H, m), 5.06 (2H, s), 7.17-7.28 (3H, m), 7.31-7.36 (1H, m), 8.44 (1H, s), 8.52 (1H, d, J=4.8 Hz), 8.63-8.64 (1H, m), 9.065-9.067 (1H, m).
ESI-MS Found: m/z 484[M+H]⁺

### Example 2-1-10:

### (Z)-(3,4-difluorophenyl)[2-(1H,1'H-spiro[furo[3,4-c]pyridine-3,4'-piperidin]-1'-ylmethyl)pyrimidin-5-yl]methanone O-(2-fluoroethyl)oxime

¹HNMR (400 MHz, CDCl₃, δ ppm): 1.83-1.86 (2H, m), 2.19-2.25 (2H, m), 2.63-2.69 (2H, m), 3.03-3.06 (2H, m), 3.98 (2H, s), 4.41-4.43 (1H, m), 4.48-4.50 (1H, m), 4.62-4.64 (1H, m), 4.74-4.76 (1H, m), 5.08 (2H, s), 7.11-7.21 (3H, m), 7.41-7.46 (1H, m), 8.46 (1H, s), 8.51 (1H, d, J=5.2 Hz), 8.80 (2H, s).
ESI-MS Found: m/z 484[M+H]⁺

### Example 2-1-11:

### (E)-(3,4-difluorophenyl)[2-(1H,1'H-spiro[furo[3,4-c]pyridine-3,4'-piperidin]-1'-ylmethyl)pyrimidin-5-yl]methanone O-(2-fluoroethyl)oxime

¹HNMR (400 MHz, CDCl₃, δ ppm): 1.81-1.84 (2H, m), 2.16-2.21 (2H, m), 2.59-2.65 (2H, m), 2.96-2.99 (2H, m), 3.95 (2H, s), 4.43-4.45 (1H, m), 4.50-4.52 (1H, m), 4.63-4.65 (1H, m), 4.75-4.77 (1H, m), 5.07 (2H, s), 7.11-7.16 (1H, m), 7.18-7.19 (1H, m), 7.24-7.31 (1H, m), 7.31-7.37 (1H, m), 8.45 (1H, s), 8.51 (1H, d, J=4.8 Hz), 8.80 (2H, s).
ESI-MS Found: m/z 484[M+H]⁺

### Example 2-1-12:

### N-[1'-(4-{(E)-(3,4-difluorophenyl)[(2-fluoroethoxy)imino]methyl}benzyl)-1H-spiro[furo[3,4-c]pyridine-3,4'-piperidin]-6-yl]acetamide

¹HNMR (400 MHz, DMSO-d6, δ ppm): 1.80-1.83 (2H, m), 1.99-2.04 (2H, m), 2.21 (3H, s), 2.46-2.51 (2H, m), 2.87-2.90 (2H, m), 3.63 (2H, s), 4.36-4.38 (1H, m), 4.43-4.45 (1H, m), 4.62-4.64 (1H, m), 4.74-4.76 (1H, m), 5.05 (2H, s), 7.07-7.14 (1H, m), 7.16-7.20 (1H, m), 7.33 (2H, d, J=8.0 Hz), 7.35-7.40 (1H, m), 7.45 (2H, d, J=8.0 Hz), 8.06 (1H, s), 8.09 (1H, s), 8.21 (1H, s).
ESI-MS Found: m/z 539[M+H]⁺

### Example 2-1-13:

### N-[1'-(4-{(Z)-(3,4-difluorophenyl)[(2-fluoroethoxy)imino]methyl}benzyl)-1H-spiro[furo[3,4-c]pyridine-3,4'-piperidin]-6-yl]acetamide

¹HNMR (400 MHz, DMSO-d6, δ ppm): 1.77-1.80 (2H, m), 1.95-2.01 (2H, m), 2.21 (3H, s), 2.41-2.47 (2H, m), 2.81-2.84 (2H, m), 3.60 (2H, s), 4.37-4.39 (1H, m), 4.44-4.46 (1H, m), 4.62-4.64 (1H, m), 4.74-4.76 (1H, m), 5.03 (2H, s), 7.11-7.15 (1H, m), 7.19-7.30 (2H, m), 7.35 (2H, d, J=8.0 Hz), 7.41 (2H, d, J=8.0 Hz), 8.04 (1H, s), 8.09 (1H, s), 8.24 (1H, s).
ESI-MS Found: m/z 539[M+H]⁺

### Example 2-2:

### Production of (E)-(3,4-difluorophenyl)[6-(1H,1'H-spiro[furo[3,4-c]pyridine-3,4'-piperidin]-1'-ylmethyl)pyridin-3-yl]methanone O-(fluoromethyl)oxime and (E)-(3,4-difluorophenyl)[6-(1H,1'H-spiro[furo[3,4-c]pyridine-3,4'-piperidin]-1'-ylmethyl)pyridin-3-yl]methanone O-(4-methylbenzenesulfonyl)oxime

At 0°C, a DMF solution (1.00 mL) of potassium carbonate (32.0 mg) and fluoromethyl 4-methylbenzenesulfonate (14.3 mg) was added to a DMF solution (500 µL) of (E)-(3,4-difluorophenyl)[6-(1H,1'H-spiro[furo[3,4-c]pyridine-3,4'-piperidin]-1'-ylmethyl)-3-pyridinyl]methanone oxime (20.1 mg) which was obtained according to the same manner as in Example 1-1 but using the compound obtained in Reference Example 5-1-1, and stirred for 1 hour. Aqueous sodium hydrogencarbonate solution was added to the reaction liquid, and extracted with diethyl ether. The organic layer was washed with water and saturated saline, and dried with anhydrous sodium sulfate. After the organic layer was concentrated under reduced pressure, the residue was purified through preparative thin-layer silica gel chromatography (chloroform/methanol = 95/5) to obtain the entitled compound, fluoromethyl-form (5.4 mg) as a colorless oil, and the entitled compound, tosyl-form (7.5 mg) as a red oil.

### Fluoromethyl form:

¹HNMR (400 MHz, CDCl₃, δ ppm): 1.80-1.83 (2H, m), 2.05-2.12 (2H, m), 2.53-2.59 (2H, m), 2.85-2.88 (2H, m), 3.78 (2H, s), 5.06 (2H, s), 5.70 (1H, s), 5.84 (1H, s), 7.11-7.15 (1H, m), 7.18-7.20 (1H, m), 7.24-7.32 (1H, m), 7.45 (1H, s), 7.48-7.51 (1H, m), 7.82-7.84 (1H, m), 8.45 (1H, s), 8.51 (1H, d, J=4.8 Hz), 8.635-8.640 (1H, m).
ESI-MS Found: m/z 469[M+H]⁺

### Tosyl form:

¹HNMR (400 MHz, CDCl₃, δ ppm): 1.80-1.83 (2H, m), 2.04-2.14 (2H, m), 2.48 (3H, s), 2.52-2.64 (2H, m), 2.82-2.90 (2H, m), 3.79 (2H, brs), 5.06 (2H, s), 7.09-7.13 (1H, m), 7.16-7.20 (2H, m), 7.27-7.33 (1H, m), 7.39 (2H, d, J=8.0 Hz), 7.48-7.52 (1H, m), 7.63-7.66 (1H, m), 7.91 (2H, d, J=8.0 Hz), 8.45 (1H, s), 8.52 (1H, d, J=4.8 Hz), 8.55-8.56 (1H, m).
ESI-MS Found: m/z 591[M+H]⁺

### Example 2-3:

In the same manner as in Example 2-1 but using the oxime obtained in Example 1-1 and 2-bromoethyl methyl ether, the following compound was obtained.

### (Z)-(3,4-difluorophenyl){4-[(6-fluoro-1H,1'H-spiro[furo[3,4-c]pyridine-3,4'-piperidin]-1'-yl)methyl]phenyl}methanone O-(2-methoxethyl)oxime

¹HNMR (400 MHz, CDCl₃, δ ppm): 1.75-1.85 (2H, m), 1.93-2.04 (2H, m), 2.38-2.48 (2H, m), 2.78-2.88 (2H, m), 3.38 (3H, s), 3.59 (2H, s), 3.66-3.72 (2H, m), 4.35 (2H, t, J=4.4 Hz), 5.03 (2H, s), 6.75-6.78 (1H, m), 7.08-7.14 (1H, m), 7.20 (1H, dt, J=7.6, 9.0 Hz), 7.26-7.36 (3H, m), 7.38-7.44 (2H, m), 7.98 (1H, s).
ESI-MS Found: m/z 512[M+H]⁺

### Example 2-4:

The oxime obtained in Example 1-1 and (2-bromoethoxy)-t-butyldimethylsilane were processed in the same manner as in Example 2-1, and then reacted with tetrabutylammonium fluoride in THF to obtain the following compound.

### (Z)-(3,4-difluorophenyl){4-[(6-fluoro-1H,1'H-spiro[furo[3,4-c]pyridine-3,4'-piperidin]-1'-yl)methyl]phenyl}methanone O-(2-hydroxyethyl)oxime

¹HNMR (400 MHz, CDCl₃, δ ppm): 1.76-1.86 (2H, m), 1.94-2.06 (2H, m), 2.38-2.50 (2H, m), 2.78-2.87 (2H, m), 3.60 (2H, s), 3.92-3.94 (2H, m), 4.29-4.32 (2H, m), 5.03 (2H, s), 6.74-6.80 (1H, s), 7.09-7.14 (1H, s), 7.20-7.30 (2H, m), 7.32-7.42 (4H, m), 7.98 (1H, s).
ESI-MS Found: m/z 498[M+H]⁺

### Example 2-5:

In the same manner as in Example 2-1 but using the oxime obtained in Example 1-1, and dimethylaminoethyl chloride hydrochloride and cesium carbonate, the compounds of Examples 2-5-1 and 2-5-2 were obtained.

### Example 2-5-1:

### (E)-(3,4-difluorophenyl){4-[(6-fluoro-1H,1'H-spiro[furo[3,4-c]pyridine-3,4'-piperidin]-1'-yl)methyl]phenyl}methanone O-[2-(dimethylamino)ethyl]oxime

¹HNMR (400 MHz, CDCl₃, δ ppm): 1.80-1.88 (2H, m), 1.96-2.07 (2H, m), 2.25 (6H, s), 2.42-2.52 (2H, m), 2.66 (2H, t, J=6.1 Hz), 2.83-2.90 (2H, m), 3.62 (2H, s), 4.30 (2H, t, J=6.1 Hz), 5.05 (2H, s), 6.75-6.79 (1H, m), 7.06-7.13 (1H, m), 7.14-7.20 (1H, m), 7.28-7.34 (2H, m), 7.38 (1H, ddd, J=2.0, 7.6, 11.2 Hz), 7.40-7.44 (2H, m), 8.00 (1H, s).
ESI-MS Found: m/z 525[M+H]⁺

### Example 2-5-2:

### (Z)-(3,4-difluorophenyl){4-[(6-fluoro-1H,1'H-spiro[furo[3,4-c]pyridine-3,4'-piperidin]-1'-yl)methyl]phenyl}methanone O-[2-(dimethylamino)ethyl]oxime

¹HNMR (400 MHz, CDCl₃, δ ppm): 1.76-1.84 (2H, m), 1.94-2.04 (2H, m), 2.27 (6H, s), 2.38-2.48 (2H, m), 2.66 (2H, t, J=6.0 Hz), 2.78-2.86 (2H, m), 3.59 (2H, s), 4.31 (2H, t, J=6.0 Hz), 5.03 (2H, s), 6.74-6.78 (1H, m), 7.06-7.12 (1H, m), 7.20 (1H, dt, J=7.6, 9.0 Hz), 7.26-7.36 (3H, m), 7.38-7.44 (2H, m), 7.98 (1H, s).
ESI-MS Found: m/z 525[M+H]⁺

### Example 2-6:

In the same manner as in Example 2-1 but using the oxime obtained in Example 1-1, and 2-chloro-N,N-dimethylacetamide, the following compound was obtained.

### 2-{[((1Z)-(3,4-difluorophenyl){4-[(6-fluoro-1H,1'H-spiro[furo[3,4-c]pyridine-3,4'-piperidin]-1'-yl)methyl]phenyl}methylene)amino]oxy}-N,N-dimethylacetamide

¹HNMR (400 MHz, CDCl₃, δ ppm): 1.75-1.85 (2H, m), 1.92-2.04 (2H, m), 2.36-2.47 (2H, m), 2.77-2.84 (2H, m), 2.95-2.98 (6H, m), 3.58 (2H, s), 4.85 (2H, s), 5.03 (2H, s), 6.74-6.78 (1H, m), 7.22 (1H, td, J=8.0, 10.0 Hz), 7.28-7.36 (3H, m), 7.38-7.42 (2H, m), 7.49 (1H, ddd, J=11.2, 8.0, 2.0 Hz), 7.98 (1H, s).
ESI-MS Found: m/z 539[M+H]⁺

### Example 2-7:

In the same manner as in Example 2-1 but using the oxime obtained in Example 1-1, and methyl bromoacetate, the following compound was obtained.

### Methyl{[((1Z)-(3,4-difluorophenyl){4-[(6-fluoro-1H,1'H-spiro[furo[3,4-c]pyridine-3,4'-piperidin]-1'-yl)methyl]phenyl}methylene)amino]oxy}acetate

¹HNMR (400 MHz, CDCl₃, δ ppm): 1.76-1.84 (2H, m), 1.94-2.04 (2H, m), 2.36-2.48 (2H, m), 2.78-2.85 (2H, m), 3.59 (2H, s), 3.78 (3H, s), 4.74 (2H, s), 5.03 (2H, s), 6.74-6.79 (1H, m), 7.20-7.25 (2H, m), 7.33-7.36 (2H, m), 7.38-7.45 (3H, m), 7.98 (1H, s).
ESI-MS Found: m/z 526[M+H]⁺

### Example 2-8:

### Production of 2- {[((1Z)-(3,4-difluorophenyl){4-[(6-fluoro-1H,1'H-spiro[furo[3,4-c]pyridine-3,4'-piperidin]-1'-yl)methyl]phenyl}methylene)amino]oxy}-N-methylacetamide

A THF solution (210 µL) of 2 M methylamine was added to a methanol solution (2.00 mL) of the compound (22.0 mg) obtained in Example 2-7, and stirred overnight at room temperature. After the reaction liquid was concentrated, the residue was purified through preparative thin-layer silica gel chromatography (chloroform/methanol = 19/1) to obtain the entitled compound (17.0 mg) as a colorless oil. ¹HNMR (400 MHz, CDCl₃, δ ppm): 1.70-1.90 (2H, m), 1.90-2.10 (2H, m), 2.40-2.50 (2H, m), 2.75-2.85 (2H, m), 2.86 (3H, d, J=4.0 Hz), 3.60 (2H, s), 4.66 (2H, s), 5.03 (2H, s), 5.90-6.05 (1H, m), 6.75-6.80 (1H, m), 7.10-7.14 (1H, m), 7.20-7.32 (2H, m), 7.34-7.38 (2H, m), 7.40-7.44 (2H, m), 7.98 (1H, s). mass spectrometry (ESI): 525.2 (M+H).
ESI-MS Found: m/z 525[M+H]⁺

### Example 2-9:

In the same manner as in Example 2-1 but using the oxime obtained in Example 1-1-4 and dichloromethane, the following compound was obtained.

### 1'-({6-[(E)-[(2-chloroethoxy)imino](3,4-difluorophenyl)methyl]pyridin-3-yl}methyl)-5-methyl-3,5-dihydro-6H-spiro[furo[3,4-c]pyridine-1,4'-piperidin]-6-one

¹HNMR (400 MHz, CDCl₃, δ ppm): 1.70-1.85 (4H, m), 2.35-2.41 (2H, m), 2.71-2.75 (2H, m), 3.49 (3H, s), 3.55 (2H, s), 3.77 (2H, t, J=5.8 Hz), 4.42 (2H, t, J=5.8 Hz), 4.78 (2H, s), 6.31 (1H, s), 7.13-7.21 (3H, m), 7.28-7.33 (1H, m), 7.73 (2H, brs), 8.49 (1H, s).
ESI-MS Found: m/z 529[M+H]⁺

### Example 2-10:

In the same manner as in Example 2-1 but using various oximes which were obtained in the same manner as in Example 1-1 but using the compound obtained in Reference Example 5-1-1, and bromoacetonitrile, the compounds of Examples 2-10-1 to 2-10-5 were obtained.

### Example 2-10-1:

### {[((1Z)-(3,4-difluorophenyl){5-[(5-methyl-6-oxo-5,6-dihydro-1'H,3H-spiro[furo[3,4-c]pyridine-1,4'-piperidin]-1'-yl)methyl]pyridin-2-yl}methylene)amino]oxy}acetonitrile

¹HNMR (400 MHz, CDCl₃, δ ppm): 1.74-1.92 (4H, m), 2.42-2.48 (2H, m), 2.78-2.81 (2H, m), 3.51 (3H, s), 3.61 (2H, s), 4.77 (2H, s), 4.80 (2H, s), 6.33 (1H, s), 7.07-7.21 (3H, m), 7.36 (1H, ddd, J=11.3, 7.8, 2.1 Hz), 7.46 (1H, d, J=7.8 Hz), 7.85 (1H, dd, J=7.9, 1.7 Hz), 8.64 (1H, d, J=1.7 Hz).
ESI-MS Found: m/z 506[M+H]⁺

### Example 2-10-2:

### {[((1E)-(3,4-difluorophenyl){5-[(5-methyl-6-oxo-5,6-dihydro-1'H,3H-spiro[furo[3,4-c]pyridine-1,4'-piperidin]-1'-yl)methyl]pyridin-2-yl}methylene)amino]oxy}acetonitrile

¹HNMR (400 MHz, CDCl₃, δ ppm): 1.70-1.88 (4H, m), 2.37-2.43 (2H, m), 2.72-2.76 (2H, m), 3.49 (3H, s), 3.57 (2H, s), 4.78 (2H, s), 4.82 (2H, s), 6.31 (1H, s), 7.08-7.13 (2H, m), 7.18-7.25 (2H, m), 7.76-7.80 (1H, brm), 7.85 (1H, d, J=8.0 Hz), 8.50 (1H, s).
ESI-MS Found: m/z 506[M+H]⁺

### Example 2-10-3:

### [({(1E)-(3,4-difluorophenyl)[5-(1H,1'H-spiro[furo[3,4-c]pyridine-3,4'-piperidin]-1'-ylmethyl)-2-pyridinyl]methylidene}amino)oxy]acetonitrile

¹HNMR (400 MHz, CDCl₃, δ ppm): 1.79-1.82 (2H, m), 2.00-2.08 (2H, m), 2.47-2.53 (2H, m), 2.81-2.84 (2H, m), 3.64 (2H, s), 4.86 (2H, s), 5.06 (2H, s), 7.12-7.16 (1H, m), 7.19 (1H, dd, J=8.4, 1.2 Hz), 7.22-7.29 (2H, m), 7.82-7.84 (1H, m), 7.89 (1H, d, J=8.4 Hz), 8.45 (1H, s), 8.52 (1H, d, J=4.8 Hz), 8.56 (1H, d, J=1.2 Hz).
ESI-MS Found: m/z 476[M+H]⁺

### Example 2-10-4:

### [({(1E)-(3,4-difluorophenyl)[3-(methyloxy)-4-(1H,1'H-spiro[furo[3,4-c]pyridine-3,4'-piperidin]-1'-ylmethyl)phenyl]methylidene}amino)oxy]acetonitrile

¹HNMR (400 MHz, CDCl₃, δ ppm): 1.78-1.83 (2H, m), 2.02-2.10 (2H, brm), 2.50-2.57 (2H, brm), 2.88-2.93 (2H, brm), 3.65 (2H, s), 3.79 (3H, s), 4.77 (2H, s), 5.04 (2H, s), 6.74 (1H, s), 6.85 (1H, d, J=7.6 Hz), 7.07-7.18 (2H, m), 7.19-7.21 (1H, brm), 7.40 (1H, ddd, J=11.2, 7.6, 1.9 Hz), 7.46-7.53 (1H, m), 8.44 (1H, s), 8.48 (1H, d, J=5.3 Hz).
ESI-MS Found: m/z 505[M+H]⁺

### Example 2-10-5:

### [({(1Z)-(3,4-difluorophenyl)[3-(methyloxy)-4-(1H,1'H-spiro[furo[3,4-c]pyridine-3,4'-piperidin]-1'-ylmethyl)phenyl]methylidene}amino)oxy]acetonitrile

¹HNMR (400 MHz, CDCl₃, δ ppm): 1.74-1.80 (2H, m), 2.00-2.06 (2H, brm), 2.45-2.54 (2H, brm), 2.83-2.88 (2H, brm), 3.64 (2H, s), 3.81 (3H, s), 4.78 (2H, s), 5.02 (2H, s), 6.84 (1H, d, J=7.8 Hz), 7.04-7.07 (1H, m), 7.11-7.26 (4H, m), 7.37 (1H, s), 8.42 (1H, s), 8.47 (1H, d, J=5.1 Hz).
ESI-MS Found: m/z 505[M+H)⁺

### Example 2-11:

In the same manner as in Example 2-1 but using the oxime obtained in Example 1-1-4 and acrylonitrile, the compounds of Examples 2-11-1 and 2-11-2 were obtained.

### Example 2-11-1:

### 3-{[((1Z)-(3,4-difluorophenyl){5-[(5-methyl-6-oxo-5,6-dihydro-1'H,3H-spiro[furo[3,4-c]pyridine-1,4'-piperidin]-1'-yl)methyl]pyridin-2-yl}methylene)amino]oxy}propanenitrile

¹HNMR (400 MHz, CDCl₃, δ ppm): 1.77-1.93 (4H, m), 2.43-2.50 (2H, m), 2.80 (2H, t, J=6.3 Hz), 2.80-2.85 (2H, m), 3.54 (3H, s), 3.63 (2H, s), 4.38 (2H, t, J=6.3 Hz), 4.83 (2H, d, J=1.5 Hz), 6.37 (1H, s), 7.08-7.20 (3H, m), 7.38 (1H, ddd, J=11.3, 7.7, 2.1 Hz), 7.59 (1H, d, J=7.8 Hz), 7.84-7.88 (1H, brm), 8.66 (1H, s).
ESI-MS Found: m/z 520[M+H]⁺

### Example 2-11-2:

### 3-{[((1E)-(3,4-difluorophenyl){5-[(5-methyl-6-oxo-5,6-dihydro-1'H,3H-spiro[furo[3,4-c]pyridine-1,4'-piperidin]-1'-yl)methyl]pyridin-2-yl}methylene)amino]oxy}propanenitrile

¹HNMR (400 MHz, CDCl₃, δ ppm): 1.75-1.90 (4H, m), 2.39-2.46 (2H, m), 2.75-2.80 (2H, m), 2.82 (2H, t, J=6.1 Hz), 3.53 (3H, s), 3.60 (2H, s), 4.44 (2H, t, J=6.1 Hz), 4.82 (2H, s), 6.35 (1H, s), 7.17-7.31 (4H, m), 7.79 (2H, s), 8.53 (1H, s).
ESI-MS Found: m/z 520[M+H]⁺

### Example 2-12:

In the same manner as in Example 2-1 but using the oxime obtained in Example 1-1-4 and methylvinyl sulfone, the compounds of Examples 2-12-1 and 2-12-2 were obtained.

### Example 2-12-1:

### 1'-{[6-((Z)-(3,4-difluorophenyl){[2-(methylsulfonyl)ethoxy]imino}methyl)pyridin-3-yl]methyl}-5-methyl-3,5-dihydro-6H-spiro[furo[3,4-c]pyridine-1,4'-piperidin]-6-one

¹HNMR (400 MHz, CDCl₃, δ ppm): 1.67-1.88 (4H, m), 2.40-2.47 (2H, m), 2.68 (3H, s), 2.75-2.80 (2H, m), 3.36 (2H, t, J=5.3 Hz), 3.50 (3H, s), 3.62 (2H, s), 4.60 (2H, t, J=5.3 Hz), 4.80 (2H, s), 6.33 (1H, s), 7.06-7.15 (3H, m), 7.32-7.42 (2H, m), 7.83 (1H, d, J=8.2 Hz), 8.60 (1H, s).
ESI-MS Found: m/z 573[M+H]⁺

### Example 2-12-2:

### 1'-{[6-((E)-(3,4-difluorophenyl){[2-(methylsulfonyl)ethoxy]imino}methyl)pyridin-3-yl]methyl}-5-methyl-3,5-dihydro-6H-spiro[furo[3,4-c]pyridine-1,4'-piperidin]-6-one

¹HNMR (400 MHz, CDCl₃, δ ppm): 1.71-1.86 (4H, m), 2.36-2.42 (2H, m), 2.70 (3H, s), 2.70-2.75 (2H, m), 3.39 (2H, t, J=5.5 Hz), 3.50 (3H, s), 3.56 (2H, s), 4.66 (2H, t, J=5.5 Hz), 4.78 (2H, s), 6.31 (1H, s), 7.08-7.14 (2H, m), 7.17-7.24 (2H, m), 7.75 (2H, s), 8.49 (1H, s).
ESI-MS Found: m/z 573[M+H]⁺

### Example 2-13:

In the same manner as in Example 2-1 but using (1-hydroxycyclopropyl)methyl methanesulfonate which was obtained in the same manner as in Reference Example 5-1 but using 1-(hydroxymethyl)cyclopropanol, and the oxime obtained in Example 1-1-4, the compounds of Examples 2-13-1 and 2-13-2 were obtained.

### Example 2-13-1:

### 1'-{[6-((Z)-(3,4-difluorophenyl){[(1-hydroxycyclopropyl)methoxy]imino}methyl)pyridin-3-yl]methyl}-5-methyl-3,5-dihydro-6H-spiro[furo[3,4-c]pyridine-1,4'-piperidin]-6-one

¹HNMR (400 MHz, CDCl₃, δ ppm): 0.60 (2H, t, J=6.2 Hz), 0.81 (2H, t, J=6.2 Hz), 1.75-1.92 (4H, m), 2.45-2.51 (2H, m), 2.81-2.85 (2H, m), 3.50 (3H, s), 3.63 (2H, s), 4.18 (2H, s), 4.80 (2H, s), 6.33 (1H, s), 7.08-7.15 (3H, m), 7.30-7.42 (2H, m), 7.88 (1H, brs), 8.61 (1H, s).
ESI-MS Found: m/z 537[M+H]⁺

### Example 2-13-2:

### 1'-{[6-((E)-(3,4-difluorophenyl){[(1-hydroxycyclopropyl)methoxy]imino}methyl)pyridin-3-yl]methyl}-5-methyl-3,5-dihydro-6H-spiro[furo[3,4-c]pyridine-1,4'-piperidin]-6-one

¹HNMR (400 MHz, CDCl₃, δ ppm): 0.67 (2H, t, J=6.2 Hz), 0.86 (3H, t, J=6.2 Hz), 1.70-1.87 (4H, m), 2.36-2.42 (2H, m), 2.74-2.76 (2H, m), 3.49 (3H, s), 3.56 (2H, s), 4.26 (2H, s), 4.78 (2H, s), 6.31 (1H, s), 7.13-7.22 (3H, m), 7.28-7.33 (1H, m), 7.74 (2H, s), 8.48 (1H, s).
ESI-MS Found: m/z 537[M+H]⁺

### Example 2-14:

### Production of (Z)-(3,4-difluorophenyl)[5-(1H,1'H-spiro[furo[3,4-c]pyridine-3,4'-piperidin[-1'-ylmethyl)pyridin-2-yl]methanone O-(2-hydroxy-2-methylpropyl)oxime and (E)-(3,4-difluorophenyl)[5-(1H,1'H-spiro[furo[3,4-c]pyridine-3,4'-piperidin]-1'-ylmethyl)pyridin-2-yl]methanone O-(2-hydroxy-2-methylpropyl)oxime

At 0°C, potassium carbonate (3.17 g) and 1,2-epoxyisobutane (4.13 mL) were added to a DMF solution (4.50 mL) of the compound (1.00 g) obtained in Example 1-1-2, and stirred at room temperature for 2 days. Aqueous sodium hydrogencarbonate solution was added to the reaction liquid, and extracted with chloroform. The organic layer was washed with water and saturated saline, and dried with anhydrous sodium sulfate. After the organic layer was concentrated under reduced pressure, the residue was purified through preparative thin-layer silica gel chromatography (chloroform/methanol = 9/1). This was subjected to separation of geometric isomers through CHIRALPAK AD (hexane/isopropyl alcohol/diethylamine = 50/50/0.05) to obtain the entitled compound (Z)-form (108 mg, faster), and the entitled compound (E)-form (29.5 mg, later), as a white amorphous substance.

### Entitled Compound (Z)-form:

¹HNMR (400 MHz, CDCl₃, δ ppm): 1.21 (6H, s), 1.82-1.85 (2H, m), 2.04-2.08 (2H, m), 2.52-2.56 (2H, m), 2.84-2.88 (2H, m), 3.68 (2H, s), 4.10 (2H, s), 5.08 (2H, s), 7.09-7.25 (2H, m), 7.20 (1H, d, J=4.8 Hz), 7.33-7.39 (1H, m), 7.48-7.50 (1H, m), 7.89 (1H, brs), 8.47 (1H, s), 8.53 (1H, d, J=4.8 Hz), 8.69-8.70 (1H, m).
ESI-MS Found: m/z 509[M+H]⁺

### Entitled Compound (E)-form:

¹HNMR (400 MHz, CDCl₃, δ ppm): 1.25 (6H, s), 1.79-1.82 (2H, m), 2.00-2.04 (2H, m), 2.46-2.51 (2H, m), 2.81-2.84 (2H, m), 3.62 (2H, s), 4.15 (2H, s), 5.05 (2H, s), 7.15-7.25 (3H, m), 7.29-7.34 (1H, m), 7.79 (2H, s), 8.45 (1H, s), 8.51 (1H, d, J=4.8 Hz), 8.54 (1H, s).
ESI-MS Found: m/z 509[M+H]⁺

In the same manner as in Examples 1-1 and 2-14 but using various ketones obtained in Reference Example 5, the compounds of Examples 2-14-1 to 2-14-5 were obtained.

### Example 2-14-1:

### (Z)-(5-chloro-2-pyridinyl)[5-(1H,1'H-spiro[furo[3,4-c]pyridine-3,4'-piperidin]-1'-ylmethyl)-2-pyridinyl]methanone O-(2-hydroxy-2-methylpropyl)oxime

¹HNMR (400 MHz, CDCl₃, δ ppm): 1.18 (6H, s), 1.73-1.78 (2H, m), 1.94-2.03 (2H, m), 2.40-2.47 (2H, m), 2.76-2.81 (2H, m), 3.57 (2H, s), 4.11 (2H, s), 5.01 (2H, s), 7.15 (1H, d, J=4.7 Hz), 7.47 (1H, d, J=8.4 Hz), 7.77 (2H, dd, J=8.4, 2.2 Hz), 7.84 (1H, d, J=8.0 Hz), 8.41 (1H, s), 8.46-8.48 (2H, m), 8.63 (1H, d, J=2.2 Hz).
ESI-MS Found: m/z 508[M+H]⁺

### Example 2-14-2:

### (E)-(3,4-difluorophenyl)[5-(1H,1'H-spiro[furo[3,4-c]pyridine-3,4'-piperidin]-1'-ylmethyl)-2-pyrimidinyl]methanone O-(2-hydroxy-2-methylpropyl)oxime

¹HNMR (400 MHz, CDCl₃, δ ppm): 1.20 (6H, s), 1.75-1.78 (2H, m), 1.93-2.01 (2H, m), 2.44-2.51 (2H, m), 2.73-2.78 (2H, m), 3.59 (2H, s), 4.21 (2H, s), 5.02 (2H, s), 7.15-7.25 (3H, m), 7.31 (1H, ddd, J=10.6, 7.7, 1.7 Hz), 8.41 (1H, s), 8.48 (1H, d, J=4.9 Hz), 8.75 (2H, s).
ESI-MS Found: m/z 510[M+H]⁺

### Example 2-14-3:

### (E)-(3,4-difluorophenyl) {5-[(6-fluoro-1H,1'H-spiro[furo[3,4-c]pyridine-3,4'-piperidin]-1'-yl)methyl]-2-pyrimidinyl}methanone O-(2-hydroxy-2-methylpropyl)oxime

¹HNMR (400 MHz, CDCl₃, δ ppm): 1.20 (6H, s), 1.76-1.79 (2H, m), 1.90-1.97 (2H, m), 2.43-2.49 (2H, m), 2.74-2.76 (2H, m), 3.58 (2H, s), 4.21 (2H, s), 5.00 (2H, s), 6.73 (1H, s), 7.15-7.25 (2H, m), 7.27-7.33 (1H, m), 7.94 (1H, s), 8.74 (2H, s).
ESI-MS Found: m/z 528[M+H]⁺

### Example 2-14-4:

### 1'-[(6-{(Z)-(3,4-difluorophenyl)[(2-hydroxy-2-methylpropoxy)imino]methyl}pyridin-3-yl)methyl]-5-methyl-3,5-dihydro-6H-spiro[furo[3,4-c]pyridine-1,4'-piperidin]-6-one

¹HNMR (400 MHz, CDCl₃, δ ppm): 1.17 (6H, s), 1.77-2.01 (4H, m), 2.58-2.66 (2H, m), 2.96-2.99 (2H, m), 3.50 (3H, s), 3.76 (2H, s), 4.06 (2H, s), 4.80 (2H, s), 6.34 (1H, s), 7.07-7.16 (3H, m), 7.29-7.35 (1H, m), 7.48 (1H, d, J=8.2 Hz), 7.89-7.93 (1H, m), 8.64 (1H, s).
ESI-MS Found: m/z 539[M+H]⁺

### 1'-[(6-{(E)-(3,4-difluorophenyl)[(2-hydroxy-2-methylpropoxy)imino]methyl}pyridin-3-yl)methyl]-5-methyl-3,5-dihydro-6H-spiro[furo[3,4-c]pyridine-1,4'-piperidin]-6-one

¹HNMR (400 MHz, CDCl₃, δ ppm): 1.21 (6H, s), 1.71-1.89 (4H, m), 2.40-2.45 (2H, m), 2.75-2.80 (2H, m), 3.49 (3H, s), 3.59 (2H, s), 4.11 (2H, s), 4.78 (2H, d, J=4.9 Hz), 6.31 (1H, s), 7.11-7.30 (4H, m), 7.75 (2H, s), 8.47 (1H, s).
ESI-MS Found: m/z 539[M+H]⁺

### Example 2-14-5:

### 1'-{[4-((Z)-(5-chloro-2-pyridinyl){[(2-hydroxy-2-methylpropyl)oxy]imino}methyl)phenyl]methyl}-5-methyl-3,5-dihydro-6H-spiro[furo[3,4-c]pyridine-1,4'-piperidin]-6-one

¹HNMR (400 MHz, CDCl₃, δ ppm): 1.17 (6H, s), 1.68-1.88 (4H, m), 2.30-2.40 (2H, brm), 2.72-2.78 (2H, brm), 3.49 (3H, s), 3.53 (2H, brs), 4.06 (2H, s), 4.77 (2H, s), 6.32 (1H, s)7.12 (1H, s), 7.29-7.32 (2H, brm), 7.36 (2H, d, J=7.6 Hz), 7.44 (1H, d, J=8.4 Hz), 7.76 (1H, dd, J=8.4, 2.3 Hz), 8.66 (1H, d, J=2.3 Hz).
ESI-MS Found: m/z 537[M+H]⁺

### Example 3-1:

### Production of (E)-{5-[(6-bromo-1H,1'H-spiro[furo[3,4-c]pyridine-3,4'-piperidin]-1'-yl)methyl]-2-pyridinyl(3,4-difluorophenyl)methanone O-(2-hydroxy-2-methylpropyl)oxime

In the same manner as in Reference Example 5-1 but using the (E)-form compound (150 mg) obtained in Reference Example 7-1, a crude product (191 mg) of the corresponding mesylate was obtained as a yellow oil. At 0°C, trifluoroacetic acid (2.00 mL) was added to the compound (165 mg) obtained in Reference Example 4-37, and stirred for 20 minutes. The reaction liquid was concentrated under reduced pressure, and chloroform (2.00 mL) was added to the residue, and at 0°C, a chloroform solution (2.50 mL) of diisopropylethylamine (380 µL) and the above mesylate (190 mg) was dropwise added to it. After stirred at 0°C for 1 hour, this was further stirred overnight at room temperature. Aqueous sodium hydrogencarbonate solution was added to the reaction liquid, and extracted with chloroform. The organic layer was washed with saturated saline, and dried with anhydrous sodium sulfate. After the organic layer was concentrated under reduced pressure, the residue was purified through preparative thin-layer silica gel chromatography (chloroform/methanol = 95/5) to obtain the entitled compound (224 mg) as a white amorphous substance.
¹HNMR (400 MHz, CDCl₃, δ ppm): 1.24 (6H, s), 1.78-1.81 (2H, m), 1.93-2.00 (2H, m), 2.42-2.48 (2H, m), 2.78-2.81 (2H, m), 3.48 (1H, s), 3.60 (2H, s), 4.14 (2H, s), 5.01 (2H, s), 7.14-7.26 (2H, m), 7.29-7.33 (1H, m), 7.36 (1H, s), 7.77 (2H, s), 8.18 (1H, s), 8.53 (1H, s).
ESI-MS Found: m/z 587[M+H]

In the same manner as in Example 3-1 but using various oxime alcohols (IVc') obtained in Reference Example 8 according to a stereospecific production method, and various amine precursors or amines (V) obtained in Reference Example 4, the compounds of Examples 3-1-1 to 3-1-11 were obtained.

### Example 3-1-1:

### 1'-{[4-((Z)-(3,4-difluorophenyl){[(2-fluoroethyl)oxy]imino}methyl)phenyl]methyl}-1H-spiro[furo[3,4-c]pyridine-3,4'-piperidin]-6(5H)-one

¹HNMR (400 MHz, CDCl₃, δ ppm): 1.75-1.80 (4H, m), 2.36-2.43 (2H, m), 2.71-2.75 (2H, m), 3.55 (2H, s), 4.38 (2H, dt, J=28.4, 4.1 Hz), 4.65 (2H, dt, J=47.6, 4.1 Hz), 4.84 (2H, s), 6.36 (1H, s), 7.08-7.27 (4H, m), 7.30 (2H, d, J=8.3 Hz), 7.38 (2H, d, J=8.3 Hz).
ESI-MS Found: m/z 498[M+H]⁺

### Example 3-1-2:

### (Z)-[3,4-bis(methyloxy)phenyl][4-(1H,1'H-spiro[furo[3,4-c]pyridine-3,4'-piperidin]-1'-ylmethyl)phenyl]methanone O-(2-fluoroethyl)oxime

¹HNMR (400 MHz, CDCl₃, δ ppm): 1.74-1.78 (2H, m), 1.95-2.03 (2H, m), 2.38-2.44 (2H, m), 2.79-2.83 (2H, m), 3.57 (2H, s), 3.82 (3H, s), 3.89 (3H, s), 4.39 (2H, dt, J=28.7, 4.1 Hz), 4.67 (2H, dt, J=47.7, 4.1 Hz), 5.02 (2H, s), 6.86-6.92 (2H, m), 7.04 (1H, s), 7.14 (1H, d, J=4.9 Hz), 7.31 (2H, d, J=8.0 Hz), 7.43 (2H, d, J=8.0 Hz), 8.42 (1H, s), 8.47 (1H, d, J=5.1 Hz).
ESI-MS Found: m/z 506[M+H]⁺

### Example 3-1-3:

### 1'-({5-[(E)-(3,4-difluorophenyl)(ethoxyimino)methyl]pyridin-2-yl}methyl)-5-methyl-3,5-dihydro-6H-spiro[furo[3,4-c]pyridine-1,4'-piperidin]-6-one

¹HNMR (400 MHz, CDCl₃, δ ppm): 1.28 (3H, t, J=7.0 Hz), 1.71-1.75 (2H, m), 1.80-1.94 (2H, m), 2.43-2.49, 2H, m), 2.78-2.81 (2H, m), 3.49 (3H, s), 3.70 (2H, s), 4.23 (2H, q, J=7.0 Hz), 4.79 (2H, s), 6.31 (1H, s), 7.05-7.08 (1H, m), 7.13-7.21 (2H, m), 7.24-7.29 (1H, m), 7.40 (1H, d, J=7.8 Hz), 7.73 (1H, dd, J=8.3, 1.9 Hz), 8.58 (1H, s).
ESI-MS Found: m/z 495[M+H]⁺

### Example 3-1-4:

### 1'-({5-[(E)-(3,4-difluorophenyl)(ethoxyimino)methyl]pyridin-2-yl}methyl)-5-methyl-1H-spiro[furo[3,4-c]pyridine-3,4'-piperidin]-6(5H)-one

¹HNMR (400 MHz, CDCl₃, δ ppm): 1.32 (3H, t, J=7.2 Hz), 1.79-1.82 (2H, m), 1.87-1.92 (2H, m), 2.50-2.55 (2H, m), 2.81-2.84 (2H, m), 3.54 (3H, s), 3.75 (2H, s), 4.27 (2H, q, J=7.2 Hz), 4.84 (2H, s), 6.39 (1H, s), 7.07 (1H, s), 7.09-7.12 (1H, m), 7.20-7.32 (2H, m), 7.41 (1H, d, J=8.4 Hz), 7.75 (1H, dd, J=8.4, 2.0 Hz), 8.62 (1H, d, J=2.0 Hz).
ESI-MS Found: m/z 495[M+H]⁺

### Example 3-1-5:

### (E)-(3,4-difluorophenyl){6-[(5-oxido-1'H,3H-spiro[furo[3,4-c]pyridine-1,4'-piperidin]-1'-yl)methyl]pyridin-3-yl}methanone O-ethyloxime

¹HNMR (400 MHz, CDCl₃, δ ppm): 1.33-1.34 (3H, m), 1.78 (2H, d, J=12.2 Hz), 1.92-2.06 (2H, m), 2.53 (2H, t, J=11.2 Hz), 2.87 (2H, d, J=11.2 Hz), 3.75 (2H, s), 4.23-4.32 (2H, m), 5.05 (2H, s), 7.03 (1H, d, J=6.3 Hz), 7.07-7.14 (1H, m), 7.18-7.34 (2H, m), 7.40 (1H, d, J=7.8 Hz), 7.73-7.78 (1H, m), 8.08-8.16 (2H, m), 8.63 (1H, d, J=2.4 Hz).
ESI-MS Found: m/z 481 [M+H]

### Example 3-1-6:

### 1'-({6-[(E)-(3,4-difluorophenyl)(ethoxyimino)methyl]pyridin-3-yl}methyl)-3,5-dihydro-6H-spiro[furo[3,4-c]pyridine-1,4'-piperidin]-6-one

¹HNMR (400 MHz, CDCl₃, δ ppm): 1.29 (3H, t, J=7.0 Hz), 1.59-1.85 (4H, m), 2.35-2.43 (2H, m), 2.74-2.78 (2H, m), 3.55 (2H, s), 4.27 (2H, q, J=7.0 Hz), 4.81 (2H, s), 6.29 (1H, s), 7.12-7.18 (3H, m), 7.24-7.30 (1H, m), 7.72-7.78 (2H, m), 8.48 (1H, s), 12.70 (1H, s).
ESI-MS Found: m/z 481[M+H]⁺

### Example 3-1-7:

### 1'-({6-[(E)-(3,4-difluorophenyl)(ethoxyimino)methyl]pyridin-3-yl}methyl)-5-ethyl-3,5-dihydro-6H-spiro[furo[3,4-c]pyridine-1,4'-piperidin]-6-one

¹HNMR (400 MHz, CDCl₃, δ ppm): 1.29 (3H, t, J=7.0 Hz), 1.32 (3H, t, J=6.9 Hz), 1.70-1.83 (4H, m), 2.34-2.41 (2H, m), 2.71-2.76 (2H, m), 3.55 (2H, s), 3.94 (2H, q, J=7.1 Hz), 4.27 (2H, q, J=6.9 Hz), 4.78 (2H, s), 6.30 (1H, s), 7.10-7.30 (4H, m), 7.71-7.78 (2H, m), 8.46 (1H, s).
ESI-MS Found: m/z 509[M+H]⁺

### Example 3-1-8:

### (E)-(3,4-difluorophenyl){5-[(6-ethoxy-1'H,3H-spiro[furo[3,4-c]pyridine-1,4'-piperidin]-1'-yl)methyl]pyridin-2-yl}methanone O-ethyloxime

¹HNMR (400 MHz, CDCl₃, δ ppm): 1.30 (3H, t, J=7.1 Hz), 1.36 (3H, t, J=7.1 Hz), 1.70-1.73 (2H, m), 1.82-1.91 (2H, m), 2.38-2.45 (2H, m), 2.73-2.79 (2H, m), 3.56 (2H, s), 4.26 (2H, t, J=7.1 Hz), 4.32 (2H, t, J=7.1 Hz), 4.97 (2H, s), 6.46 (1H, s), 7.12-7.21 (2H, m), 7.25-7.30 (1H, m), 7.70-7.77 (2H, m), 7.95 (1H, s), 8.49 (1H, s).
ESI-MS Found: m/z 509[M+H]⁺

### Example 3-1-9:

### 1'-({6-[(E)-(3,4-difluorophenyl)(ethoxyimino)methyl]pyridin-3-yl}methyl)-5-methyl-3,5-dihydro-4H-spiro[furo[3,4-c]pyridine-1,4'-piperidin]-4-one

¹HNMR (400 MHz, CDCl₃, δ ppm): 1.33 (3H, t, J=7.2 Hz), 1.71-1.74 (2H, m), 1.85-1.92 (2H, m), 2.41-2.47 (2H, m), 2.78-2.81 (2H, m), 3.57 (3H, s), 3.59 (2H, s), 4.30 (2H, q, J=7.2 Hz), 5.00 (2H, s), 6.05 (1H, d, J=6.8 Hz), 7.13-7.24 (2H, m), 7.29-7.34 (2H, m), 7.72-7.74 (1H, m), 7.79 (1H, d, J=6.8 Hz), 8.54 (1H, s).
ESI-MS Found: m/z 495[M+H]⁺

### Example 3-1-10:

### N-{1'-[(6-{(E)-(3,4-difluorophenyl)[(ethyloxy)imino]methyl}-3-pyridinyl)methyl]-3H-spiro[furo[3,4-c]pyridine-1,4'-piperidin]-6-yl}acetamide

¹HNMR (400 MHz, CDCl₃, δ ppm): 1.33 (3H, t, J=7.2 Hz), 1.74-1.77 (2H, m), 1.99-2.07 (2H, m), 2.21 (3H, s), 2.43-2.48 (2H, m), 2.80-2.83 (2H, m), 3.60 (2H, s), 4.30 (2H, q, J=7.2 Hz), 5.05 (2H, s), 7.14-7.24 (1H, m), 7.30-7.35 (1H, m), 7.81 (2H, s), 8.08 (1H, s), 8.11 (1H, s), 8.35 (1H, brs), 8.50 (1H, s).
ESI-MS Found: m/z 522[M+H]⁺

### Example 3-1-11:

### (E)-(3,4-difluorophenyl){5-[(5-oxido-1H,1'H-spiro[furo[3,4-c]pyridine-3,4'-piperidinl-1'-yl)methyl]-2-pyridinyl}methanone O-ethyloxime

¹HNMR (400 MHz, CDCl₃, δ ppm): 1.31 (3H, t, J=7.4 Hz), 1.78-1.95 (4H, m), 2.40-2.50 (2H, m), 2.75-2.85 (2H, m), 3.60 (2H, s), 4.30 (2H, q, J=7.4 Hz), 5.02 (2H, s), 7.12-7.25 (3H, m), 7.31 (1H, ddd, J=2.0, 8.0, 11.2 Hz), 7.76 (1H, dd, J=7.8, 2.0 Hz), 7.81 (1H, d, J=7.8 Hz), 8.06 (1H, s), 8.14 (1H, dd, J=6.8, 1.5 Hz), 8.51 (1H, d, J=1.0 Hz).
APCI-MS Found: m/z 481[M+H]⁺

In addition to the above-mentioned Examples, the following compounds were prepared according to the same methods as the above-mentioned methods.

### Example 3-1-12:

### (Z)-(3,4-difluorophenyl)[3-[(2-fluoroethyl)oxy]-4-(1H,1'H-spiro[furo[3,4-c]pyridine-3,4'-piperidin]-1'-ylmethyl)phenyl]methanone O-(2-hydroxyethyl)oxime

¹HNMR (400 MHz, CDCl₃, δ ppm): 1.75-1.85 (2H, m), 1.95-2.10 (2H, m), 2.47-2.60 (2H, m), 2.82-2.94 (2H, m), 3.70 (2H, s), 3.92-3.94 (2H, m), 4.15-4.28 (2H, m), 4.30-4.35 (2H, m), 4.65-4.85 (2H, m), 5.05 (2H, s), 6.92 (1H, dd, J=1.6, 8.0 Hz), 7.05 (1H, d, J=1.6 Hz), 7.10-7.13 (1H, m), 7.17-7.31 (3H, m), 7.41 (1H, d, J=7.8 Hz), 8.45 (1H, s), 8.50 (1H, d, J=4.9 Hz).
ESI-MS Found: m/z 542[M+H]⁺

### Example 3-1-13 and Example 3-1-14:

### (E)-(3,4-difluorophenyl){5-[1-(1H,1'H-spiro[furo[3,4-c]pyridine-3,4'-piperidin]-1'-yl)ethyl]-2-pyridinyl}methanone O-(2-hydroxy-2-methylpropyl)oxime

After the above racemic compound was produced, it was optically resolved under the condition mentioned below to obtain each enantiomers.

### Example 3-1-13 (R) or (S), CHIRALPAK AD-H (hexane/isopropyl alcohol/diethylamine = 20/80/0.08), faster

¹H-NMR (400 MHz, CDCl₃, δ ppm): 1.21 (6H, s), 1.40 (3H, d, J=6.7 Hz), 1.68-2.03 (4H, m), 2.35-2.44 (2H, m), 2.66-2.71 (1H, m), 2.89-2.93 (1H, m), 3.58 (1H, q, J=6.7 Hz), 4.11 (2H, s), 4.99 (2H, s), 7.13-7.22 (3H, m), 7.28 (1H, ddd, J=11.0, 7.6, 2.0 Hz), 7.72-7.75 (2H, m), 8.40 (1H, s), 8.47 (1H, d, J=4.7 Hz), 8.52 (1H, s).
ESI-MS Found: m/z 523[M+H]⁺

### Example 3-1-14 (R) or (S), CHIRALPAK AD-H (hexane/isopropyl alcohol/diethylamine = 20/80/0.08), slower

¹H-NMR (400 MHz, CDCl₃, δ ppm): 1.21 (6H, s), 1.40 (3H, d, J=6.7 Hz), 1.68-2.03 (4H, m), 2.35-2.44 (2H, m), 2.66-2.71 (1H, m), 2.89-2.93 (1H, m), 3.58 (1H, q, J=6.7 Hz), 4.11 (2H, s), 4.99 (2H, s), 7.13-7.22 (3H, m), 7.28 (1H, ddd, J=11.0, 7.6, 2.0 Hz), 7.72-7.75 (2H, m), 8.40 (1H, s), 8.47 (1H, d, J=4.7 Hz), 8.52 (1H, s).
ESI-MS Found: m/z 523[M+H]⁺

### Example 3-1-15:

### 1'-[(6-{(E)-(3,4-difluorophenyl)[(fluoromethoxy)imino]methyl}-3-pyridinyl)methyl]-5-methyl-3,5-dihydro-6H-spiro[furo[3,4-c]pyridine-1,4'-piperidin]-6-one

¹HNMR (400 MHz, CDCl₃, δ ppm): 1.75-1.95 (4H, m), 2.36-2.48 (2H, m), 2.72-2.82 (2H, m), 3.53 (3H, s), 3.60 (2H, s), 4.81 (2H, s), 5.79 (2H, d, J = 55.2 Hz), 6.34 (1H, s), 7.13-7.34 (4H, m), 7.77-7.89 (2H, m), 8.54 (1H, d, J = 1.6 Hz).
ESI-MS Found: m/z 499[M+H]⁺

### INDUSTRIAL APPLICABILITY

The compounds of the invention have an MCH-1R antagonistic effect and are useful, for example, as a preventive or a remedy for metabolic disorders such as obesity, diabetes, hormone disorder, hyperlipidemia, gout, fatty liver, hepatitis, cirrhosis; cardiovascular disorders such as stenocardia, acute or congestive heart failure, myocardial infarction, coronary atherosclerosis, hypertension, renal diseases, electrolyte abnormality; central nervous system or peripheral nervous system disorders such as bulimia, emotional disturbance, depression, anxiety, epilepsy, delirium, dementia, schizophrenia, attention-deficit hyperactivity disorder, memory impairment, sleep disorders, cognitive failure, dyskinesia, paresthesias, smell disorders, morphine tolerance, drug dependence, alcoholism; reproductive disorders such as infertility, preterm labor and sexual dysfunction; and other digestive disorders, respiratory disorders, cancer or pigmentation et al.

## Claims

1. A diarylketimine derivative of a formula (I) or a pharmaceutically-acceptable salt thereof: 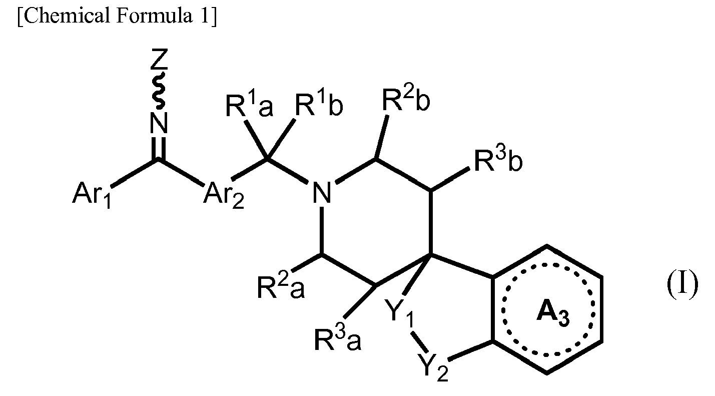 [wherein R^{1a} and R^{1b} are the same or different, each representing a hydrogen atom, or a C₁₋₆ alkyl group optionally having a substituent;
R^{2a} and R^{2b} are the same or different, each representing a hydrogen atom, or a C₁₋₆ alkyl group optionally having a substituent, or R^{2a} and R^{2b}, taken together, form -C(R⁴)₂-C(R⁵)₂-;
R^{3a} and R^{3b} are the same or different, each representing a hydrogen atom, or a C₁₋₆ alkyl group optionally having a substituent, or R^{3a} and R^{3b}, taken together, form -C(R⁶)₂-C(R⁷)₂-;
Y₁ represents -O- or -C(R⁸)₂-;
Y₂ represents -C(O)- or -C(R⁹)₂-, or Y₁ and Y₂, taken together, form -C(R¹⁰)=C(R¹¹)-;
Z represents -OR¹², -N(R^{13a})(R^{13b}), -NR¹⁴-COOR¹⁵, -NR¹⁶-COR¹⁷, -C(R^{18a})(R^{18b})(R^{18c}),-O-SO₂R¹⁹ or -SO₂R²⁰;
R⁴, R⁵, R⁶, R⁷, R⁸, R⁹, R¹⁰, R¹¹, R^{13a}, R^{13b}, R¹⁴, R¹⁵, R¹⁶, R¹⁷, R^{18a}, R^{18b} and R^{18c} are the same or different, each representing a hydrogen atom, or a C₁₋₆ alkyl group optionally having a substituent;
R¹² represents a hydrogen atom, a C₁₋₆ alkyl group optionally having a substituent, or a C₃₋₆ cycloalkyl group optionally having a substituent, wherein the C₁₋₆ alkyl group or the C₃₋₆ cycloalkyl group may be substituted with a substituent selected from a group consisting of halogen, hydroxy, C₁₋₆ alkoxy, C₁₋₆ alkoxycarbonyl, C₁₋₆ alkylsulfonyl, (C₁₋₆ alkyl)amino, di(C₁₋₆ alkyl)amino, carbamoyl, (C₁₋₆ alkyl)carbamoyl, di(C₁₋₆ alkyl)carbamoyl and cyano;
R¹⁹ and R²⁰ each represent a C₁₋₆ alkyl group or a phenyl group optionally substituted with a C₁₋₆ alkyl group;
Ar₁ represents a 6-membered aromatic carbocyclic group optionally substituted with a substituent selected from a group α, or represents a 6-membered aromatic nitrogen-containing heterocyclic group optionally substituted with a substituent selected from the group α;
Ar₂ represents a group derived from a 6-membered aromatic carbocyclic ring, a 6-membered aromatic nitrogen-containing heterocyclic ring, a 5-membered aromatic heterocyclic ring or a pyridone ring by removing two hydrogen atoms from the ring, wherein the 6-membered aromatic carbocyclic ring, the 6-membered aromatic nitrogen-containing heterocyclic ring, the 5-membered aromatic heterocyclic ring or the pyridone ring may be optionally substituted with a substituent selected from the group α;
the formula: 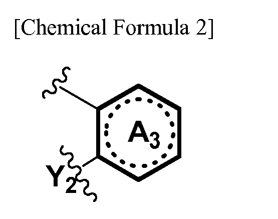
[this is hereinafter referred to as A₃] represents a group selected from the following group: 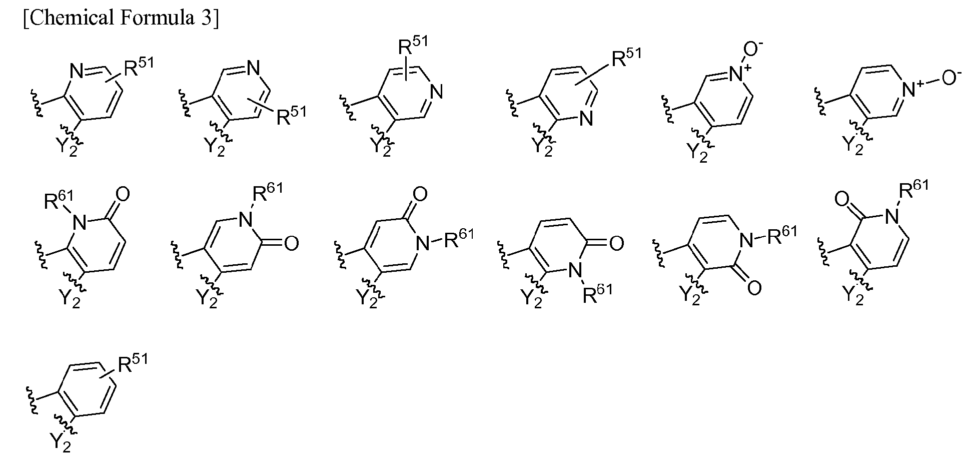 [wherein R⁵¹ represents a hydrogen atom, a hydroxyl group, a halogen, a C₁₋₆ alkyl group, a halo-C₁₋₆ alkyl group, a C₁₋₆ alkyloxy group, a halo-C₁₋₆ alkyloxy group or a C₁₋₆ alkylcarbonylamino group; R⁶¹ represents a hydrogen atom, or a C₁₋₆ alkyl group optionally having a substituent]];
Substituent group α:
halogen, cyano, hydroxy, amino, mono-C₁₋₆ alkylamino, di-C₁₋₆ alkylamino, C₁₋₆ alkyl, halo-C₁₋₆ alkyl, C₁₋₆ alkyloxy, halo-C₁₋₆ alkyloxy, C₁₋₆ alkyloxy-C₁₋₆ alkyl, C₁₋₆ alkyloxycarbonyl, C₁₋₆ alkyloxycarbonylamino, C₁₋₆ alkyloxycarbonyl(C₁₋₆ alkyl)amino, C₁₋₆ alkylcarbonyl, C₁₋₆ alkylcarbonyloxy, C₁₋₆ alkylcarbonylamino, C₁₋₆ alkylcarbonyl(C₁₋₆ alkyl)amino, carbamoyl, mono-C₁₋₆ alkylcarbamoyl, di-C₁₋₆ alkylcarbamoyl, carbamoylamino, mono-C₁₋₆ alkylcarbamoylamino, di-C₁₋₆ alkylcarbamoylamino, mono-C₁₋₆ alkylcarbamoyl(C₁₋₆ alkyl)amino, di-C₁₋₆ alkylcarbamoyl(C₁₋₆ alkyl)amino, carbamoyloxy, mono-C₁₋₆ alkylcarbamoyloxy, di C₁₋₆-alkylcarbamoyloxy, C₁₋₆ alkylsulfonyl, C₁₋₆ alkylsulfonylamino, C₁₋₆ alkylsulfonyl-(C₁₋₆ alkyl)amino, sulfamoyl, mono-C₁₋₆ alkylsulfamoyl, di-C₁₋₆ alkylsulfamoyl, sulfamoylamino, mono-C₁₋₆ alkylsulfamoylamino, di-C₁₋₆ alkylsulfamoylamino, mono-C₁₋₆ alkylsulfamoyl(C₁₋₆ alkyl)amino and di-C₁₋₆ alkylsulfamoyl(C₁₋₆ alkyl)amino.

2. The compound or the pharmaceutically-acceptable salt thereof as claimed in claim 1, wherein R^{1a} and R^{1b} are the same or different, each representing a hydrogen atom or a methyl group.

3. The compound or the pharmaceutically-acceptable salt thereof as claimed in claim 1 or 2, wherein R^{2a} and R^{2b} are the same or different, each representing a hydrogen atom or a methyl group.

4. The compound or the pharmaceutically-acceptable salt thereof as claimed in claim 1 or 2, wherein R^{2a} and R^{2b}, taken together, form -CH₂-CH₂-.

5. The compound or the pharmaceutically-acceptable salt thereof as claimed in any of claims 1 to 4, wherein R^{3a} and R^{3b} are both hydrogen atoms.

6. The compound or the pharmaceutically-acceptable salt thereof as claimed in any of claims 1 to 5, wherein Ar₁ is a 6-membered aromatic carbocyclic group substituted with one or two fluorine atoms or chlorine atoms, or a 6-membered aromatic nitrogen-containing heterocyclic group substituted with one or two fluorine atoms or chlorine atoms.

7. The compound or the pharmaceutically-acceptable salt thereof as claimed in claim 6, wherein Ar₁ is a phenyl group substituted with one or two fluorine atoms or chlorine atoms, or a pyridinyl group substituted with one or two fluorine atoms or chlorine atoms.

8. The compound or the pharmaceutically-acceptable salt thereof as claimed in any of claims 1 to 7, wherein Ar₂ is a group derived from a benzene ring, a pyridine ring, a pyrimidine ring, a pyrazine ring, a pyridazine ring or a pyridone ring by removing two hydrogen atoms from the ring.

9. The compound or the pharmaceutically-acceptable salt thereof as claimed in claim 8, wherein Ar₂ is a 1,4-phenylenediyl group, a 3-methoxyphenylene-1,4-diyl group, a 3-methanesulfonylphenylene-1,4-diyl group, a 2-fluorophenylene-1,4-diyl group, a 3-fluorophenylene-1,4-diyl group, a 2-methylphenylene-1,4-diyl group, a 3-methylphenylene-1,4-diyl group, a pyridine-2,5-diyl group, a pyrimidine-2,5-diyl group, a pyrazine-2,5-diyl group, a pyridazine-3,6-diyl group, a thiophene-2,5-diyl group, or a pyridone-diyl group.

10. The compound or the pharmaceutically-acceptable salt thereof as claimed in any of claims 1 to 9, wherein A₃ is selected from the following group: 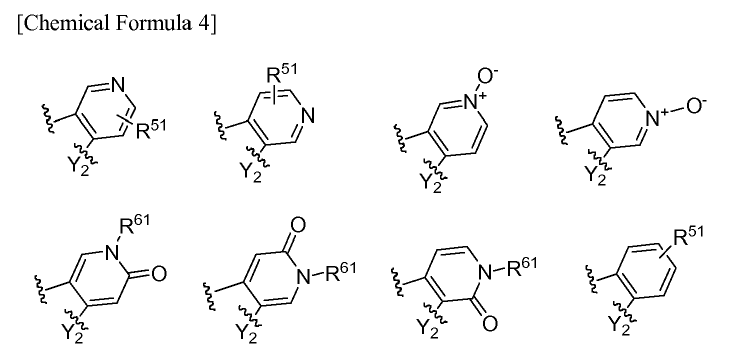 [wherein R⁵¹ and R⁶¹ have the same meanings as in claim 1].

11. The compound or the pharmaceutically-acceptable salt thereof as claimed in claim 10, wherein A₃ is the following: 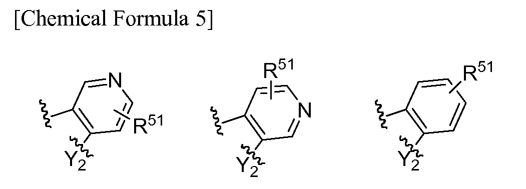 and R⁵¹ is selected from a group consisting of a hydrogen atom, a fluorine atom, a bromine atom, a methoxy group, an ethoxy group, a methylcarbonylamino group and an ethylcarbonylamino group.

12. The compound or the pharmaceutically-acceptable salt thereof as claimed in claim 10, wherein A₃ is the following: 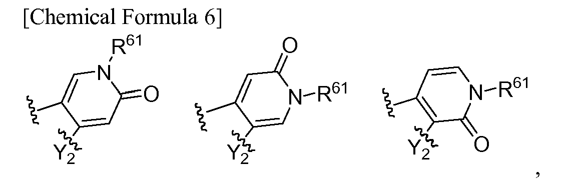 and R⁶¹ is selected from a group consisting of a hydrogen atom, a methyl group and an ethyl group.

13. The compound or the pharmaceutically-acceptable salt thereof as claimed in any of claims 1 to 12, wherein Z is OR¹².

14. The compound or the pharmaceutically-acceptable salt thereof as claimed in claim 13, wherein Z is a hydroxyl group, a methoxy group, an ethoxy group, an n-propyloxy group, an isopropyloxy group, a 2-fluoroethoxy group, a 2,2-difluoroethoxy group, a 2-hydroxyethoxy group, a dimethylamino group, a dimethylcarbamoylmethoxy group, a difluoromethyloxy group, a 2-hydroxy-2-methylpropyloxy group or a cyanomethyloxy group.

15. The compound or the pharmaceutically-acceptable salt thereof as claimed in any of claims 1 to 14, wherein Y₁ and Y₂ are both -CH₂-.

16. The compound or the pharmaceutically-acceptable salt thereof as claimed in any of claims 1 to 14, wherein Y₁ is -O-, and Y₂ is -CH₂-.

17. The compound or the pharmaceutically-acceptable salt thereof as claimed in claim 1, wherein the compound of formula (I) is selected from the following group:
(Z)-(3,4-difluorophenyl){4-[(6-fluoro-1H,1'H-spiro[furo[3,4-c]pyridine-3,4'-piperidin]-1'-yl)methyl]phenyl}methanone O-methyloxime,
(E)-(3,4-difluorophenyl){5-[(6-fluoro-1H,1'H-spiro[furo[3,4-c]pyridine-3,4'-piperidin]-1'-yl)methyl]-2-pyridinyl}methanone O-methyloxime,
(E)-(3,4-difluorophenyl)[5-(1H,1'H-spiro[furo[3,4-c]pyridine-3,4'-piperidin]-1'-ylmethyl)pyridin-2-yl]methanone O-(2-fluoroethyl)oxime,
(E)-(3,4-difluorophenyl)[2-(1H,1'H-spiro[furo[3,4-c]pyridine-3,4'-piperidin]-1'-ylmethyl)pyrimidin-5-yl]methanone O-(2-fluoroethyl)oxime,
(E)-(3,4-difluorophenyl)[5-(1H,1'H-spiro[furo[3,4-c]pyridine-3,4'-piperidin]-1'-ylmethyl)pyrimidin-2-yl]methanone O-ethyloxime,
N- {1'-[(6-{(E)-(3,4-difluorophenyl)[(ethyloxy)imino]methyl}-3-pyridinyl)methyl]-3H-spiro[furo[3,4-c]pyridine-1,4'-piperidin]-6-yl}acetamide,
(E)-(3,4-difluorophenyl)[5-(1H,1'H-spiro[furo[3,4-c]pyridine-3,4'-piperidin]-1'-ylmethyl)pyridin-2-yl]methanone O-(2-hydroxy-2-methylpropyl)oxime,
1'-({6-[(E)-(3,4-difluorophenyl)(ethoxyimino)methyl]pyridin-3-yl}methyl)-5-methyl-3,5-dihydro-6H-spiro[furo[3,4-c]pyridine-1,4'-piperidin]-6-one,
1'-({6-[(E)-(3,4-difluorophenyl)(ethoxyimino)methyl]pyridin-3-yl}methyl)-3,5-dihydro-6H-spiro[furo[3,4-c]pyridine-1,4'-piperidin]-6-one,
1'-({5-[(E)-(3,4-difluorophenyl)(ethoxyimino)methyl]pyridin-2-yl}methyl)-5-methyl-3,5-dihydro-6H-spiro[furo[3,4-c]pyridine-1,4'-piperidin]-6-one,
1'-({6-[(E)-(3,4-difluorophenyl)(hydroxyimino)methyl]pyridin-3-yl}methyl)-5-methyl-3,5-dihydro-6H-spiro[furo[3,4-c]pyridine-1,4'-piperidin]-6-one,
{[((1E)-(3,4-difluorophenyl){5-[(5-methyl-6-oxo-5,6-dihydro-1'H,3H-spiro[furo[3,4-c]pyridine-1,4'-piperidin]-1'-yl)methyl]pyridin-2-yl}methylene)amino]oxy}acetonitrile,
1'-[(6- {(E)-(3,4-difluorophenyl)[(2-hydroxy-2-methylpropoxy)imino]methyl}pyridin-3-yl)methyl]-5-methyl-3,5-dihydro-6H-spiro[furo[3,4-c]pyridine-1,4'-piperidin]-6-one,
(Z)-(3,4-difluorophenyl){4-[(6-fluoro-1H,1'H-spiro[furo[3,4-c]pyridine-3,4'-piperidin]-1'-yl)methyl]phenyl}methanone O-(2-methoxyethyl)oxime,
methyl {[((1Z)-(3,4-difluorophenyl){4-[(6-fluoro-1H,1'H-spiro[furo[3,4-c]pyridine-3,4'-piperidin]-1'-yl)methyl]phenyl}methylene)amino]oxy} acetate,
(Z)-(3,4-difluorophenyl){4-[(6-fluoro-1H,1'H-spiro[furo[3,4-c]pyridine-3,4'-piperidin]-1'-yl)methyl]phenyl}methanone O-(2-hydroxyethyl)oxime,
2-{[((1Z)-(3,4-difluorophenyl){4-[(6-fluoro-1H,1'H-spiro[furo[3,4-c]pyridine-3,4'-piperidin]-1'-yl)methyl]phenyl}methylene)amino] oxy}-N,N-dimethylacetamide,
2-{[((1Z)-(3,4-difluorophenyl){4-[(6-fluoro-1H,1'H-spiro[furo[3,4-c]pyridine-3,4'-piperidin]-1'-yl)methyl]phenyl}methylene)amino] oxy}-N-methylacetamide,
(E)-(3,4-difluorophenyl){5-[(5-oxido-1H,1'H-spiro[furo[3,4-c]pyridine-3,4'-piperidin]-1'-yl)methyl]-2-pyndinyl}methanone O-ethyloxime,
(Z)-(5-chloropyridin-2-yl)[4-(1H,1'H-spiro[furo[3,4-c]pyridine-3,4'-piperidin]-1'-ylmethyl)phenyl]methanone O-ethyloxime,
(Z)-(3,4-difluorophenyl)[3-methyl-4-(1H,1'H-spiro[furo[3,4-c]pyridine-3,4'-piperidin]-1'-ylmethyl)phenyl]methanone O-methyloxime,
[({(1E)-(3,4-difluorophenyl)[5-(1H,1'H-spiro[furo[3,4-c]pyridine-3,4'-piperidin]-1'-ylmethyl)-2-pyridinyl]methylidene}amino)oxy] acetonitrile,
1'-{[4-((Z)-(5-chloro-2-pyridinyl){[(2-hydroxy-2-methylpropyl)oxy]imino}methyl)phenyl]methyl}-5-methyl-3,5-dihydro-6H-spiro [furo[3,4-c]pyridine-1,4'-piperidin]-6-one,
[({(1E)-(3,4-difluorophenyl)[3-(methyloxy)-4-(1H,1'H-spiro[furo[3,4-c]pyridine-3,4'-piperidin]-1'-ylmethyl)phenyl]methylidene}amino)oxy]acetonitrile,
[({(1Z)-(3,4-difluorophenyl)[3-(methyloxy)-4-(1H,1'H-spiro[furo[3,4-c]pyridine-3,4'-piperidin]-1'-ylmethyl)phenyl]methylidene}amino)oxy]acetonitrile,
1'-[(4-(Z)-(6-chloro-3-pyridinyl)[(ethyloxy)imino]methyl}phenyl)methyl]-5-methyl-3,5-dihydro-6H-spiro[furo[3,4-c]pyridine-1,4'-piperidin]-6-one,
1'-({6-[(E)-[(cyclopropyloxy)imino](3,4-difluorophenyl)methyl]-3-pyridinyl}methyl)-5-methyl-3,5-dihydro-6H-spiro[furo[3,4-c]pyridine-1,4'-piperidin]-6-one,
(E)-(3,4-difluorophenyl)[5-(1H,1'H-spiro[furo[3,4-c]pyridine-3,4'-piperidin]-1'-ylmethyl)-2-pyrimidinyl]methanone O-(2-hydroxy-2-methylpropyl)oxime,
(E)-(3,4-difluorophenyl){5-[(6-fluoro-1H,1'H-spiro[furo[3,4-c]pyridine-3,4'-piperidin]-1'-yl)methyl]-2-pyrimidinyl}methanone O-(2-hydroxy-2-methylpropyl)oxime,
(E)-(3,4-difluorophenyl)[5-(1H,1'H-spiro[furo[3,4-c]pyridine-3,4'-piperidin]-1'-ylmethyl)-2-pyridinyl]methanone oxime,
(R)-(E)-(3,4-difluorophenyl) {5-[1-(1H,1'H-spiro[furo[3,4-c]pyridine-3,4'-piperidin]-1'-yl)ethyl]-2-pyridinyl}methanone O-(2-hydroxy-2-methylpropyl)oxime,
(E)-(3,4-difluorophenyl){5-[1-(1H,1'H-spiro[furo[3,4-c]pyridine-3,4'-piperidin]-1'-yl)ethyl]-2-pyridinyl}methanone O-(2-hydroxy-2-methylpropyl)oxime,
(S)-(E)-(3,4-difluorophenyl){5-[1-(1H,1'H-spiro[furo[3,4-c]pyridine-3,4'-piperidin]-1'-yl)ethyl]-2-pyridinyl}methanone O-(2-hydroxy-2-methylpropyl)oxime, and
1'-[(6- {(E)-(3,4-difluorophenyl)[(fluoromethoxy)imino]methyl}-3-pyridinyl)methyl]-5-methyl-3,5-dihydro-6H-spiro[furo[3,4-c]pyridine-1,4'-piperidin]-6-one.

18. A melanin concentrating hormone receptor antagonist comprising a compound of any of claims 1 to 17 or a pharmaceutically-acceptable salt thereof as the active ingredient.

19. A pharmaceutical composition comprising a pharmaceutically-acceptable additive and a therapeutically-effective amount of a compound of any of claims 1 to 17 or a pharmaceutically-acceptable salt thereof.

20. A preventive or a remedy for bulimia, obesity, diabetes, fatty liver, depression or anxiety comprising a compound of any of claims 1 to 17 or a pharmaceutically-acceptable salt thereof as the active ingredient.
